# EUROPEAN PATENT SPECIFICATION

(11) **EP 1 838 881 B1**
(45) Date of publication and mention of the grant of the patent: **22.02.2012**
(21) Application number: 06706287.7
(22) Date of filing: 17.01.2006
(51) Int. Cl.: C12Q 1/70

(54) **METHOD FOR THE DETECTION OF HPV AND PROBES, PRIMERS AND KITS**
VERFAHREN ZUM NACHWEIS VON HPV SOWIE SONDEN, PRIMER UND KITS
METHODE DE DETECTION ET MATIERES ASSOCIEES

(30) Priority: 18.01.2005 GB 0500996
(43) Date of publication of application: 03.10.2007
(73) Proprietor: DDL Diagnostic Laboratory B.V., 2275 CX Voorburg (NL); GlaxoSmithKline Biologicals s.a., 1330 Rixensart Brussels (BE)
(72) Inventor: COLAU, Brigitte Desiree Alberte, GlaxoSmithKline Biologicals, B-1330 Rixensart (BE); KLETER, Gijsbertus Everardus Maria, Delft Diagnostic Laboratory BV, NL-2275 CX Voorburg (NL); QUINT, Wilhelmus Gregorius, Delft Diagnostic Laboratory BV, NL-2275 CX Voorburg (NL); VAN ALEWIJK, Dirk Cornelis Jerrefaas Gelde, Delft Diagnostic Laboratory BV, NL-2275 CX Voorburg (NL); VAN DEN MUNCKHOF, Henricus Arno Marie, Delft Diagnostic Laboratory BV, NL-2275 CX Voorburg (NL); VAN DOORN, Leendert Jan, Delft Diagnostic Laboratory BV, NL-2275 CX Voorburg (NL)
(74) Representative: Privett, Kathryn Louise
(86) International application number: PCT/EP2006/000421
(87) International publication number: WO 2006/077102

(56) References cited:
- WO-A-97/39010
- WO-A-99/63118
- DE-A1- 19 903 056
- US-A- 5 447 839
- US-A- 5 639 871
- DATABASE EMBL [Online] 24 December 2002 (2002-12-24), "Sequence 357 from patent US 6482588." XP002416707 retrieved from EBI accession no. EM_PAT:AR255073 Database accession no. AR255073 & WO 99/14377 A2 (INNOGENETICS NV [BE]; DELFTS DIAGNOSTIC LAB B V [NL]; DOORN LEEN JAN V) 25 March 1999 (1999-03-25)
- DATABASE EMBL [Online] 19 December 2003 (2003-12-19), "Sequence 79 from Patent WO03083142." XP002416708 retrieved from EBI accession no. EM_PAT:AX926131 Database accession no. AX926131 & WO 03/083142 A2 (NORCHIP AS [NO]; KARLSEN FRANK [NO]; ALLARD SUSAN JOYCE [GB]) 9 October 2003 (2003-10-09)
- GRAVITT P E ET AL: "Genotyping of 27 human papillomavirus types by using L1 consensus PCR products by a single-hybridization, reverse line blot detection method." JOURNAL OF CLINICAL MICROBIOLOGY. OCT 1998, vol. 36, no. 10, October 1998 (1998-10), pages 3020-3027, XP002402896 ISSN: 0095-1137
- COUTLÉE F ET AL: "Comparison between vaginal tampon and cervicovaginal lavage specimen collection for detection of human papillomavirus DNA by the polymerase chain reaction. The Canadian Women's HIV Study Group." JOURNAL OF MEDICAL VIROLOGY. JAN 1997, vol. 51, no. 1, January 1997 (1997-01), pages 42-47, XP002402897 ISSN: 0146-6615
- DE VILLIERS E-M ET AL: "Classification of papillomaviruses" VIROLOGY, ACADEMIC PRESS,ORLANDO, US, vol. 324, no. 1, 20 June 2004 (2004-06-20), pages 17-27, XP004512668 ISSN: 0042-6822

## Description

### FIELD OF THE INVENTION

The present invention relates to the field of detection and identification of Human Papillomavirus (HPV) infections

### BACKGROUND OF THE INVENTION

Cervical cancer is the second most common malignancy in women, following breast cancer. Carcinoma of the cervix is unique in that it is the first major solid tumor in which HPV DNA is found in virtually all cases and in precursor lesions worldwide.

Over 100 HPV types have been characterized and are numbered in chronological order of isolation. HPV is epitheliotropic and infects only the skin (cutaneous types) or the mucosa of the respiratory and anogenital tract (mucosal types). More than 40 HPV types are known to infect the uterine cervix. Based on the induced benign, premalignant or malignant lesions, HPV is divided into low-risk (e.g., HPV types 6, 11, 42, 43 and 44) and high-risk types (e.g., types 16, 18, 31, 33 and 45), respectively. The high-risk types account for more than 99% of all invasive cervical cancers. Consequently, detection and identification of HPV types is very important. The high-risk types are by definition consistently found in high grade SIL (Squamous Intraepithelial Lesion) and carcinoma *in-situ* whereas low risk types are mainly found in low grade SIL. This epidemiological observation is supported by molecular findings. For instance, the E6 and E7 proteins from low-risk types 6 and 11 bind p53 and pRB too weakly to immortalize keratinocytes in vitro or to induce malignant transformation in vivo (Woodworth et al., 1990). The circular ds-DNA genome of low-risk HPV types remains episomal whereas the genome of high-risk HPV types is able to integrate into the human genome.

Screening for malignant and premalignant disorders of the cervix is usually performed according to the Papanicoloau (PAP) system. The cervical smears are examined by light microscopy and the specimens containing morphologically abnormal cells are classified into PAP I to V, at a scale of increasing severity of the lesion. This cytomorphological method is an indirect method and measures the possible outcome of an HPV infection. Therefore, HPV DNA detection and typing is of importance in secondary screening in order to select patients for monitoring (follow-up) and treatment. This means that cervical smears classified as PAP II (atypical squamous metaplasia) or higher classes should be analyzed for low-risk and high risk HPV types. Follow-up studies have shown that only high-risk HPV types are involved in the progression from cytologically normal cervix cells to high grade SIL (Remminck et al., 1995).These results indicate that the presence of high-risk HPV types is a prognostic marker for development and detection of cervical cancer.

Diagnosis of HPV by culture is not possible. Also diagnosis by detection of HPV antibodies appears to be hampered by insufficient sensitivity and specificity. Direct methods to diagnose an HPV infection are mainly based on detection of the viral DNA genome by different formats of DNA/DNA or RNA/DNA hybridization with or without prior amplification of HPV DNA. The polymerase chain reaction (PCR) is a method that is highly efficient for amplification of minute amounts of target DNA. Nowadays, mainly three different primer pairs are used for universal amplification of HPV DNA ("broad spectrum primers"). Three of these primer pairs, MY11 /MY09, GP5/GP6 and the SPF10 system, are directed to conserved regions among different HPV types in the LI region (Manos et al., 1989; Van den Brule et al., 1990, WO9914377). The PGMY system, a modification of the MY09/11 is also used (see Gravitt, P., 2000. Improved amplification of genital human papillomaviruses. J. Clin. Microbiol. 38:357-361). Another primer pair, CPl/CPllg, is directed to conserved regions in the E1 region (Tieben et al., 1993) but CPI/II is not often used.

There are several methods to identify the various HPV types.

HPV DNA can be typed by PCR primers that recognize only one specific type. This method is known as type-specific PCR. Such methods have been described for HPV types 6, 11, 16, 18, 31 and 33 (Claas et al., 1989; Cornelissen et al., 1989; Falcinelli et al., 1992; Van den Brule et al., 1990; Young et al., 1989). The primers are aimed at the E5, L1, E6, L1, E2 and E1 regions of the HPV genome for types 6, 11, 16, 18, 31 and 33, respectively (Baay et al., 1996).

Another method is general amplification of a genomic part from all HPV types followed by hybridization with two cocktails of type-specific probes differentiating between the oncogenic and non-oncogenic groups, respectively. A similar typing method has been described without prior amplification of HPV DNA. In the hybrid capture assay (Hybrid Capture Sharp Assay; Digene, Silver Springs, MD), each sample is tested for a group of "high-risk" HPV types (eg 16, 18, 31, 33, 35, 39, 45, 51, 52, 56, 58, 59 and 68) and for another group of "low-risk" HPV types (eg 6, 11, 42, 43 and 44) (Cox et al., 1995).

A detection and typing system disclosed in WO9914377, utilises a PCR amplification step and a reverse line blot hybridization with type specific probes.

At present, formal classification of human papillomaviruses is based on sequence analysis of a 291 bp fragment from the L1 region (Chan et al. J Virol. 1995 May;69(5):3074-83, DeVilliers et al., Virology. 2004 Jun 20;324(1):17-27) Phylogenetic analysis of these sequences allows classification of the different HPV types. By definition, if the sequence difference across this region between two HPV isolates is higher than 10% they are classified as different types. Consequently, if the sequence differs more than 10% from any known HPV type it is classified as a novel HPV type. HPV isolates that differ between 2-10% are classified as different subtypes. Finally, if the sequence variation is below 2%, the 2 isolates are classified within the same subtype as different variants.

W099/14377 discloses a method for the detection of HPV in a biological sample.

There is still a need for improved detection and typing systems.

### STATEMENT OF INVENTION

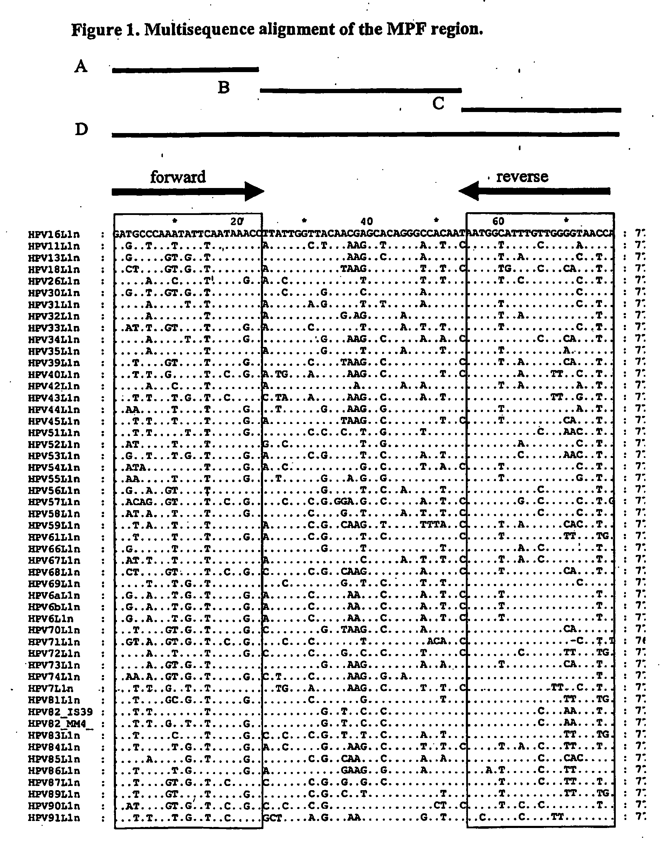

The present invention relates to a method for typing of any HPV nucleic acid possibly present in a sample, the method comprising the steps of contacting any such nucleic acid with at least one probe capable of specific hybridization within the B region of HPV, said region being indicated in Figure 1, and then analysing HPV type(s) based upon the hybridisation result so obtained.

The invention further relates to a method in which an amplification step is carried out to amplify any HPV nucleic acid possibly present in a biological sample prior to the hybridization step.

As such the invention relates to a method for detection and/or typing of any HPV nucleic acid possibly present in a biological sample, the method comprising the steps of:
(i) amplification of a polynucleic acid fragment comprising the B region of any HPV nucleic acid in the sample, said B region being indicated in Figure 1, and
(ii) contacting any amplified fragments from step (i) with at least one probe capable of specific hybridization with the B region of HPV, said B region being indicated in Figure 1.

The invention also relates to a for detection and/or typing of HPV possibly present in a biological sample, the method comprising:
(i) amplification of a polynucleic acid fragment of HPV by use of-
   - a 5' primer specifically hybridizing to the 'A' region or of the genome of HPV 16, said 'A' region being indicated in Figure 1, and
   - a 3' primer specifically hybridizing to the 'C' region of the genome of at least one HPV type, said 'C' region being indicated in Figure 1;
(ii) hybridizing the amplified fragments from step (i) with at least one probe capable of specific hybridization with the 'B' region of HPV, said region being indicated in Figure 1.

The invention further relates to a method in which an amplification step is carried out to amplify any signal used to detect hybridisation of the probe with any HPV nucleic acid possibly present in a biological sample. Signal amplification can occur with or without a step to amplify any HPV nucleic acid possibly present in the sample.

The invention further relates to a method for typing of any HPV nucleic acid possibly present in a biological sample, the method comprising a step to detect the presence of any HPV nucleic acid present in a sample prior to or simultaneously with any typing step.

The invention further relates to oligonucleotide probes and primers enabling said method of detection and/or identification, of HPV.

The invention further relates to protocols according to which said amplification and hybridization steps can be performed. One format for the hybridization step is, for instance, the reverse hybridization format..

The invention further relates to kits comprising primers and/or probes and/or instructions for use in carrying out the invention.

### FIGURES

Figure 1 illustrates an alignment of different HPV sequences with reference to the sequence of an HPV 16 sequence Genbank accession number K02718.1, and showing location of the A, B , C and D regions.
Figure 2 illustrates the phylogenetic tree of the B region,
Figure 3 illustrates an example of a PCR product, using single PCR primers,
Figure 4 illustrates a gel multiplex PCR,
Figure 5 illustrates results that may be obtained using a line probe assay,
Figure 6 illustrates a general method for detection and typing of DNA using the Luminex (bead based) approach,
Figures 7illustrates a possible HPV "MPF" genotyping assay; and
Figure 8 HPV illustrates "MPF" genotyping patterns of HPV types 16, 18, 26, 31, 33 and 35.

### DETAILED DESCRIPTION

The present invention generally relates to a method for detection and/or typing of any HPV nucleic acid possibly present in a biological sample, the method comprising the steps of contacting any such nucleic acid present with at least one probe capable of specific hybridization within the B region of the HPV genome, said B region being indicated in Figure 1, and then detecting any specific hybridization that might result to determine if there is HPV nucleic acid in the sample, and to which HPV type it might belong.

We have determined that the 77 nucleotide D region of the HPV genome (see Figure 1), and especially the interprimer B region 31 nucleotides, is highly informative in respect of HPV typing.

The method of the invention thus generally comprises hybridization of nucleic acid from HPV with a probe capable of hybridizing B region of HPV, said hybridization event, or even absence of a hybridisation event, providing information which allows different HPV types to be discriminated.

The hybridisation of probe with target nucleic acid takes place under reaction conditions where specific hybridisation of the probe can occur.

The analysis of HPV type(s) present in the sample may be carried out at different levels of resolution.

Analysis may be at a resolution suitable to identify individual HPV types, such as HPV 16, 18, or HPV 1, for example.

Analysis of types may also be carried out at a lower resolution, for example to identify whether an individual has any HPV type of a given category - such as a high risk cancer type or low risk cancer type, or a cutaneous type.

Whilst the typing assay of the present invention is suitably able to provide information on all specific types found in a sample, nevertheless it may not be necessary (from the point of view of the user) to be able to discriminate between exact HPV types, and the output of the assay may only need to be at the level of categories of HPV types.

The invention thus relates to a method of HPV typing, the method allowing the identification of high risk HPV types, without indication of which specific high risk type is present in a sample.

The category of high risk types (those consistently found in high grade SIL [Squamous Intraepithelial Lesion] and carcinoma *in-situ*) include HPV 16, 18, 31, 33, 35, 39, 45, 51, 52, 56, 58, 59, 66, and 68.

The category of low risk types (mainly found in low grade SIL) include types HPV 6, 11, 34, 40, 42, 43, 44, 53, 54, 70, and 74.

Preferably the specific probes used in the invention are capable of specific hybridisation within the 31 nucleotide "B" region, where this region is given by reference to the sequence of Figure 1. These regions correspond to nucleotides 6566 - 6596 (B region) of the HPV 16 reference sequence K02718.

It will be appreciated that reference to D and B regions using the numbering of Figure 1 herein includes equivalent regions in other HPV sequences which are not specifically listed, and which may vary from the HPV reference sequence or other sequences given. An equivalent A, B, C or D region in another HPV genome may be identified on the basis of, for example, sequence homology or identity with the sequences of Figure 1.

Sequence comparisons of nucleic acid identity/homology are readily carried out by the skilled person, for example using the BLAST and BLAST 2.0 algorithms, which are described in Altschul et al., Nucl. Acids Res. 25:3389-3402 (1977), and Altschul et al., J. Mol. Biol. 215:403-410 (1990), respectively. BLAST and BLAST 2.0 can be used, for example with the default parameters, to determine percent sequence identity for the polynucleotides of the invention. Software for performing BLAST analyses is publicly available through the National Center for Biotechnology Information.

Thus the invention can be seen to relate to probes and to the use of probes which are capable of specific hybridization within the D region, suitably within the B region, of HPV, said regions being indicated in Figure 1 or are capable of specific hybridization within an equivalent region in another HPV genome, the equivalent region being assessed by nucleic acid identity and/or homology. For the avoidance of doubt all probes described herein are claimed individually and in groups of (where appropriate) at least 5, 10, 15, 20, 25, 30, 35, 40 probes, groups being selected from the tables in which the probes are listed.

The present disclosure also relates to nucleic acid fragments consisting essentially of the isolated 77 base pair D region and the isolated 31 base pair B region, either region being in single or double stranded nucleic acid form, as RNA or DNA, and to use of these nucleic acid fragments regions in typing of HPV.

One feature of the present invention is selection of probes.

Probes which specifically hybridise to B regions of the HPV genome are preferably able to provide information (via hybridisation results) as to the type of the HPV strain present, either alone or in combination with information from another probe or probes. Information about HPV type is preferably obtained by positive detection of hybridisation of a probe with target nucleic acid, but may also be obtained by absence of hybridisation of a given probe.

Suitably a probe of the present invention is capable of specific hybridization within the B region, of the genome of only one HPV type, and thus enables specific identification of this HPV type, when this type is present in a biological sample.

Thus an embodiment of the invention relates to a method for typing of any HPV nucleic acid possibly present in a biological sample, the method comprising the steps of contacting any such nucleic acid with at least one probe capable of specific hybridization within the B region, of the genome of only one HPV type, said regions being indicated in Figure 1, and then analysing HPV type(s) based upon the hybridisation result so obtained.

A probe of the present invention may still provide useful information if it is capable of specific hybridization within the B region of the genome of a limited number of types, such as only 2 HPV types. For example this can enable identification of these types, or may enable specific identification of each type in combination with information from another probe.

Probes capable of giving information about HPV types, such as those above, are generally considered as type specific probes herein. Preferred type specific probes are capable of specific hybridization within the B region, of the genome of only one HPV type. According to another preferred embodiment of the present invention, a probe capable of specific hybridization with the B region of the genome of only one HPV type, more particularly specifically hybridizes to the 31 bp B region situated between the A region and the C region, as indicated in Figure 1.

The different types of HPV in a sample can be identified by hybridization of nucleic acids of said types of HPV to at least one, preferably at least two, more preferably at least three, even more preferably at least four and most preferably at least five oligonucleotide probes.

Table 4 contains a list of preferred probes specifically hybridizing to the D region. These probes may be used together, suitably under the same conditions of hybridization and washing. Preferred is a reverse hybridization format, such as a line probe assay format for example. All probes listed are herein individually claimed. Moreover, all combinations of probes are herein contemplated.

The probes listed in Table 4 specifically hybridise to the B and/or D region of HPV and are able to provide information about specific types of HPV target nucleic acid that may be present in a sample.

It will be clear to one skilled in the art that probes other than those listed in Table 4 may be chosen within said B region, preferably probes that specifically hybridize to only one HPV-type and/or which are capable of providing information allowing HPV type determination.

Probes for use in the present invention may have an additional spacer sequence which does not form part of the probe itself but which can allows for attachment to a solid support, for example. The spacer region may be added enzymatically or chemically and may be 5' or 3' of the probe.

Suitably the use of probes of the invention allow typing of at least 5 different HPV types, preferably at least 6, 7, 8, 9, 10,11,12,13,14,15,16,17,18,19,20,21,22,23,24,25,26,27,28,29,30, 31,32,33,34,35,36,37,38,39, 40 ,41,42,43,44,45, 46, 47, 48, 49, 50 or at least 51 different HPV types. Most preferably the present invention allows more than 30 different HPV types to be differentiated, suitably more than 35, more than 40, more than 45 and suitably more than 50 different HPV types.

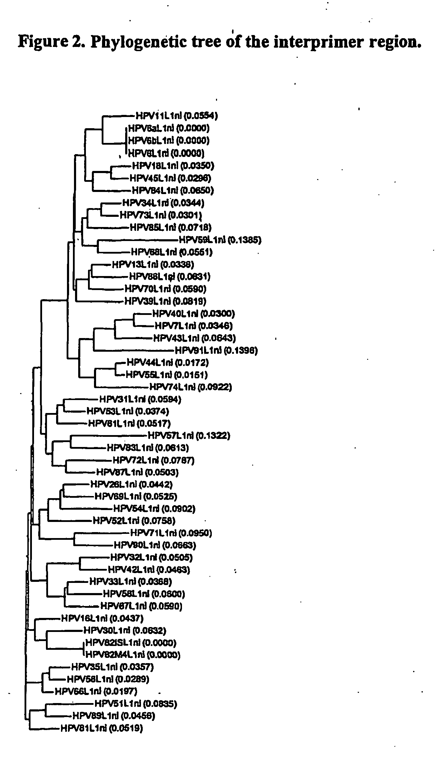

Suitably all of the HPV types given in the phylogenetic tree of Figure 2, or substantially all, can be differentiated using the invention outlined herein.

Any HPV nucleic acid present in the sample is preferably first amplified, for example by PCR or other suitable amplification process, prior to hybridization. Amplification of any target nucleic acid may be carried out using so called "broad spectrum" primers or primer sets that allow for amplification of all HPV nucleic acid in a sample, regardless of type.

Reference to HPV nucleic acid present in a sample thus includes nucleic acid that has been amplified from a sample, where this is clear from the context (i.e. an amplification step is present prior to hybridisation).

Suitably the amplification of any target DNA includes amplification of the 31 nucleotide B region of Figure 1.

Thus, in one embodiment the present invention relates to a method for detection and/or typing of any HPV nucleic acid possibly present in a biological sample, the method comprising the steps of:
(i) amplification of a polynucleic acid fragment comprising the B region of any HPV nucleic acid in the sample, said B region being indicated in Figure 1, and
(ii) contacting any amplified fragments from step (i) with at least one probe capable of specific hybridization with the B region of HPV said B region being indicated in Figure 1.

Suitably the amplification of any target nucleic acid includes amplification of the 77 nucleotide fragment of Figure 1, i.e the D region of Figure 1.

Thus, in one embodiment the present invention relates to a method for detection and/or typing of any HPV nucleic acid possibly present in a biological sample, the method comprising the steps of:
(i) amplification of a polynucleic acid fragment comprising the D region of any HPV nucleic acid in the sample, said D region being indicated in Figure 1, and
(ii) contacting any amplified fragments from step (i) with at least one probe capable of specific hybridization with the B region of HPV said B region being indicated in Figure 1.

In a further embodiment the invention provides a method for detection and/or typing of HPV possibly present in a biological sample, the method comprising:
(i) amplification of a polynucleic acid fragment of HPV by use of-
   - a 5' primer specifically hybridizing to the 'A' region or of the genome of HPV 16, said 'A' region being indicated in Figure 1, and
   - a 3' primer specifically hybridizing to the 'C' region of the genome of at least one HPV type, said `C' region being indicated in Figure 1;
(ii) hybridizing the amplified fragments from step (i) with at least one probe capable of specific hybridization with the 'B' region, said regions being indicated in Figure 1.

Suitably the region to be amplified comprises the D region 77 nucleotides 6543 -6619 of the HPV genome, where this numbering is given by reference to the HPV 16 reference sequence of Figure 1, or consists of this region, or consists essentially of this region.

The region to be amplified is suitably no more than fragment 6543 - 6619 of the HPV genome, numbering given with reference to the HPV 16 reference sequence, or equivalent region in other HPV genomes.

According to another preferred embodiment of the present invention, the 3' end of said 5' primer specifically hybridizing to the A region of the genome of at least one HPV type, is situated at position 6565 of the genome of HPV 16 (reference strain Genbank accession number K02718.1), or at the corresponding position of any other HPV genome, as indicated in Figure 1.

According to another preferred embodiment of the present invention, the 3' end of said 3' primer specifically hybridizing to the C region of the genome of at least one HPV type, is situated at position 6597 of the genome of HPV 16 (Genbank accession number K02718.1), or at the corresponding position of any other HPV genome, as indicated in figure 1.

Preferred primers for amplification of nucleic acid in a sample include those listed in Tables 1 and 2. These are claimed individually and in the form of combinations. Preferred are primer pairs, comprising a forward and reverse primer.

Suitably primers for general amplification of HPV nucleic acid prior to specific typing are able to amplify all HPV nucleic acid present in a sample. Preferred are groups of primers capable of amplification of all HPV nucleic acid in a sample, suitably the group comprising one or more primers from the set listed in Tables 1 and 2. Optionally, all primers listed in Tables 1 and 2 may be used. Primer combinations are suitably able to be used under the same reaction conditions.

Amplification of nucleic acid may be carried out on any suitable fragment which comprises the D or B region of the invention. Preferred fragments for amplification are less than 200 nucleotides, preferably less than 150 nucleotides, preferably less than 100 nucleotides in length. Preferred fragments for amplification are short enough to allow detection both in cervical swabs and from samples embedded in paraffin, for example.

In another aspect of the invention the primers and probes disclosed in the present invention may also be used in quantitative PCR protocols or quantitative hybridisation protocols. Quantitative PCR (QPCR) allows quantification of starting amounts of DNA, cDNA, or RNA templates. QPCR can be based on the detection of a fluorescent reporter molecule that increases as PCR product accumulates with each cycle of amplification. Fluorescent reporter molecules include dyes that bind double-stranded DNA (i.e. SYBR Green I) or sequence-specific probes (i.e. Molecular Beacons or TaqMan® Probes).

As discussed above certain probes may provide information about the exact HPV type, for example if they are able to hybridise to a given type but not to other types (i.e type specific probes). Probes that are specific for the D region may also be used to more generally determine if there is any HPV nucleic acid present in a sample without necessarily giving typing information. Such probes may be referred to as 'universal probes' herein. Samples which are found to be positive for HPV nucleic acid can then be specifically typed using specific typing methods, such as type specific probes or type specific PCR. Alternatively samples can be both probed with universal probes and specifically typed simultaneously.

Universal probes may contain inosine residues as part of the nucleic acid probe sequence, which allows for some flexibility in hybridisation to target nucleic acid, and can allow hybridisation to the D region of different HPV types. Optionally primers may also contain inosine, where useful.

For the avoidance of doubt, probes that specifically hybridise to the D and /or B region of any HPV nucleic acid in a sample may be universal (if that they hybridise to multiple HPV types in the D and or B region and/or do not give specific typing information) or type-specific probes which allow an unknown HPV nucleic acid to be typed.

Where the target DNA is amplified prior to typing, then universal probes which fall within the preferred D or B regions may also be used to detect HPV nucleic acid..

The invention thus also relates to probes, or groups of probes, which are able to detect the presence of any HPV nucleic acid in a sample.

Universal probes may be used to detect HPV nucleic acid e.g., using the DNA Enzyme Immuno Assay (DEIA) technique, for example as referred to in WO9914377 and described in for example in Clin Diagn Virol. 1995 Feb;3(2):155-64. This method is used for rapid and specific detection of PCR products. PCR products are generated by a primer set, of which either the forward or the reverse primer contain biotin at the 5' end. This allows binding of the biotinylated amplimers to streptavidin-coated microtiter wells. PCR products are denatured by sodium hydroxide, which allows removal of the non-biotinylated strand. Specific labelled oligonucleotide probes (e.g. with digoxigenin) are hybridized to the single stranded immobilized PCR product and hybrids are detected by enzyme-labelled conjugate and colorimetric or fluorimetric methods.

In the present invention there are provided a group of universal probes suitable for determination of the presence of HPV nucleic acid in a sample, suitably in the DEIA technique. Suitably such probes can be used under the same reaction conditions. Preferred probes are given in Table 3. All probes described therein are claimed individually, and in combination. The invention suitably provides a combination of any 2 probes of Table 3, suitably any 3, and 4, and 5 or more probes for general detection of HPV (ie detection of any HPV type), preferably all probes included in Table 3.

A separate embodiment the invention relates to use of universal probes that specifically hybridise within the D region of the HPV genome , such as those of Table 3, in combination with a subsequent or simultaneous typing step.

After the hybridization between the probe and any target DNA, detection of the hybridization may be carried out by any suitable means. For example, the probe and/or nucleic acid target may be detectably labelled. To assist in detection it is preferred that the target and/or the signal are amplified. PCR amplification of the target DNA is especially preferred.

The hybridisation between probe and target is preferably carried out in the presence of a solid support, although this is not obligatory. One or more of the probe and target nucleic acid may be immobilised, for example, being fixed to a beads, plates, slide or a microtitre dish. Alternatively neither probe nor target may be immobilised. Hybridisation may be carried out in the context of a liquid medium.

Detection of binding maybe carried out using flow cytometry, for example using the Luminex™ flow cytometry system (see, for example, WO9714028 and http://www.luminexcorp.com/).

Target specific probes, and mixtures of different target specifc probes, for use with bead-based detection systems such as Luminex are disclosed in the examples herein, and are *per se* embodiments of the present invention. Mixtures may include from 2-100 different probe types, such as 5- 70, 10 - 60, 20 - 50 probe types, including mixtures of 3, 4, 5, 6, 7, 8, 9, 10, 11,12,13,14,15, 16, 17, 18 ,19 20, 25, 30, 35, 40, 45 or more different probe types. Such probes coupled to spacer sequences, and when coupled to beads, as described herein also form part of the present invention *per se.*

Beads for use in the present invention, and which may also be referred to as microspheres herein, are suitably beads that are suitable for use in flow cytometric analysis. Beads are suitably able to be coupled to a probe to detect interaction between a probe and a target. In one aspect beads are labelled with a unique fluorescent molecule or combination of molecules. Suitably the label on or in the beads is able to be identified by use of laser excitation of one or more fluorochromes within the bead. In one aspect the bead is a polystyrene bead.

Detection of binding may also be carried out in the context of a microarray, using for example methods as described in EP373203, EP386229, EP0804731 and EP619321. Such techniques are well known to the person skilled in the art.

According to another preferred embodiment of the present invention, the aforementioned methods of detection and/or identification of HPV are characterized further in that the hybridization step involves a reverse hybridization format. In one embodiment the probes are immobilized to certain locations on a solid support. In another embodiment the probes are hybridised to beads, in which case they do not adopt a fixed position relative to one another.

Suitably any HPV nucleic acid in a sample is amplified as described above, and the amplified HPV polynucleic acids are labelled in order to enable the detection of the hybrids formed.

According to this embodiment, at least one probe, or a set of a least 2, preferably at least 3, more preferably at least 4 and most preferably at least 5 probes is used. When at least 2 probes are used, said probes are designed in such a way that they specifically hybridize to their target sequences under the same hybridization conditions and the same wash conditions.

In preferred reverse hybridization assays the oligonucleotide probes are immobilized on a solid support as parallel lines (Stuyver et al., 1993; international application WO 94/12670). The reverse hybridization format has many practical advantages as compared to other DNA techniques or hybridization formats, especially when the use of a combination of probes is preferable or unavoidable to obtain the relevant information sought.

Optionally, where required, the detection and typing methods of the present invention include a type specific PCR step after the hybridization step, , for example as disclosed in WO03014402. Type specific PCR is designed to amplify a specific HPV nucleic acid type, for example HPV 16 DNA only, as compared with non specific primers which may be used prior to HPV typing and generally serve to amplify nucleic acid form multiple HPV types.

The present invention also relates to type specific primers that are capable of amplification of HPV nucleic acid comprising the D and/or B region of the HPV genome.

In another embodiment the invention thus relates to a method comprising:
1 Amplification of nucleic acid from any HPV present in a biological sample,
2 Detection of any HPV nucleic acid present in a biological sample,
3 Typing of the HPV nucleic acid in samples in which such HPV nucleic acid has been detected by contacting such nucleic acid with at least one probe capable of specific hybridization within the B region, of HPV, said region being indicated in Figure 1, and then analysing HPV type based upon the hybridisation result so obtained, and
4 Optionally, amplification and detection of any nucleic in a sample using type specific primers for types not identified in step 3.

Steps 2 and 3 may be carried out simultaneously.

The present invention also relates to kits for use in the present invention, to detect and/or identify HPV types.

A kit can comprise at least 2 primers suitable for amplification of nucleic acid from the genome of HPV, preferably primers capable of amplification of at least fragment 6566 - 6596 of the HPV genome, such as primers given in Tables 1 and 2.

A kit can comprise at least 2 probes capable of specific hybridization to fragment 6543 - 6619 of the HPV genome, with numbering given in respect of Figure 1. Preferred probes are capable of allowing discrimination between different HPV types, with suitable probes listed in Table 4.

A kit can comprise instructions for carrying out the above methods for HPV identification and typing analysis, in combination with a primer and/or probe as indicated above.

A kit can comprise at least one primer and at least one probe, as given above.

A kit can comprise a probe or primer of the present invention immobilised onto a solid support. The support can be a bead, microtitre plate or slide, for example.

A kit can comprise a universal probe or probes, suitably a probe or probes given in Table 3.

The present invention also relates to diagnostic kits for detection and/or identification of HPV possibly present in a biological sample, comprising the following components: (i) at least one suitable primer or at least one suitable primer pair as defined above; (ii) at least one suitable probe, preferably at least 2, more preferably at least 3, even more preferably at least 4 and most preferably at least 5 suitable probes, optionally fixed to a solid support.

Suitably a kit additionally comprises one or more of the following:
(iii) a hybridization buffer, or components necessary for the production of said buffer, or instructions to prepare said buffer;
(iv) a wash solution, or components necessary for the production of said solution, or instructions to prepare said solution;
(v) a means for detection of the hybrids formed;
(vi) a means for attaching the probe(s) to a known location on a solid support.

The following definitions and explanations will permit a better understanding of the present invention.

HPV isolates that display a sequence difference of more than 10% to any previously known type in a 291 bp fragment from the L1 region (Chan et al., 1995) are classified as different HPV "types". HPV isolates that differ between 2 and 10% are classified as different "subtypes". If the sequence variation is below 2%, the isolates are classified within the same subtype as different "variants". The term "type" when applied to HPV refers to any of the three categories defined above.

The target material in the samples to be analyzed may either be DNA or RNA, e.g. genomic DNA, messenger RNA, viral RNA or amplified versions thereof. These molecules are in this application also termed "nucleic acids" or "polynucleic acids".

Well-known extraction and purification procedures are available for the isolation of RNA or DNA from a sample (e.g. in Sambrook et al., 1989).

The term "probe" according to the present invention generally refers to a single- stranded oligonucleotide which is designed to specifically hybridize to HPV polynucleic acids.

The term "primer" generally refers to a single stranded oligonucleotide sequence capable of acting as a point of initiation for synthesis of a primer extension product which is complementary to the nucleic acid strand to be copied. The length and the sequence of the primer must be such that they allow to prime the synthesis of the extension products.

Preferably the primer is about 10-50 nucleotides long. Specific length and sequence will depend on the complexity of the required DNA or RNA targets, as well as on the conditions at which the primer is used, such as temperature and ionic strength.

The expression "primer pair" or "suitable primer pair" in this invention refers to a pair of primers allowing the amplification of part or all of the HPV polynucleic acid fragment for which probes are able to bind.

The term "target" or "target sequence" of a probe or a primer according to the present invention is a sequence within the HPV polynucleic acids to which the probe or the primer is completely complementary or partially complementary (where partially complementary allows for some degree of mismatch). It is to be understood that the complement of said target sequence is also a suitable target sequence in some cases. Probes of the present invention are suitably complementary to at least the central part of their target sequence. In most cases the probes are completely complementary to their target sequence. The term "type-specific target sequence" refers to a target sequence within the polynucleic acids of a given HPV type that contains at least one nucleotide difference as compared to any other HPV-type.

"Specific hybridization" of a probe to a region of the HPV polynucleic acids means that said probe forms a duplex with part of this region or with the entire region under the experimental conditions used, and that under those conditions said probe does not form a duplex with other regions of the polynucleic acids present in the sample to be analysed. It should be understood that probes that are designed for specific hybridisation within a region of HPV polynucleic acid may fall entirely within said region or may to a large extent overlap with said region (i.e. form a duplex with nucleotides outside as well as within said region).

Suitably the specific hybridisation of a probe to a nucleic acid target region occurs under stringent hybridisation conditions , such as 3X SSC, 0.1% SDS, at 50 °C.

The skilled person knows how to vary the parameters of temperature, probe length and salt concentration such that specific hybridisation can be achieved. Hybridization and wash conditions are well known and exemplified in Sambrook, et al., Molecular Cloning: A Laboratory Manual, Second Edition, Cold Spring Harbor, N.Y., (1989), particularly Chapter 11 therein. When needed, slight modifications of the probes in length or in sequence can be carried out to maintain the specificity and sensitivity required under the given circumstances. Probes and/ or primers listed herein may be extended by 1, 2, 3, 4 or 5 nucleotides, for example, in either direction (upstream or downstream of region D).

Preferred stringent conditions are suitably those which allow for a type specific probe binding to only one HPV type. Thus in an embodiment of the invention the method for typing of any HPV nucleic acid possibly present in a biological sample comprises the steps of contacting any such nucleic acid with at least one probe which is capable of hybridisation to the D and/or B region of HPV under stringent conditions.

Probes which specifically hybridise to the D and/or B regions of the HPV genome as defined herein suitably at least 95% complementary to the target sequence over their length, suitably greater than 95% identical such as 96%, 97%, 98%, 99% and most preferably 100% complementary over their length to the target HPV sequence. The probes of the invention can be complementary to their target sequence at all nucleotide positions, with 1, 2, or more mismatches possibly tolerated depending upon the length of probe, temperature, reaction conditions and requirements of the assay, for example.

Suitably each nucleotide of the probe can form a hydrogen bond with its counterpart target nucleotide.

Preferably the complementarity of probe with target is assessed by the degree of A:T and C:G base pairing, such that an adenine nucleotide pairs with a thymine, and such that a guanine nucleotide pairs with a cytosine, or vice versa. In the RNA form, T may be replaced by U (uracil).

Where inosine is used in universal probes, for example, or in primers, then complementarity may also be assessed by the degree of inosine (probe)- target nucleotide interactions.

As such, the present invention can also be seen to relate to a method for detection and/or typing of any HPV nucleic acid possibly present in a biological sample, the method comprising the steps of contacting any such nucleic acid with at least one probe, the probe having 1, or 0 nucleotide mismatches across its length to the B region, of an HPV genome, said region being indicated in Figure 1, and then analysing HPV type based upon the hybridisation result so obtained.

"Specific hybridization" of a primer to a region of the HPV polynucleic acids means that, during the amplification step, said primer forms a duplex with part of this region or with the entire region under the experimental conditions used, and that under those conditions said primer does not form a duplex with other regions of the polynucleic acids present in the sample to be analysed. It should be understood that primers that are designed for specific hybridization to a region of HPV polynucleic acids, may fall within said region or may to a large extent overlap with said region (i.e. form a duplex with nucleotides outside as well as within said region).

An embodiment of the present invention requires the detection of single base pair mismatches and stringent conditions for hybridization of probes are preferred, allowing only hybridization of exactly complementary sequences. However, it should be noted that, since the central part of the probe is essential for its hybridization characteristics, possible deviations of the probe sequence versus the target sequence may be allowable towards the extremities of the probe when longer probe sequences are used. Variations are possible in the length of the probes.

Said deviations and variations, which may be conceived from the common knowledge in the art, should however always be evaluated experimentally, in order to check if they result in equivalent hybridization characteristics as the exactly complementary probes.

Preferably, the probes of the invention are about 5 to 50 nucleotides long, more preferably from about 10 to 25 nucleotides. Particularly preferred lengths of probes include 5, 6, 7, 8, 9, 10, 11, 12, 13, 14, 15, 16, 17, 18, 19, 20, 21, 22, 23, 24 or 25 nucleotides (without counting any spacer sequences that may be present). The nucleotides as used in the present invention may be ribonucleotides, deoxyribonucleotides and modified nucleotides such as inosine or nucleotides containing modified groups which do not essentially alter their hybridization characteristics.

Probe sequences are represented throughout the specification as single stranded DNA oligonucleotides from the 5' to the 3' end. It is obvious to the person skilled in the art that any of the below-specified probes can be used as such, or in their complementary form, or in their RNA form (wherein T is replaced by U).

The probes according to the invention can be prepared by cloning of recombinant plasmids containing inserts including the corresponding nucleotide sequences, if need be by excision of the latter from the cloned plasmids by use of the adequate nucleases and recovering them, e.g. by fractionation according to molecular weight. The probes according to the present invention can also be synthesized chemically, for instance by the conventional phospho-triester method.

The fact that amplification primers do not have to match exactly with the corresponding target sequence in the template to warrant proper amplification is amply documented in the literature (Kwok et al., 1990). However, when the primers are not completely complementary to their target sequence, it should be taken into account that the amplified fragments will have the sequence of the primers and not of the target sequence.

Primers may be labelled with a label of choice (e.g. biotin). The amplification method used can be either polymerase chain reaction (PCR; Saiki et al., 1988), ligase chain reaction (LCR; Landgren et al., 1988; Wu & Wallace, 1989; Barany, 1991), nucleic acid sequence-based amplification (NASBA; Guatelli et al., 1990; Compton, 1991), transcription-based amplification system (TAS; Kwoh et al., 1989), strand displacement amplification (SDA; Walker et al., 1992) or amplification by means of QB replicase (Lomeli et al., 1989) or any other suitable method to amplify nucleic acid molecules known in the art.

The oligonucleotides used as primers or probes may also comprise nucleotide analogues such as phosphorothiates (Matsukura et al., 1987), alkylphosphorothiates or peptide nucleic acids (Egholm M, Buchardt O, Christensen L, Behrens C, Freier SM, Driver DA, Berg RH, Kim SK, Norden B, Nielsen PE. PNA hybridizes to complementary oligonucleotides obeying the Watson-Crick hydrogen-bonding rules.Nature. 1993 Oct 7;365(6446):566-8) or may contain intercalating agents (Asseline et al., 1984). As most other variations or modifications introduced into the original DNA sequences of the invention these variations will necessitate adaptions with respect to the conditions under which the oligonucleotide should be used to obtain the required specificity and sensitivity. However the eventual results of hybridization will be essentially the same as those obtained with the unmodified oligonucleotides. The introduction of these modifications may be advantageous in order to positively influence characteristics such as hybridization kinetics, reversibility of the hybrid-formation, biological stability of the oligonucleotide molecules, etc.

The term "solid support" can refer to any substrate to which an oligonucleotide probe can be coupled, provided that it retains its hybridization characteristics and provided that the background level of hybridization remains low. Usually the solid substrate will be a microtiter plate (e.g. in the DEIA technique), a membrane (e.g. nylon or nitrocellulose) or a microsphere (bead) or a chip. Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH2 groups, SH groups, carboxylic groups, or coupling with biotin, haptens or proteins.

As discussed above, hybridisation may take place in a liquid media, and binding of probe to target assessed by, for example, flow cytometry.

The term "labelled" generally refers to the use of labelled nucleic acids. Labelling may be carried out by the use of labelled nucleotides incorporated during the polymerase step of the amplification such as illustrated by Saiki et al. (1988) or Bej et al. (1990) or labelled primers, or by any other method known to the person skilled in the art. The nature of the label may be isotopic ("P, "S, etc.) or non-isotopic (biotin, digoxigenin, etc.).

The "sample" may be any material which may contain HPV nucleic acid, such as biological material, for example taken either directly from a human being (or animal), or after culturing (enrichment), or may be recombinant HPV nucleic acid expressed in a host cell. Biological material may be e.g. urine, or scrapes/biopsies from the urogenital tract or any part of the human or animal body.

The sets of probes of the present invention will generally include at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31,32,33, 34,35,36,37,38, 39, 40, 41, 42,43,44,45,46,47,48,49,50 or more probes.

Said probes may be applied in two or more (possibly as many as there are probes) distinct and known positions on a solid substrate. Often it is preferable to apply two or more probes o together in one and the same position of said solid support. The invention relates to a solid support attached to 1 or more probes of the present invention.

For designing probes with desired characteristics, the following useful guidelines known to the person skilled in the art can be applied.

Because the extent and specificity of hybridization reactions such as those described herein are affected by a number of factors, manipulation of one or more of those factors will determine the exact sensitivity and specificity of a particular probe, whether perfectly complementary to its target or not. The importance and effect of various assay conditions are explained further herein.

The stability of the [probe: target] nucleic acid hybrid should be chosen to be compatible with the assay conditions. This may be accomplished by avoiding long AT-rich sequences, by terminating the hybrids with G:C base pairs, and by designing the probe with an appropriate Tin. The beginning and end points of the probe should be chosen so that the length and %GC result in a Tm about 2°C higher than the temperature at which the final assay will be performed. The base composition of the probe is significant because G-C base pairs exhibit greater thermal stability as compared to A-T base pairs due to additional hydrogen bonding. Thus, hybridization involving complementary nucleic acids of higher G-C content will be more stable at higher temperatures.

Conditions such as ionic strength and incubation temperature under which a probe will be used should also be taken into account when designing a probe. It is known that the degree of hybridization will increase as the ionic strength of the reaction mixture increases, and that the thermal stability of the hybrids will increase with increasing ionic strength. On the other hand, chemical reagents, such as formamide, urea, DMSO and alcohols, which disrupt hydrogen bonds, will increase the stringency of hybridization. Destabilization of the hydrogen bonds by such reagents can greatly reduce the Tm. In general, optimal hybridization for synthetic oligonucleotide probes of about 10-50 bases in length occurs approximately 5'C below the melting temperature for a given duplex. Incubation at temperatures below the optimum may allow mismatched base sequences to hybridize and can therefore result in reduced specificity.

It is desirable to have probes which hybridize only under conditions of high stringency. Under high stringency conditions only highly complementary nucleic acid hybrids will form; hybrids without a sufficient degree of complementarity will not form. Accordingly, the stringency of the assay conditions determines the amount of complementarity needed between two nucleic acid strands forming a hybrid. The degree of stringency is chosen such as to maximize the difference in stability between the hybrid formed with the target and the nontarget nucleic acid. In the present case, single base pair changes need to be detected, which requires conditions of very high stringency.

The length of the probe sequence can also be important. In some cases, there may be several sequences from a particular region, varying in location and length, which will yield probes with the desired hybridization characteristics. In other cases, one sequence may be significantly better than another which differs merely by a single base. While it is possible for nucleic acids that are not perfectly complementary to hybridize, the longest stretch of perfectly complementary base sequence will normally primarily determine hybrid stability.

While oligonucleotide probes of different lengths and base composition may be used, preferred oligonucleotide probes of this invention are between about 5 to 50 (more particularly 10-25) bases in length and have a sufficient stretch in the sequence which is perfectly complementary to the target nucleic acid sequence.

Regions in the target DNA or RNA which are known to form strong internal structures inhibitory to hybridization are less preferred. Likewise, probes with extensive self-complementarity should be avoided. As explained above, hybridization is the association of two single strands of complementary nucleic acids to form a hydrogen bonded double strand.

It is implicit that if one of the two strands is wholly or partially involved in a hybrid that it will be less able to participate in formation of a new hybrid. There can be intramolecular and intermolecular hybrids formed within the molecules of one type of probe if there is sufficient self complementarity. Such structures can be avoided through careful probe design. By designing a probe so that a substantial portion of the sequence of interest is single stranded, the rate and extent of hybridization may be greatly increased. Computer programs are available to search for this type of interaction. However, in certain instances, it may not be possible to avoid this type of interaction.

In order to identify different HPV types with the selected set of oligonucleotide probes, any hybridization method known in the art can be used (conventional dot-blot, Southern blot, sandwich, etc.). However, in order to obtain fast and easy results if a multitude of probes are involved, a reverse hybridization format may be most convenient. In a preferred embodiment the selected probes are immobilized to a solid support in known distinct locations (dots, lines or other Figures). In another preferred embodiment the selected set of probes are immobilized to a membrane strip in a line fashion. Said probes may be immobilized individually or as mixtures to delineated locations on the solid support. A specific and very user-friendly embodiment of the above-mentioned preferential method is disclosed in Example 4 of WO9914377, which may be adapted in the present invention. The HPV polynuceleic acids can be labelled with biotin, and the hybrid can then, via a biotine-streptavidine coupling, be detected with a non-radioactive colour developing system.

The term "hybridization buffer" means a buffer allowing a hybridization reaction between the probes and the polynucleic acids present in the sample, or the amplified products, under the appropriate stringency conditions.

The term "wash solution" means a solution enabling washing of the hybrids formed under the appropriate stringency conditions.

Throughout this specification and the claims which follow, unless the context requires otherwise, the word "comprises", and variations such as "comprises" and "comprising", will be understood to imply the inclusion of a stated integer or step or group of stated integers or steps but not to the exclusion of any other integer or step or group of integers or steps. 'Comprising' also implies the inclusion of the meanings, 'consisting of and 'consisting essentially of'.

Embodiments of the invention include:
(a) A method for typing of any HPV nucleic acid possibly present in a sample, the method comprising the steps of:
   (i) contacting any such nucleic acid with at least one probe capable of specific hybridization within the B region of the HPV genome, said region being indicated in Figure 1, and
   (ii) analysing the HPV type based upon the hybridisation result so obtained.
) b A method according to statement (a) wherein the probe is capable of specific hybridization within the B region of the genome of only one HPV type.
c A method according to statement (a) wherein the probe is selected from the list consisting of the sequences listed in Table 4.
d) A method according to any preceding statement wherein any HPV nucleic acid present in the sample is amplified prior to hybridization.
(e) A method according to statement (e) wherein the amplification step uses a primer selected from the list comprising:HPV-MPF1F1, HPV-MPF1F2, HPV-MPF1F3, HPV-MPF1F4, HPV-MPF1F5, HPV-MPF1F6, HPV-MPF1F7, HPV-MPF1F8, HPV-MPF1F9, HPV-MPF1F10, HPV-MPF2R1, HPV-MPF2R2, HPV-MPF2R3, HPV-MPF2R4, HPV-MPF2R5, HPV-MPF2R6, HPV-MPF2R7, HPV-MPF2R8.
(f) A method according to any preceding statement wherein the presence of HPV nucleic acid is confirmed in the sample prior to the typing step.
(g) A method according to any preceding statement wherein the hybridisation between probe and target is carried out in the presence of a solid support.
(h) A method according to statement (h) wherein the hybridization step uses a reverse hybridization format.
(i) A method according to statement (h) wherein the probe is hybridised onto a bead.
(j) A method according to statement (j) wherein detection of hybridisation is analysed using flow cytometry.
(k) A kit comprising at least 2 primers suitable for amplification of nucleic acid from the B or D region of an HPV genome.
(l) A kit according to statement (l) wherein the primers are selected from the list consisting of HPV-MPF1F1, HPV-MPF1F2, HPV-MPF1F3, HPV-MPF1F4, HPV-MPF1F5, HPV-MPF1F6, HPV-MPF1F7, HPV-MPF1F8, HPV-MPF1F9, HPV-MPF1F10, HPV-MPF2R1, HPV-MPF2R2, HPV-MPF2R3, HPV-MPF2R4, HPV-MPF2R5, HPV-MPF2R6, HPV-MPF2R7 and HPV-MPF2R8.
(m) A kit comprising at least 2 probes capable of specific hybridization to the D region or B region of HPV genome.
(n) A kit according to statement (n) wherein the probes are any two probes selected from Table 4.
(o) A kit comprising any primer of Table 1 or 2 or any probe of Table 3 and instructions for carrying out the above methods for HPV identification and typing analysis.
(p) A kit comprising a probe capable of specific hybridization to the D region or B region of HPV genome attached to a solid support.
(q) A kit according to any of statements (l) - (q) additionally comprising any probe of Table 3.
(r) A probe suitable for use in the method of statement A, the probe being selected from Table 4.
(s) A primer suitable for use in the method of statement (e), the probe being selected from Tables 1 and 2.

### References

Baay, M.F.D., W.G.V. Quint, J. Koudstaal, H. Hollema, J.M. Duk, M.P.M. Burger, E. Stolz, and P. Herbrink. 1995. Comprehensive study of several general and type-specific primer pairs for detection of human papillomavirus DNA by PCR in paraffin-embedded cervical carcinomas. 34:745-747.
Claas, E.C.J., W.J.G. Melchers, H.C. van der Linden, J. Lindeman, and W.G. V. Quint. 1989. Human papillomavirus detection in parafinn embedded cervical carcinomas and metastases of the carcinomas by the polymerase chain reaction. Am. J. Pathol. 135:703709.
Cornelissen, M.T.E., J.G. van den Tweel, A.P.H.B. Struyk, M.F. Jebbink, M. Bri&, J. van der Noordaa, and J. ter schegget. 1989. Localization of human pappilomavirus type 16 DNA using the polymerase chain reaction in the cervix uteri of women with cervical intraepithelial neoplasia. J. Gen. Virol. 70:2555-2562.
Cox, J.Th., A.T. Lorincz, M.H. Schiffman, M.E. Sherman, A. Cullen, and R. J. Kurman. Human papillomavirus testing by hybrid capture appears to be useful in triaging women with a cytological diagnosis of atypical squamous cells of undetermined significance. Am. J. Obstet Gynecol 1995; 164: 1461-1471.
Falcinelli, C., E. Claas, B. Kleter, and W.G.V. Quint. 1992. Detection of the human papillomavirus type 16 mRNA-transcripts in cytological abnormal scrappings. J. Med. Virol. 37:93-98.
Manos, M.M., Y. Ting, D.K. Wright, A.J. Lewis, T.R. Broker, and S.M. Wolinsky. 1989. The use of polymerase chain reaction amplification for the detection of genital human papillomaviruses. Cancer Cells 7:209-214.
Saiki, R.K., D.H. Gelfland, S. Stoffel, S.J. Scharf, R. Higuchi, G.T. Horn, K.B. Mullis, and H.A. Erlich. 1988. Primer-directed enzymatic amplification of DNA with a thermostable DNA polymerase. Science 239:487- 49 1.
Sambrook et al. 1989 Molecular Cloning: A Laboratory Manual, 2nd Edition, Cold Spring Harbour Laboratory Press
Stuyver, L., R. Rossau, A. Wyseur, M. Duhamel, B. Vanderborght, H. Van Heuverswyn, and G. Maertens. 1993. Typing of hepatitis C virus isolates and characterization of new subtypes using a line probe assay. J. Gen. Virol. 74:1093-1102.
Tieben L.M., J. ter Schegget, R.P. Minnaar, J.N. Bouwes Bavinck, RIM. Berkhout, B.J. Vermeer, M.f Jebbink, and H.L. Smits. 1993. Detection of cutaneous and genital HPV types in clinical samples by PCR using consensus primers. J. Virol. Methods 42:265-280.
Van den Brule, A.J.C., P. J.F. Snijders, R.L.J. Gordijn, O.P. Bleker, C.J.L.M. Meijer, and J.M.M. Walboomers. 1990. General primer-mediated polymerase chain reaction permits the detection of sequenced and still unsequenced human papillornavirus genotypes in cervical scrapes and carcinomas. Int. J. Cancer 45:644-649.
Young, L.S., I.S. Bevan, M.A. Johnson, P.I. Blomfield, T. Bromidge, N.J. Maitland, and G.B.J. Woodman. 1989. The polymerase chain reaction: A new epidemiological tool for investigatfing cervical human papillomavirus infection. Brit. Med. J. 298:14-18.
Woodworth, C.D., Waggoner, S., Bames, W., Stoler, M.H., Di Paolo, J.A. 1990. Human cervical and foreskin eptithelial cells immortalized by human papillomavirus DNAs exhibit displastic differentiation in vivo. Cancer Res. 50: 3709-3715.
Kwok S, Kellogg DE, McKinney N, Spasic D, Goda L, Levenson C, Sninsky JJ Effects of primer-template mismatches on the polymerase chain reaction: human immunodeficiency virus type 1 model studies. Nucleic Acids Res. 1990, 25;18(4):999-1005
Saiki RK, Gelfand DH, Stoffel S, Scharf SJ, Higuchi R, Horn GT, Mullis KB, Erlich HA Primer-directed enzymatic amplification of DNA with a thermostable DNA polymerase. Science 1988,29;239(4839):487-491
Wu DY, Wallace RB
The ligation amplification reaction (LAR)--amplification of specific DNA sequences using sequential rounds of template-dependent ligation.
Genomics 1989, 4(4):560-569
Barany F The ligase chain reaction on a PCR world. PCR Methods Appl. 1991, 1(1):5-16
   Erratum in: PCR Methods Appl. 1991, 1(2):149
Guatelli JC, Whitfield KM, Kwoh DY, Barringer KJ, Richman DD, Gingeras TR. Isothermal, in vitro amplification of nucleic acids by a multienzyme reaction modelled after retroviral replication. Proc Natl Acad Sci U S A 1990, 87(5):1874-1878
   Erratum in: Proc Natl Acad Sci U S A 1990 87(19):7797
Compton J Nucleic acid sequence-based amplification. Nature 1991, 7;350(6313):91-92
Kwoh DY, David GR, Whitfield KM, Chappelle HL, DiMichele LJ, Gingeras TR Transcription-based amplification system and detection of amplified human immunodeficiency virus type 1 with a bead-based sandwich hybridization format. Proc Natl Acad Sci U S A 1989, 86(4):1173-1177
Walker GT, Fraiser MS, Schram JL, Little MC, Nadeau JG, Malinoswi DP Strand displacement amplification--an isothermal, in vitro DNA amplification technique. Nucleic Acids Res. 1992 11;20(7):1691-1696
Lomeli H, Tyagi S, Pritchard CG, Lizardi PM, Kramer FR Quantitative assays based on the use of replicatable hybridization probes. Clin Chem 1989, 35(9):1826-1831
Matsukura M, Shinozuka K, Zon G, Mitsuya H, Reitz M, Cohen JS, Broder S Phosphorothioate analogs of oligodeoxynucleotides: inhibitors of replication and cytopathic effects of human immunodeficiency virus. Proc Natl Acad Sci U S A 1987, 84(21):7706-7710
Asseline U, Delarue M, Lancelot G, Toulme F, Thuong NT, Montenay-Garestier T, Helene C Nucleic acid-binding molecules with high affinity and base sequence specificity: intercalating agents covalently linked to oligodeoxynucleotides. Proc Natl Acad Sci U S A 1984, 81(11):3297-3301
Bej AK, Steffan RJ, DiCesare J, Haff L, Atlas RM Detection of coliform bacteria in water by polymerase chain reaction and gene probes. Appl Environ Microbiol. 1990, 56(2):307-314
Bej AK, Mahbubani MH, Miller R, DiCesare JL, HaffL, Atlas RM Multiplex PCR amplification and immobilized capture probes for detection of bacterial pathogens and indicators in water. Mol Cell Probes 1990, 4(5):353-365
Remmink AJ, Walboomers JM, Helmerhorst TJ, Voorhorst FJ, Rozendaal L, Risse EK, Meiier CJ, Kenemans P. persistent high-risk HPV genotypes in dysplastic cervical lesions is associated with progressive disease: natural history up to 36 months. Int J Cancer. 1995 May 4;61(3):306-11.
Chan SY, Delius H, Halpern AL, Bernard HU. Analysis of genomic sequences of 95 papillomavirus types: uniting typing, phylogeny, and taxonomy. J Virol. 1995 May;69(5):3074-83.
de Villiers EM, Fauquet C, Broker TR, Bernard HU, zur Hausen H. Classification of papillomaviruses. Virology. 2004 Jun 20;324(1):17-27.
Altschul SF, Gish W, Miller W, Myers EW, Lipman DJ Basic local alignment search tool. J Mol Biol. 1990 Oct 5;215(3):403-10
Altschul SF, Madden TL, Schaffer AA, Zhang J, Zhang Z, Miller W, Lipman DJ. Gapped BLAST and PSI-BLAST: a new generation of protein database search programs. Nucleic Acids Res. 1997 Sep 1;25(17):3389-402.
Zella D, Cavicchini A, Cattaneo E, Cimarelli A, Bertazzoni U. Utilization of a DNA enzyme immunoassay for the detection of proviral DNA of human immunodeficiency virus type 1 by polymerase chain reaction.Clin Diagn Virol. 1995 Feb;3(2):155-64

### Example 1

The following approach can be used to type HPV DNA.

### Composition of PCR mix (amplification of HPV DNA from sample)

| **Component** | **µl per reaction** |
|---|---|
| 10x PCR buffer | 5 |
| 1 mM dNTP's | 10 |
| 25 mM MgCl₂ | 5 |
| Forward primer 20pmol/µl | 1 |
| Reverse primer 20 pmol/µl | 1 |
| AmpliTaq Gold (5U/µl) | 0.3 |
| Water | 17.7 |
| Total volume | 40 |

10 µl target DNA is added, making a final volume of 50 µl.

### Universal primers to be used

| |
|---|
| HPV-MPF1F1 (10pmol/µl) |
| HPV-MPF1F2 (10pmol/µl) |
| HPV-MPF1F3 (10pmol/µl) |
| HPV-MPF1F4 (10pmol/µl) |
| HPV-MPF1F5 (10pmol/µl) |
| HPV-MPF1F6 (10pmol/µl) |
| HPV-MPF1F7 (10pmol/µl) |
| HPV-MPF1F8 (10pmol/µl) |
| HPV-MPF1F9 (10pmol/µl) |
| HPV-MPF1F10 (10pmol/µl) |
| HPV-MPF2R1-bio (10pmol/µl) |
| HPV-MPF2R2-bio (10pmol/µl) |
| HPV-MPF2R3-bio (10pmol/µl) |
| HPV-MPF2R4-bio (10pmol/µl) |
| HPV-MPF2R5-bio (10pmol/µl) |
| HPV-MPF2R6-bio (10pmo/µl) |
| HPV-MPF2R7-bio (10pmol/µl) |
| HPV-MPF2R8-bio (10pmol/µl) |

### PCR program

9 min 94°C, activation of AmpliTaq Gold 40 cycles, comprising:
30 sec 94°C
45 sec 52°C
45 sec 72°C

Final incubation of 5 min 72°C

The following plasmids containing HPV genomic DNA have been used for multiplex PCR (complete MPF set):
- HPV 16
- HPV 18
- HPV31
- HPV33
- HPV45
- HPV52
- HPV56
- HPV66
- HPV35
- HPV67
- HPV11
- HPV26
- HPV53
- HPV58
- HPV71
- HPV13
- HPV39
- HPV54
- HPV69
- HPV70
- HPV74
- HPV7

All yielded a fragment of the expected size.

The following plasmids containing HPV genomic DNA have been used for single PCR (single forward + single reverse):
- HPV16
- HPV35
- HPV59
- HPV18
- HPV56
- HPV68
- HPV39
- HPV33
- HPV6
- HPV51
- HPV26
- HPV40
- HPV43

All yielded a fragment of the expected size.

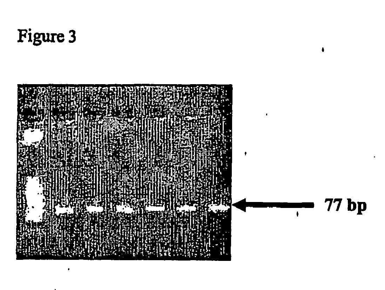

### Example of a PCR product, using single PCR primers (see figure 3)

| | |
|---|---|
| Lane 1: | marker |
| Lane 2: | HPV18 |
| Lane 3: | HPV56 |
| Lane 4: | HPV39 |
| Lane 5: | HPV26 |
| Lane 6: | HPV43 |
| Lane 7: | HPV33 |

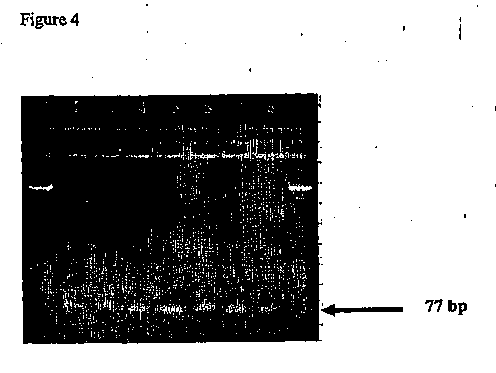

### Gel multiplex PCR (see fig 4)

| | |
|---|---|
| Lane 1: | marker |
| Lane 2: | HPV16 |
| Lane 3: | HPV18 |
| Lane 4: | HPV31 |
| Lane 5: | HPV33 |
| Lane 6: | HPV45 |
| Lane 7: | HPV52 |
| Lane 8: | HPV56 |
| Lane 9: | marker |

### Reverse hybridisation (line probe assay) conditions

10 µl of a PCR product can be hybridized to a strip, containing some of the selected probes. Suitable conditions to be used are as follows:

### Reverse hybridization profile:

| **Step** | **Temperature** | **Incubation time** |
|---|---|---|
| Denaturation | Room temp | 10 min |
| Hybridization | 50°C | 60 min |
| Stringent wash | 50°C | 30 min |
| Conjugate | Room temp | 30 min |
| Substrate | Room temp | 30 min |

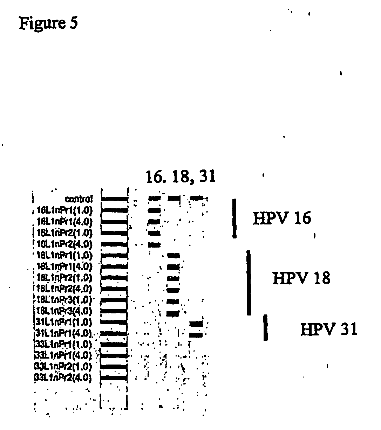

Hybridisation is suitably carried out at 3X SSC, 0.1% SDS, 50 °C. The results in Figure 5 were obtained.

### TABLES

### General primer set

**Table 1. Forward primers (MPF for)**

| Name | **sequence 5' → 3'** | **SEQ ID NO:** |
|---|---|---|
| HPV-MPF1F1 | GATGCCCAAATATTCAATAAACC | 1 |
| HPV-MPF1F2 | GATGCICAAATATTTAATAAACC | 2 |
| HPV-MPF1F3 | GAITCICAATTATTTAATAAACC | 3 |
| HPV-MPF1F4 | GAIGCICAGTTGTTTAATAAACC | 4 |
| HPV-MPF1F5 | GATTCICAATTGTTTAACAAACC | 5 |
| HPV-MPF1F6 | GAITCICAGTTATTTAACAAGCC | 6 |
| HPV-MPF1F7 | GAITCICAGTTATTTAATAAGCC | 7 |
| HPV-MPF1F8 | GAIGCICAATTGTTTAATAAGCC | 8 |
| HPV-MPF1F9 | GAITCICAATTATTTAATAAGCC | 9 |
| HPV-MPF1F10 | GATTCTCAAATTTTTAATAAGCC | 10 |

**Table 2. Reverse primers (MPF rev)**

| Name | **sequence 5' → 3'** | **SEQ ID NO:** |
|---|---|---|
| HPV-MPF2R1 | TTICCCCAICAAATGCCATT | 11 |
| HPV-MPF2R2 | TTITTCCAICAAATGCCATT | 12 |
| HPV-MPF2R3 | TTICCAAAACAAATGCCATT | 13 |
| HPV-MPF2R4 | TCATTAAACCAACAAATGCCATT | 14 |
| HPV-MPF2R5 | TGATTAAACCAICAAATACCATT | 15 |
| HPV-MPF2R6 | TTATGCCAGCAAACACCATT | 16 |
| HPV-MPF2R7 | TGATTATGCCAACAIATACCATT | 17 |
| HPV-MPF2R8 | TTICCCCAACAIATACCATT | 18 |

**Universal probes for general detection of MPF amplimers**

**Table 3. DEIA probes**

| **Probe name** | **Sequence 5'-3'** | **Start position in** **Figure 1** | **SEQ ID NO:** |
|---|---|---|---|
| HPV MPF P1 | AAGCCITAITGGCTGCA | 19 | 19 |
| HPV MPF P1-2 | AAIAAGCCITAITGGCTGCA | 16 | 20 |
| HPV MPF P1-3 | TTTAAIAAGCCITAITGGCTGCA | 13 | 21 |
| HPV MPF P2 | TGGATICAAAAIGCCCAGG | 28 | 22 |
| HPV MPF P2-2 | TGGATICAAAAIGCCCAGGG | 28 | 23 |
| HPV MPF P3 | TTTAATAAACCATATTGGITGCAA | 13 | 24 |
| HPV MPF P4 | TTTAATAAACCATATTGGTTACA | 13 | 25 |
| HPV MPF P5 | TTTAATAAICCTTATTGGTTGCA | 13 | 26 |
| HPV MPF P6 | TTTAATAAGCCITAITGGTTACA | 13 | 27 |
| HPV MPF P6-2 | TTTAATAAGCCITAITGGTTACAA | 13 | 28 |
| HPV MPF P7 | AATAAGCCITATTGGCTACA | 16 | 29 |
| HPV MPF P7-2 | TTTAATAAGCCITATTGGCTACA | 13 | 30 |
| HPV MPF P8 | AATAAACCTTATTGGTTACAACGA | 16 | 31 |

**Table 4 - Type-specific probes. Type-specific probes Probe sequence 5' → 3'**

| Probe name | Sequence 5'-3' | Polarity | Start postion in Figure 1 | length | SEQ ID NO: |
|---|---|---|---|---|---|
| 11L1nPr1 | GGCTTCAAAAGGCTCAG | + | 29 | 17 | 32 |
| 13L1nPr1 | ATTGGTTACAAAAGGCC | + | 26 | 17 | 33 |
| 13L1nPr2 | TGGTTACAAAAGGCCC | + | 28 | 16 | 34 |
| 16L1nPr1 | TTATTGGTTACAACGAGCA | + | 24 | 19 | 35 |
| 16L1nPr2 | TTATTGGTTACAACGAGC | + | 24 | 18 | 36 |
| 16L1nPr3 | CTTATTGGTTACAACGAG | + | 23 | 18 | 37 |
| 18L1nPr1 | AGGCACAGGGTCATAAC | + | 38 | 17 | 38 |
| 18L1nPr2 | AGGCACAGGGTCATAAg | + | 38 | 17 | 39 |
| 18L1nPr3 | AAGGCACAGGGTCATAAg | + | 37 | 18 | 40 |
| 18L1nPr4 | GTTACATAAGGCACAGG | + | 30 | 17 | 41 |
| 26L1nPr1 | GTGCACAGGGTCATAAT | + | 38 | 17 | 42 |
| 26L1nPr2 | TGGTTACAACGTGCACA | + | 28 | 17 | 43 |
| 30L1nPr1 | TACTGGTTGCAACGCG | + | 25 | 16 | 44 |
| 30L1nPr2 | TTACTGGTTGCAACGCG | + | 24 | 17 | 45 |
| 31L1nPr1 | GGATGCAACGTGCTCA | + | 29 | 16 | 46 |
| 31L1nPr2 | GGATGCAACGTGCTC | + | 29 | 15 | 47 |
| 32L1nPr1 | ACAGCAGGCACAAGGC | + | 33 | 16 | 48 |
| 33L1nPr1 | CATATTGGCTACAACGTG | + | 23 | 18 | 49 |
| 33L1nPr2 | CCATATTGGCTACAACG | + | 22 | 17 | 50 |
| 33L1nPr3 | CCATATTGGCTACAACGa | + | 22 | 18 | 51 |
| 34L1nPr1 | CCCAGGGACAAAACAA | + | 41 | 16 | 52 |
| 35L1nPr1 | AACCATATTGGTTGCAAC | + | 20 | 18 | 53 |
| 35L1nPr2 | TTGCAACGTGCACAAG | + | 31 | 16 | 54 |
| 35L1nPr3 | ACCATATTGGTTGCAAC | + | 21 | 17 | 55 |
| 39L1nPr1 | CCTTATTGGCTACATAAGG | + | 22 | 19 | 56 |
| 39L1nPr2 | CTTATTGGCTACATAAGG | + | 23 | 18 | 57 |
| 40L1nPr1 | AAGCCATTGTGGATACAA | + | 19 | 18 | 58 |
| 42L1nPr1 | CAACAAGCACAAGGACA | + | 34 | 17 | 59 |
| 43L1nPr2 | AACCCTTATGGATACAAAAG | + | 20 | 20 | 60 |
| 43L1Pr1 | AACCCTTATGGATACAAAA | + | 20 | 19 | 61 |
| 44L1nPr1 | AAGGCGCAGGGCCAC | + | 37 | 15 | 62 |
| 44L1nPr2 | TTTTGGTTGCAAAAGGC | + | 25 | 17 | 63 |
| 45L1nPr1 | GGTTACATAAGGCCCAG | + | 29 | 17 | 64 |
| 45L1nPr2 | GGTTACATAAGGCCCA | + | 29 | 16 | 65 |
| 45L1nPr3 | AGCCCAGGGCCATAAg | + | 39 | 16 | 66 |
| 45L1nPr4 | CCCAGGGCCATAACA | + | 41 | 15 | 67 |
| 45L1nPr5 | CCAGGGCCATAACAAg | + | 42 | 16 | 68 |
| 51L1nPr1 | TATTGGCTCCACCGTG | + | 25 | 16 | 69 |
| 51L1nPr2 | TTATTGGCTCCACCGT | + | 24 | 16 | 70 |
| 51L1nPr3 | ATTGGCTCCACCGTG | + | 26 | 15 | 71 |
| 52L1nPr1 | CGTACTGGTTACAACGTG | + | 23 | 18 | 72 |
| 52L1nPr2 | CCGTACTGGTTACAACGa | + | 22 | 18 | 73 |
| 52L1nPr3 | GCCGTACTGGTTACAAC | + | 21 | 17 | 74 |
| 53L1nPr1 | ACGTGCCCAGGGACAT | + | 37 | 16 | 75 |
| 54L1nPr1 | GCCCAGGGTCAAAACA | + | 40 | 16 | 76 |
| 54L1nPr2 | ACTGGTTACAACGGGC | + | 26 | 16 | 77 |
| 55L1nPr1 | TTTTTGGTTGCAAAGGG | + | 24 | 17 | 78 |
| 55L1nPr2 | TTTTGGTTGCAAAGGGC | + | 25 | 17 | 79 |
| 56L1nPr1 | CCCAAGGCCATAATAAT | + | 41 | 17 | 80 |
| 56L1nPr2 | GCCCAAGGCCATAATA | + | 40 | 16 | 81 |
| 56L1nPr3 | TGCCCAAGGCCATAAT | + | 39 | 16 | 82 |
| 56L1nPr4 | GCCCAAGGCCATAATAAg | + | 40 | 18 | 83 |
| 57L1nPr1 | TTACTGGCTGCGGAGG | + | 24 | 16 | 84 |
| 58L1nPr1 | CTTATTGGCTACAGCGT | + | 23 | 17 | 85 |
| 58L1nPr2 | CTTATTGGCTACAGCGTG | + | 23 | 18 | 86 |
| 59L1nPr1 | AAGGCTCAGGGTTTAAAC | + | 37 | 18 | 87 |
| 66L1nPr1 | TTGCAACGTGCACAGG | + | 31 | 16 | 88 |
| 66L1nPr2 | TGCAACGTGCACAGG | + | 32 | 15 | 89 |
| 67L1nPr1 | CAACGCGCACAAGGTC | + | 34 | 16 | 90 |
| 67L1nPr2 | ACAACGCGCACAAGGT | + | 33 | 16 | 91 |
| 68L1nPr1 | GGCACAGGGACACAAC | + | 39 | 16 | 92 |
| 68L1nPr2 | GGCACAGGGACACAAg | + | 39 | 16 | 93 |
| 69L1nPr1 | GGTTACAGCGTGCCCA | + | 29 | 16 | 94 |
| 6L1nPr1 | GGCTACAAAAAGCCCAG | + | 29 | 17 | 95 |
| 6L1nPr2 | TGGCTACAAAAAGCCCA | + | 28 | 17 | 96 |
| 70L1nPr1 | CCTATTGGTTGCATAAGG | + | 23 | 18 | 97 |
| 70L1nPr2 | TATTGGTTGCATAAGGC | + | 25 | 17 | 98 |
| 70L1nPr3 | CCCTATTGGTTGCATAA | + | 22 | 17 | 99 |
| 71L1nPr1 | GCCTTACTGGCTACAAC | + | 21 | 17 | 100 |
| 72L1nPr1 | CTATTGGCTACAGCGC | + | 24 | 16 | 101 |
| 72L1nPr2 | CGCCCAGGGTCACAA | + | 39 | 15 | 102 |
| 73L1nPr1 | GCACAGGGACAAAATAA | + | 40 | 17 | 103 |
| 74L1nPr1 | CCTTTTGGCTACAAAAGG | + | 23 | 18 | 104 |
| 7L1nPr1 | AACCTTTGTGGATACAAAA | + | 20 | 19 | 105 |
| 81L1nPr1 | GCTACAACGGGCACAG | + | 30 | 16 | 106 |
| 81L1nPr2 | CCTTATTGGCTACAACG | + | 22 | 17 | 107 |
| 82L1nPr1 | TTATTGGTTGCATCGCG | + | 24 | 17 | 108 |
| 83L1nPr1 | TACTGGCTGCATCGTG | + | 25 | 16 | 109 |
| 89L1nPr1 | TACTGGTTGCAAAAGGC | + | 25 | 17 | 110 |
| 85L1nPr1 | CTGCACAAAGCCCAGG | + | 31 | 16 | 111 |
| 85L1nPr2 | CTGCACAAAGCCCAG | + | 31 | 15 | 112 |
| 85L1nPr3 | TGCACAAAGCCCAGG | + | 32 | 15 | 113 |
| 86L1nPr1 | GGTTACAGAAGGCGCA | + | 29 | 16 | 114 |
| 87L1nPr1 | TATTGGCTGCAGCGGG | + | 25 | 16 | 115 |
| 89L10nPr1 | TATTGGCTGCACCGTG | + | 25 | 16 | 116 |
| 90L1nPr1 | TACTGGCTGCAACGAG | + | 25 | 16 | 117 |
| 91L1nPr1 | AACCGCTTTGGATGCAA | + | 20 | 17 | 118 |

| | | | | | |
|---|---|---|---|---|---|
| Lower case nucleotide is not HPV specific | | | | | |

**Additional information indicating those probes listed above which can be T-tailed at the 3' end, if desired.**

| **name** | **Probe sequence** | **start** | **length** | **T-tail** | **SEQ ID NO:** |
|---|---|---|---|---|---|
| 11L1nPr1 | GGCTTCAAAAGGCTCAG | 29 | 17 | | 32 |
| 13L1nPr1 | ATTGGTTACAAAAGGCC | 26 | 17 | | 33 |
| 13L1nPr2 | TGGTTACAAAAGGCCC | 28 | 16 | | 34 |
| 16AF1L1p1.CH | ggtGTTGCAACGAGCACA | 27 | 15 | | 281 |
| 16AF1L1p2.CH | ggGGTTGCAACGAGCAC | 27 | 15 | | 282 |
| 16AF1L1p3.CH | ATATTGGTTGCAACGAG | 24 | 17 | | 283 |
| 16AF1L1p4.CH | cTATTGGTTGCAACGAG | 24 | 16 | | 284 |
| 16AF1L1p5.CH | TTGGTTGCAACGAGC | 27 | 15 | **3' 100xT** | 285 |
| 16AF1L1p6.CH | GGTTGCAACGAGCA | 29 | 14 | **3' 100xT** | 286 |
| 16AF1L1p7.CH | TGGTTGCAACGAGC | 28 | 14 | **3' 100xT** | 287 |
| 16L1nPr1 | TTATTGGTTACAACGAGCA | 24 | 19 | | 35 |
| 16L1nPr2.CH | TTATTGGTTACAACGAGC | 24 | 18 | | 36 |
| 16L1nPr3.CH | CTTATTGGTTACAACGAG | 23 | 18 | | 37 |
| 16L1nPr4.CH | GAGCACAGGGCCAC | 38 | 14 | **3' 100xT** | 288 |
| 16L1nPr5.CH | AGCACAGGGCCACA | 39 | 14 | **3' 100xT** | 289 |
| 18L1nPr1 | AGGCACAGGGTCATAAC | 38 | 17 | | 38 |
| 18L1nPr2 | AGGCACAGGGTCATAAg | 38 | 16 | | 39 |
| 18L1nPr3 | AAGGCACAGGGTCATAAg | 37 | 17 | | 40 |
| 18L1nPr4 | GTTACATAAGGCACAGG | 30 | 17 | | 41 |
| 18L1nPr4.CH | agtGTTACATAAGGCACAGG | 27 | 17 | | 290 |
| 18L1nPr5.CH | agttTTACATAAGGCACAGG | 27 | 16 | | 291 |
| 18L1nPr6.CH | ccccTTACATAAGGCACAGG | 27 | 16 | | 292 |
| 18L1nPr7.CH | TTACATAAGGCACAGG | 31 | 16 | **3' 100xT** | 293 |
| 26L1nPr2 | TGGTTACAACGTGCACA | 28 | 17 | | 43 |
| 26L1nPr1.CH | GTGCACAGGGTCATAAT | 38 | 17 | | 294 |
| 26L1nPr3.CH | GTGCACAGGGTCATAA | 38 | 16 | | 295 |
| 26L1nPr4.CH | ACGTGCACAGGGTC | 36 | 15 | | 296 |
| 26L1nPr5.CH | TGCACAGGGTCATAATA | 39 | 17 | **3' 100xT** | 297 |
| 26L1nPr6.CH | TGCACAGGGTCATAAT | 39 | 16 | **3' 100xT** | 298 |
| 26L1nPr7.CH | GTTACAACGTGCACAG | 30 | 16 | **3' 100xT** | 299 |
| 30L1nPr1 | TACTGGTTGCAACGCG | 25 | 16 | | 44 |
| 30L1nPr2 | TTACTGGTTGCAACGCG | 24 | 17 | | 45 |
| 31L1nPr1 | GGATGCAACGTGCTCA | 29 | 16 | | 46 |
| 31L1nPr2 | GGATGCAACGTGCTC | 29 | 15 | | 47 |
| 31L1nPr3.CH | ggGGATGCAACGTGCTC | 27 | 15 | | 300 |
| 31L1nPr4.CH | ACCATATTGGATGCAAC | 21 | 17 | | 301 |
| 31L1nPr5.CH | CATATTGGATGCAACG | 23 | 16 | | 302 |
| 31L1nPr6.CH | GGATGCAACGTGCTC | 29 | 15 | **3' 100xT** | 303 |
| 32L1nPr1 | ACAGCAGGCACAAGGC | 33 | 16 | | 48 |
| 33L1nPr1 | CATATTGGCTACAACGTG | 23 | 18 | | 49 |
| 33L1nPr2 | CCATATTGGCTACAACG | 22 | 17 | | 50 |
| 33L1nPr3 | CCATATTGGCTACAACGa | 22 | 17 | | 51 |
| 33L1nPr3.CH | CCATATTGGCTACAACG | 22 | 17 | | 304 |
| 33L1nPr4.CH | CATATTGGCTACAACGT | 23 | 17 | | 305 |
| 34L1nPr1 | CCCAGGGACAAAACAA | 41 | 16 | | 52 |
| 35L1nPr1 | AACCATATTGGTTGCAAC | 20 | 18 | | 53 |
| 35L1nPr2.CH | TTGCAACGTGCACAAG | 31 | 16 | | 54 |
| 35L1nPr3.CH | ACCATATTGGTTGCAAC | 21 | 17 | | 55 |
| 35L1nPr4.CH | GTGCACAAGGCCATAAg | 38 | 16 | **3' 100xT** | 306 |
| 35L1nPr5.CH | TTGCAACGTGCACAAG | 31 | 16 | **3' 100xT** | 307 |
| 35L1nPr6.CH | GTGCACAAGGCCATA | 38 | 15 | **3' 100xT** | 308 |
| 35L1nPr7.CH | TGCACAAGGCCATA | 39 | 14 | **3' 100xT** | 309 |
| 39L1nPr1 | CCTTATTGGCTACATAAGG | 22 | 19 | | 56 |
| 39L1nPr2 | CTTATTGGCTACATAAGG | 23 | 18 | | 57 |
| 39L1nPr3.CH | AGCCTTATTGGCTACATAA | 20 | 19 | | 310 |
| 39L1nPr4.CH | GCCTTATTGGCTACATAA | 21 | 18 | | 311 |
| 39L1nPr5.CH | AAGCCTTATTGGCTACATAAc | 19 | 20 | **3' 100xT** | 312 |
| 39L1nPr6.CH | GCCTTATTGGCTACATAAG | 21 | 19 | | 313 |
| 40L1nPr1 | AAGCCATTGTGGATACAA | 19 | 18 | | 58 |
| 42L1nPr1 | CAACAAGCACAAGGACA | 34 | 17 | | 314 |
| 43L1nPr1 | AACCCTTATGGATACAAAA | 20 | 19 | | 315 |
| 43L1nPr2 | AACCCTTATGGATACAAAAG | 20 | 20 | | 60 |
| 44L1nPr1 | AAGGCGCAGGGCCAC | 37 | 15 | | 62 |
| 44L1nPr2 | TTTTGGTTGCAAAAGGC | 25 | 17 | | 63 |
| 45L1nPr1 | GGTTACATAAGGCCCAG | 29 | 17 | | 64 |
| 45L1nPr2 | GGTTACATAAGGCCCA | 29 | 16 | | 65 |
| 45L1nPr3 | AGCCCAGGGCCATAAg | 39 | 15 | | 66 |
| 45L1nPr4 | CCCAGGGCCATAACA | 41 | 15 | | 67 |
| 45L1nPr5 | CCAGGGCCATAACAAg | 42 | 15 | | 68 |
| 45L1nPr6.CH | ggtGTTACATAAGGCCCAG | 27 | 16 | | 316 |
| 45L1nPr7.CH | CCAGGGCCATAACAA | 42 | 15 | | 317 |
| 45L1nPr8.CH | CCAGGGCCATAACAAg | 42 | 15 | **3' 100xT** | 318 |
| 45L1nPr9.CH | AAGCCATATTGGTTACATA | 19 | 19 | **3' 100xT** | 319 |
| 45L1nPr10.CH | TTACATAAGGCCCAGG | 31 | 16 | **3' 100xT** | 320 |
| 51L1nPr1 | TATTGGCTCCACCGTG | 25 | 16 | | 69 |
| 51L1nPr3 | ATTGGCTCCACCGTG | 26 | 15 | | 71 |
| 51L1nPr2.CH | TTATTGGCTCCACCGT | 24 | 16 | | 321 |
| 51L1nPr4.CH | ggATTGGCTCCACCGTG | 24 | 15 | | 322 |
| 52L1nPr1 | CGTACTGGTTACAACGTG | 23 | 18 | | 72 |
| 52L1nPr2 | CCGTACTGGTTACAACGa | 22 | 17 | | 73 |
| 52L1nPr3a | GCCGTACTGGTTACAAC | 21 | 17 | | 323 |
| 52L1nPr3.CH | CCGTACTGGTTACAAC | 22 | 16 | | 324 |
| 52L1nPr4.CH | ACCGTACTGGTTACAAC | 21 | 17 | | 325 |
| 53L1nPr1.CH | ACGTGCCCAGGGACAT | 36 | 16 | | 326 |
| 53L1nPr2.CH | AACGTGCCCAGGGAC | 35 | 15 | | 327 |
| c53L1nPr3.CH | ACGTGCCCAGGGAC | 36 | 14 | | 328 |
| 53L1nPr4.CH | TGCCCAGGGACATA | 39 | 14 | **3' 100xT** | 329 |
| 53L1nPr5.CH | GCCCAGGGACATAAT | 40 | 15 | **3' 100xT** | 330 |
| 53L1CPr6.CH | ATATTGGCTGCAACGT | 24 | 16 | | 331 |
| 53L1CPr7 | TATTGGCTGCAACGT | 25 | 15 | | 332 |
| 54L1nPr1 | GCCCAGGGTCAAAACA | 40 | 16 | | 76 |
| 54L1nPr2 | ACTGGTTACAACGGGC | 26 | 16 | | 77 |
| 55L1nPr1 | TTTTTGGTTGCAAAGGG | 24 | 17 | | 78 |
| 55L1nPr2 | TTTTGGTTGCAAAGGGC | 25 | 17 | | 78 |
| 56L1nPr1 | CCCAAGGCCATAATAAT | 41 | 17 | | 80 |
| 56L1nPr2 | GCCCAAGGCCATAATA | 40 | 16 | | 81 |
| 56L1nPr3 | TGCCCAAGGCCATAAT | 39 | 16 | | 82 |
| 56L1nPr4 | GCCCAAGGCCATAATAAg | 40 | 17 | | 83 |
| 56L1nPr4.CH | gGCCCAAGGCCATAATAA | 39 | 17 | | 333 |
| 56L1nPr5.CH | gGCCCAAGGCCATAATA | 39 | 16 | | 334 |
| 56L1nPr6.CH | TGCCCAAGGCCATAAT | 39 | 16 | | 335 |
| 57L1nPr1 | TTACTGGCTGCGGAGG | 24 | 16 | | 84 |
| 58L1nPr2 | CTTATTGGCTACAGCGTG | 23 | 18 | | 86 |
| 58L1riPr1.CH | CTTATTGGCTACAGCGT | 23 | 17 | | 336 |
| 58L1nPr3.CH | CTTATTGGCTACAGCG | 23 | 16 | | 337 |
| 59L1nPr1 | AAGGCTCAGGGTTTAAAC | 37 | 18 | | 87 |
| 59Pr2.CH | CAAGGCTCAGGGTTTAAA | 36 | 18 | | 338 |
| 59L1nPr3.CH | CAAGGCTCAGGGTTTAA | 36 | 17 | | 339 |
| 61L1nCPr1 | AGGGCCACAACAATG | 44 | 15 | | 340 |
| 61L1nCPr2 | GGGCCACAACAATG | 45 | 14 | | 341 |
| 66L1nPr1 | TTGCAACGTGCACAGG | 31 | 16 | | 88 |
| 66L1nPr2 | TGCAACGTGCACAGG | 32 | 15 | | 89 |
| 66L1nPr2.CH | gTGCAACGTGCACAGG | 31 | 15 | | 342 |
| 66L1nPr3.CH | ggGCAACGTGCACAGG | 31 | 14 | | 343 |
| 66L1nPr4.CH | TGCAACGTGCACAGG | 32 | 15 | **3' 100xT** | 344 |
| c66L1nPr5.CH | GCAACGTGCACAGG | 33 | 14 | **3' 100xT** | 345 |
| 66L1nPr6.CH | TGCACAGGGCCATA | 39 | 14 | **3' 100xT** | 346 |
| 66L1nPr7.CH | TGCAACGTGCACAG | 32 | 14 | **3' 100xT** | 347 |
| 67L1nPr1 | CAACGCGCACAAGGTC | 34 | 16 | | 90 |
| 67L1nPr2 | ACAACGCGCACAAGGT | 33 | 16 | | 91 |
| 68L1nPr1 | GGCACAGGGACACAAC | 39 | 16 | | 92 |
| 68L1nPr2 | GGCACAGGGACACAAg | 39 | 15 | | 93 |
| 68L1nPr2.CH | GGCACAGGGACACAA | 39 | 15 | | 348 |
| 68L1nPr3.CH | AGGCACAGGGACACA | 38 | 15 | | 349 |
| 68L1nPr4.CH | GGCACAGGGACACA | 39 | 14 | | 350 |
| 68L1nPr5.CH | GGCACAGGGACACA | 39 | 14 | **3' 100xT** | 351 |
| 68L1nPr6.CH | CCCTATTGGCTGCAC | 22 | 15 | **3' 100xT** | 352 |
| 68L1nPr7.CH | GCTGCACAAGGCACA | 30 | 15 | **3' 100xT** | 353 |
| 68L1nPr8.CH | CTGCACAAGGCACAG | 31 | 15 | **3' 100xT** | 354 |
| 68L1nPr9.CH | GCTGCACAAGGCAC | 30 | 14 | **3' 100xT** | 355 |
| 68L1nPr10.CH | GCACAAGGCACAGG | 33 | 14 | **3' 100xT** | 356 |
| 69L1nPr1 | GGTTACAGCGTGCCCA | 29 | 16 | | 94 |
| 6L1nPr1 | GGCTACAAAAAGCCCAG | 29 | 17 | | 95 |
| 6L1nPr2 | TGGCTACAAAAAGCCCA | 28 | 17 | | 96 |
| 70L1nPr1 | CCTATTGGTTGCATAAGG | 23 | 18 | | 97 |
| 70L1nPr2 | TATTGGTTGCATAAGGC | 25 | 17 | | 98 |
| 70L1nPr3.CH | CCCTATTGGTTGCATAA | 22 | 17 | | 357 |
| 70L1nPr4.CH | CCTATTGGTTGCATAAGG | 23 | 18 | | 358 |
| 71L1nPr1 | GCCTTACTGGCTACAAC | 21 | 17 | | 100 |
| 72L1nPr1 | CTATTGGCTACAGCGC | 24 | 16 | | 101 |
| 72L1nPr2 | CGCCCAGGGTCACAA | 39 | 15 | | 102 |
| 73L1nPr1 | GCACAGGGACAAAATAA | 40 | 17 | | 103 |
| 74L1nPr1 | CCTTTTGGCTACAAAAGG | 23 | 18 | | 104 |
| 7L1nPr1 | AACCTTTGTGGATACAAAA | 20 | 19 | | 105 |
| 81L1nPr1 | GCTACAACGGGCACAG | 30 | 16 | | 106 |
| 81L1nPr2 | CCTTATTGGCTACAACGn | 22 | 17 | | 107 |
| 82L1nPr1 | TTATTGGTTGCATCGCG | 24 | 17 | | 108 |
| 82L1nPr2.CH | gTATTGGTTGCATCGCG | 24 | 16 | | 359 |
| 82L1nPr3.CH | ATTGGTTGCATCGCG | 26 | 15 | **3' 100xT** | 360 |
| 83L1nPr1 | TACTGGCTGCATCGTG | 25 | 16 | | 109 |
| 84L1nPr1 | TACTGGTTGCAAAAGGC | 25 | 17 | | 110 |
| 85L1nPr1 | CTGCACAAAGCCCAGG | 31 | 16 | | 111 |
| 85L1nPr2 | CTGCACAAAGCCCAG | 31 | 15 | | 112 |
| 85L1nPr3 | TGCACAAAGCCCAGG | 32 | 15 | | 113 |
| 86L1nPr1 | GGTTACAGAAGGCGCA | 29 | 16 | | 114 |
| 87L1nPr1 | TATTGGCTGCAGCGGG | 25 | 16 | | 115 |
| 89L1nPr1 | TATTGGCTGCACCGTG | 25 | 16 | | 116 |
| 90L1nPr1 | TACTGGCTGCAACGAG | 25 | 16 | | 117 |
| 91L1nPr1 | AACCGCTTTGGATGCAA | 20 | 17 | | 118 |

| | | | | | |
|---|---|---|---|---|---|
| Lower case nt is not specific | | | | | |

### Introduction examples 2 - 12

### MATERIALS & METHODS:

**Standard hybridization procedure (step-wise) according to Wallace et al (2005) *supra* is as follows:**
1. Select the appropriate oligonucleotide-coupled microsphere sets.
2. Resuspend the microspheres by vortex and sonication for approximately 20 seconds.
3. Prepare a Working Microsphere Mixture by diluting coupled microsphere stocks to 150 microspheres of each set/µl in 1.5x TMAC (1x TMAC = 2mol/l TMAC / 0.15% Sarkosyl / 75mmol/l Tris, 6mmol/l EDTA) Hybridization Buffer (Note: 33 µl of Working Microsphere Mixture is required for each reaction)
4. Mix the Working Microsphere Mixture by vortex and sonication for approximately 20 seconds.
5. To each sample or background well, add 33 µl of Working Microsphere Mixture.
6. To each background well, add 17 µl dH₂O.
7. To each sample well add amplified biotinylated DNA and dH₂O to a total volume of 17 µl (Note: 7 µl of a PCR reaction is used for detection).
8. Mix reaction wells gently by pipetting up and down several times.
9. Incubate at 99°C for 5 minutes to denature the amplified biotinylated DNA in a thermocycler.
10. Incubate the reaction plate at hybridization temperature (55°C) for 15 minutes.
11. During incubation, prepare a filter plate by rinsing twice with ice cold 1 x TMAC. Next, fill each well of the filter plate with ice cold 1x TMAC.
12. During incubation, prepare fresh reporter mix by diluting streptavidin-R-phycoerythrin to 2µg/ml in 1x TMAC hybridization buffer (Note: 75 µl of reporter mix is required for each reaction), and place it in an oven or water bath at the hybridization temperature.
13. Terminate the hybridization reaction by transferring the entire reaction to the filter plate containing ice cold wash buffer.
14. After transfer, wash the filter plate stringently twice with ice cold 1x TMAC wash buffer by intervening vacuum filtration.
15. Add 75 µl of reporter mix to each well and mix gently by pipetting up and down several times.
16. The entire plate is allowed to reach room temperature for approximately 30 minutes.
17. Incubate the reaction plate at hybridization temperature for 30 minutes.
18. Terminate the incubation by vacuum filtration.
19. Wash twice with 1x TMAC wash buffer by intervening vacuum filtration.
20. Dissolve a reaction in with 1x TMAC wash buffer by intervening vacuum filtration.
21. Analyze at room temperature on the Luminex™ 100 analyzer according to the system manual.

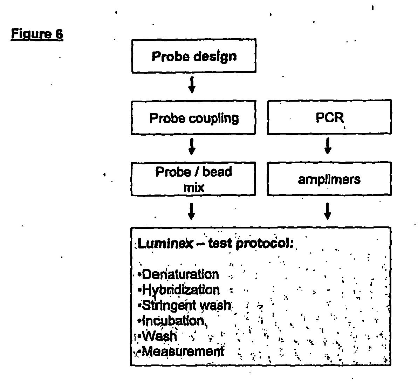

### [See Figure 6. General schematic overview of the work-flow as described by Wallace et al (2005)]

The sensitivity and specificity of the test is based on specific hybridization between probe and target nucleic acid sequences. Therefore, the hybridization and wash but also the incubation with PE appeared to be crucial steps in the procedure. The protocol was adapted in order to maximize the specificity and sensitivity of the reaction, by optimizing different parameters, such as temperatures and diffusion kinetics. These adaptations are indicated in the optimized hybridization protocol (see below).

### Materials:

### A. Buffers

| **0.1 M MES pH 4.5** | **(COUPLING BUFFER)** | | |
|---|---|---|---|
| **Reagent** | **Catalog Number** | **Final Concentration** | **Amount/ 250 ml** |
| MES (2[N-Morpholino] ethanesulfonic acid) | Sigma M-2933 | 0.1 M | 4.88 g |
| dH₂O | - | - | Up to 250ml |
| 5 N NaOH | Fisher SS256-500 | ------ | ∼ 5 drops |

| | | | |
|---|---|---|---|
| **Filter (45µm) Sterilize and store at 4°C** | | | |

| **0.02% TWEEN** | **(WASH BUFFER I)** | | |
|---|---|---|---|
| **Reagent** | **Catalog Number** | **Final Concentration** | **Amount/ 250 ml** |
| TWEEN 20 (Polyoxyethylenesorbitan monolaurate) | Sigma P-9416 | 0.02% | 50 µl |
| dH₂O | ------ | ------ | 250 ml |

| | | | |
|---|---|---|---|
| **Filter (45µm) Sterilize and store at Room Temperature** | | | |

| **20% Sarkosyl** | | | |
|---|---|---|---|
| **Reagent** | **Catalog Number** | **Final Concentration** | **Amount/ 250 ml** |
| Sarkosyl (N-Lauroylsarcosine) | Sigma L-9150 | 20% | 50g |
| dH₂O | ------ | ------ | 250 ml (adjust to) |

| | | | |
|---|---|---|---|
| **Filter (45µm) Sterilize and store at Room Temperature** | | | |

| **TE pH 8.0** | **(SAMPLE DILUENT)** | | |
|---|---|---|---|
| **Reagent** | **Catalog Number** | **Final Concentration** | **Amount/ 250 ml** |
| Tris EDTA Buffer pH 8.0 100X | Sigma T-9285 | 1 X | 2.5 ml |
| dH₂O | ------ | ------ | 247.5 ml |

| | | | |
|---|---|---|---|
| **Filter (45µm) Sterilize and store at Room Temperature** | | | |

| **4.5x SSC / 0.15%Sarkosyl Hybridization Buffer (MICROSPHERE DILUENT)** | | | |
|---|---|---|---|
| **Reagent** | **Catalog Number** | **Final Concentration** | **Amount/ 50 ml** |
| 20x SSC (3M Sodium chloride, 0.3M Sodium citrate dehydrate, pH 7.0) | Cambrex US51232 | 4.5x | 11.25 ml |
| 20% Sarkosyl | ------ | 0.15% | 0.375 ml |
| dH₂O | ------ | ------ | 38.375 ml |

| | | | |
|---|---|---|---|
| **Filter (45µm) Sterilize and store at Room Temperature** | | | |

| **3x SSC / 0.1%Sarkosyl / 1mg/ml Casein Stringent Wash Buffer** | | | |
|---|---|---|---|
| **Reagent** | **Catalog Number** | **Final Concentration** | **Amount/ 50 ml** |
| 20x SSC | Cambrex US51232 | 3x | 7.5 ml |
| 20% Sarkosyl | ------ | 0.1% | 0.250 ml |
| 50mg/ml Casein (pH7.2) | VWR BDHA440203H | ------ | 1 ml |
| dH₂O | ------ | ------ | 41.25 ml |

| | | | |
|---|---|---|---|
| **Filter (45µm) Sterilize and store at 4°C** | | | |

| **1x SSC / 0.1%Sarkosyl / 1mg/ml Casein Wash Buffer** | | | |
|---|---|---|---|
| **Reagent** | **Catalog Number** | **Final Concentration** | **Amount/ 50 ml** |
| 20x SSC | Cambrex US51232 | 1x | 2.5 ml |
| 20% Sarkosyl | ------ | 0.1% | 0.250 ml |
| 50mg/ml Casein (pH7.2) | VWR BDHA440203H | ------ | 1 ml |
| dH₂O | ------ | ------ | 46.25 ml |

| | | | |
|---|---|---|---|
| **Filter (45µm) Sterilize and store at 4°C** | | | |

### B. Beads

1. Bead types used are L100-C123-01 up to L100-C172-01 (Luminex™ Corp., Austin, TX).

### C. Probes (see examples)

1. Probes were supplied by Eurogentec (Seraing, Belgium)

### D. Equipment

| **Equipment** | **Type** |
|---|---|
| Thermocycler | ABI GeneAmp PCR system 9700 |
| Thermo mixer | EppendorfThermomixer comfort |
| Water bath | GFL 1001 |
| Incubation Oven | Memmert U25U |
| Luminex™ | Luminex™ X100 |

### Methods & Protocols:

### I. Probe coupling

1. Bring a fresh aliquot of -20°C, desiccated Pierce EDC [1-Ethyl-3-[dimethylaminopropyl]carbodiimid hydrochlorid] powder to room temperature.
2. Resuspend the amine-substituted oligonucleotide ("probe" or "capture" oligo) to 0.2mM (0.2nmol/µl) in dH₂O.
3. Resuspend the stock microspheres by vortex and sonication for approximately 20 seconds.
4. Transfer 5.0 x 10⁶ of the stock microspheres to a USA Scientific microfuge tube.
5. Pellet the stock microspheres by microcentrifugation at ≥ 8000 x g for 1-2 minutes.
6. Remove the supernatant and resuspend the pelleted microspheres in 50 µl of 0.1 M MES, pH 4.5 by vortex and sonication for approximately 20 seconds.
7. Prepare a 1:10 dilution of the 0.2mM capture oligo in dH₂O (0.02nmol/µl).
8. Add 2 µl (0.04 nmol) of the 1:10 diluted capture oligo to the resuspended microspheres and mix by vortex.
9. Prepare a fresh solution of 20 mg/ml EDC in dH2O. Dissolve 10mg EDC in 500µl dH2O, maximally 1 minute before use. Aliquots of 10mg EDC (powder) were stored dry at -80°C packed together with silica gel.
10. One by one for each reaction, add 2.5 µl of freshly prepared 20 mg/ml EDC to the microspheres and mix by vortex (Note: The aliquot of EDC powder should now be discarded).
11. Incubate for 30 minutes at room temperature in the dark.
12. Prepare a second fresh solution of 20 mg/ml EDC in dH2O.
13. One by one for each reaction, add 2.5 µl of fresh 20 mg/ml EDC to the microspheres and mix by vortex (Note: The aliquot of EDC powder should now be discarded).
14. Incubate for 30 minutes at room temperature in the dark.
15. Add 1.0 ml of 0.02% Tween-20 to the coupled microspheres.
16. Pellet the coupled microspheres by microcentrifugation at ≥ 8000 x g for 1-2 minutes.
17. Remove the supernatant and resuspend the coupled microspheres in 1.0 ml of 0.1% SDS by vortex.
18. Pellet the coupled microspheres by microcentrifugation at ≥ 8000 x g for 1-2 minutes.
19. Remove the supernatant and resuspend the coupled microspheres in 100 µl of TE, pH 8.0 by vortex and sonication for approximately 20 seconds.
20. Pellet the coupled microspheres by microcentrifugation at ≥ 8000 x g for 1-2 minutes.
21. Remove the supernatant and resuspend the coupled microspheres in 100 µl of TE, pH 8.0 by vortex and sonication for approximately 20 seconds.
22. Enumerate the coupled microspheres by hemacytometer:
   a. Dilute the resuspended, coupled microspheres 1:100 in dH₂0.
   b. Mix thoroughly by vortex.
   c. Transfer 10 µl to the hemacytometer.
   d. Count the microspheres within the 4 large squares of the hemacytometer grid.
   e. Microspheres/µl = (Sum of microspheres in 4 large squares) x 2.5 x 100 (dilution factor). (Note: maximum is 50,000 microspheres/µl.)
23. Store coupled microspheres refrigerated at 2-10°C in the dark.

### II. Optimized hybridization & wash protocol

1. Select the appropriate oligonucleotide-coupled microsphere sets.
2. Resuspend the microspheres by vortex and sonication for approximately 20 seconds.
3. Prepare a Working Microsphere Mixture by diluting coupled microsphere stocks to 150 microspheres of each set/µl in 4.5xSSC/0.15%Sarkocyl Hybridization Buffer (Note: 33 µl of Working Microsphere Mixture is required for each reaction).
4. Mix the Working Microsphere Mixture by vortex and sonication for approximately 20 seconds.
5. To each sample or background well, add 33 µl of Working Microsphere Mixture.
6. To each background well, add 17 µl TE, pH 8.
7. To each sample well add amplified biotinylated DNA and TE, pH 8.0 to a total volume of 17 µl (Note: 4 µl of a robust 50 µl PCR reaction is usually sufficient for detection).
8. Mix reaction wells gently by pipetting up and down several times.
9. Incubate at 95-100°C for 5 minutes to denature the amplified biotinylated DNA in a thermocycler.
10. Incubate the reaction plate at 60°C for 3 minutes in a thermocylcer.
11. Transfer the reaction plate to a thermomixer pre-heated at hybridization temperature (Note: An 8-channel pipettor can be used to transfer the reactions in 8 wells simultaneously).
12. Incubate the reaction plate at hybridization temperature for 15 minutes and 500rpm
13. During incubation, prepare the Millipore filter plate by rinsing with distilled water. Next, fill each well of the filter plate with 200µl 3xSSC/0.1%Sarkosyl/1mg/ml Casein wash Buffer at hybridization temperature and place it in an oven at the hybridization temperature.
14. During incubation, prepare fresh reporter mix by diluting streptavidin-R-phycoerythrin to 2µg/ml in 3xSSC/0.1%Sarkocyl/1mg/ml Casein stringent wash buffer (Note: 75 µl of reporter mix is required for each reaction), and place it in an oven or water bath at the hybridization temperature.
15. Terminate the hybridization reaction by transferring the entire reaction to the filter plate containing wash buffer at hybridization temperature
16. After transfer, wash the filter plate twice with 100µl 3xSSC/0.1%Sarkocyl/1mg/ml Casein stringent wash buffer at hybridization temperature by intervening vacuum filtration
17. Add 75 µl of reporter mix to each well and mix gently by pipetting up and down several times.
18. Incubate the reaction plate at hybridization temperature for 15 minutes
19. Terminate the incubation by vacuum filtration.
20. Wash twice with 100µl 1xSSC/0.1%Sarkosyl/1mg/ml Casein wash buffer at room temperature by intervening vacuum filtration
21. Dissolve a reaction in 100µl 1xSSC/0.1%Sarkosyl/1mg/ml Casein wash buffer at room temperature
22. Analyze 50 µl at room temperature on the Luminex™ 100 analyzer according to the system manual.

### III. Read-out

1. Data was read out using the Luminex™ 100 IS version 2.3 software
2. During measurement the following parameters are used:
a. Sample volume: 50µl
b. Sample timeout: 60 sec.
c. XY heater temp (°C): 35
d. Doublet Discriminator Gate:

| | | |
|---|---|---|
| i. | Low Limit: | 8000 |
| ii. | High Limit: | 18500 |

e. Statistic: median

### IV. Data management

1. Data was saved in a raw CSV file (comma delimited *.csv) containing all standard output as provided by the Luminex™100 IS2.3 software.
2. The median signals obtained were transferred to an Excel file for calculation of the target to probe ratio and signal to noise ratio (see also layout and calculations).

The present invention addresses different items of the Luminex™ procedure, including the optimization of the probe design and optimization of the test protocol.

In the following text, data will be presented in the order of the work-flow, as outlined in figure 2.

### Figure 6. General schematic overview of the adapted work-flow

### Presentation of results in the examples (layout and calculations):

The examples and claims involved are specified and explained as follows. Results are mainly presented as tables containing raw data (MFI = median fluorescent intensity), variables (e.g. temperature), probes, and targets as analyzed, calculations, and remarks. The calculations include a target to probe ratio (%target/probe) and a signal to noise ratio (signal/noise).

The target to probe ratio is calculated per probe and displays each of the signals as a percentage of the positive control which is set at 100% (see also example Table 15).

The signal to noise ratio is also calculated per probe. Each signal is divided by the median of all signals obtained (see also example Table 16).

Both the target to probe ratio and signal to noise ratio give a good overall indication on signal intensity and specificity.

Certain examples use probes from the SPF10 primer and probe sets, described in EP1012348. This patent provides a technical background to the techniques used in the present patent application.

The SPF10 primer set generates small amplimers of only 65 bp in length, with an interprimer region of 22 nucleotides. This severely limits the possibilities to position the probes with respect to the different mismatches between all HPV genotypes.

### Example 2

### Objective:

To examine if maintenance of the hybridization temperature after the hybridization step has a significant positive effect on signal specificity.

### Introduction:

After hybridization between the immobilized probe on the bead and the denatured target sequence in solution, the unbound material needs to be washed away before incubation with the reporter reagent Streptavidin-R-phycoerythrin (PE). This is achieved by using a filter plate (MSBVN12, Millipore), where the beads and all attached molecules are separated from molecules free in solution. The reaction volume is small and therefore vulnerable to rapid temperature changes in its environment. We examined the effect of changes in temperature after hybridization temperature.

### Materials and methods:

The effect of incubation at a temperature lower than the hybridization on the Luminex™ signal was investigated using the SPF₁₀ model system.

A Luminex™ bead was used, carrying a probe for HPV 31 (probe 31SLPr31, see table 5a). This probe is specific for identification of HPV 31 sequences amplified with the SPF₁₀ primer set. To assess any cross-reactivity amplimers of HPV44 and HPV16 were used. Target sequences of HPV 31 and HPV 44 differ in 1 position and target sequences of sequences of HPV 31 and HPV 16 differ in 4 positions (Table 5b).

Hybridization was performed at 50°C and assays were run in duplicate. Subsequently, one set of reactions were treated according to the standard protocol and the beads were immediately washed in the filter plate at 4°C. The duplicate set of reactions was first incubated at room temperature (RT) for 1 minute before starting the same standard wash at 4°C. In contrast to Wallace et al (2005), wash buffer was added after the samples were transferred to the filter plate (see also example 3).

### Results:

Results are shown in the Table 5c. As demonstrated, incubation at RT for just 1 minute after hybridization and before the stringent wash causes an increase in signal but also decreases specificity (shown by higher signals observed for HPV44). This can be explained by the reduction in stringency, caused by the brief temperature drop after hybridization.

### Conclusion

The temperature of the reaction should be maintained after the hybridization step. After hybridization the beads should be washed as quickly as possible without any delay to prevent any decrease in temperature.

### Example 3

### Objective:

To examine if a dilution wash, immediately after hybridization, has a significant positive effect on the specificity of the signal.

### Introduction:

The standard Luminex™ assay procedure comprises a risk for introducing aspecific binding if the washing is not immediately following the hybridization step (see also example 2). To minimize this risk the dilution of the sample immediately after hybridization was examined.

### Materials and methods:

To investigate this effect, a mixture of two Luminex™ beads was used, one bead carrying a probe for HPV 31 (name: 31SLPr31, see table 6a) and another bead carrying HPV 51 (name: 51SLPr2, see table 6a). These probes are specific for identification of HPV 31 and HPV 51 sequences amplified with the SPF₁₀ primer set, respectively. To observe possible cross reactivity with 31SLPr31 amplimers of HPV44 and HPV16 were used. Target sequences of HPV 31, and HPV 44 and 16 differ in 1 and 4 positions, respectively (Table 6b). To observe possible cross reactivity with 51SLPr2 amplimers of HPV33 and HPV16 were used. Target sequences of HPV 51 and HPV 44 and 16 each differ in 4 positions (Table 6c).

Hybridization was performed at 50°C, using the standard protocol.

Subsequently, the first set of reactions was immediately washed in the filter plate at 4°C without any additional wash. In contrast to Wallace et al (2005), wash buffer was added after the samples were transferred to the filter plate.

The effect of an additional direct and indirect dilution wash procedure, immediately following the hybridization step was investigated as follows. For the direct and indirect procedures a wash buffer (3x SSC / 0.1%Sarkosyl / 1mg/ml Casein. This is the stringent Wash Buffer) was used at 50°C.

The second set of beads was washed by the direct procedure. The direct procedure comprises a dilution of the hybridization mix (50µl) with 200µl of wash buffer at hybridization temperature in the thermocycler followed by a transfer of the entire diluted sample to the filter plate.

The third hybridization reaction was washed by the indirect procedure. The indirect procedure comprises a dilution by a rapid transfer of the 50µl of the hybridization mix to the filter plate which was already prefilled with 200µl of wash buffer at hybridization temperature (see also Wallace et al, 2005).

### Results:

Results are shown in the table 6d. Both additional wash procedures yield a decrease of the absolute signal, as compared to the standard procedure, but at the same time the specificity of the signal increases significantly. There were no significant differences between the direct and indirect wash procedures. In practice, the direct dilution wash in the thermocycler is less practical, and therefore, the indirect dilution wash procedure is preferred.

### Conclusion:

The use of an additional dilution-wash step after hybridization has a significant positive effect on signal specificity. For practical reasons, the indirect dilution wash procedure is preferred.

### Example 4

### Objective:

To examine if maintenance of the hybridization temperature during the stringent wash before incubation with Streptavidin-R-phycoerythrin, has a significant positive effect on the signal specificity.

### Introduction:

The negative effect of a temperature drop after stringent hybridization, as described above, implies that temperature of the stringent wash itself also can be of influence. Therefore, the effect of the stringent wash temperatures at 50°C, RT or 4°C was investigated.

### Materials and methods:

The effect of different stringent wash buffer temperatures, following the hybridization step before incubation with Streptavidin-R-phycoerythrin was investigated using the SPF₁₀ model **system as follows.**

To investigate this effect, a Luminex™ bead was used, carrying a probe for HPV 31 (name: 31SLPr31, see table 7a). This probe is specific for identification of HPV 31 sequences amplified with the SPF₁₀ primer set. To observe possible cross reactivity with 31SLPr31 amplimers of HPV44 and HPV 16 were used. Target sequences of HPV 31 and HPV 44 and 16 differ in 1 and 4 positions, respectively (Table 7b).

Hybridization was performed at 50°C. Subsequently, the set of reactions were transferred to a filter plate containing wash buffer at 50°C, RT, or 4°C, respectively.

### Results:

Results are shown in table 7c. The absolute level of the positive control signal does not differ between 50°C and RT, and is slightly decreased after washing at 4°C. However, washing at 50°C results in a significant increase of signal specificity, whereas washing at RT or 4°C results in a decrease of signal specificity. Therefore, an indirect dilution wash procedure at hybridization temperature of 50°C is preferred.

### Conclusion:

Maintenance of the hybridization temperature during the stringent wash before incubation with Streptavidin-R-phycoerythrin, has a significant effect on the signal specificity.

### Example 5

### Objective:

To examine if the use of a thermomixer has a significant positive effect on signal intensity.

### Introduction:

The kinetics of a hybridization reaction can be influenced by mixing the components during the reaction.

Therefore we investigated the influence of using a thermomixer during hybridization.

### Materials and methods:

The effect of diffusion kinetic using a thermomixer during hybridization was investigated using the MPF model system as follows.

Two Luminex™ beads were used, carrying either a probe for HPV 18 (name: 18MLPr7, see table 8a) or HPV51 (name: 51MLPr2, see table 8a). These probes are specific for identification of HPV18 and HPV51 sequences amplified with the MPF primer set.

The two beads were mixed and hybridized with MPF amplimers of HPV 18 and HPV 51. Target sequences of HPV 18 and HPV51 differ in 7 positions (Table 8b and c). Reactions were tested in duplicate.

One reaction was denatured and hybridized in a thermocycler, without shaking.(see also Wallace et al, 2005)

The duplicate reaction was denatured in a thermocycler for denaturation, and immediately transferred to a thermomixer for hybridization. Hybridization was performed at 50°C. Subsequently, the beads were immediately washed in the filter plate at 50°C, using the optimized hybridization and wash protocol.

**Results:** Results are shown in table 8d. Use of a thermo-mixer significantly increases the absolute signal of the positive control, whereas the background remained unaffected. This resulted in an overall increase of signal specificity.

These results demonstrate that the signal intensity will be increased (improved) by using a thermo-mixer.

### Conclusion:

The use of a thermo-mixer has a significant positive effect on the signal intensity and specificity.

### Example 6

### Objective:

To examine if incubation with Streptavidin-R-phycoerythrin at the hybridization temperature has a significant positive effect on the signal intensity.

### Introduction:

In general, temperature affects the kinetics of any reaction, including the detection of hybrids with the reporter PE. Therefore, the influence of temperature for PE incubation and the subsequent wash was investigated.

### Materials and methods:

Luminex™ beads were used, carrying a probe for HPV51 (name: 51SLPr2, see table 9a). This probe is specific for identification HPV51 sequences amplified with the SPF₁₀ primer set. To observe possible cross reactivity with this probe, SPF10 amplimers of HPV33 and HPV16 were used. Target sequences of HPV 51, HPV33 and HPV16 differ at 4 positions (Table 9b).

Hybridization was performed at 50°C in two replicates, using the optimized hybridization and wash protocol outlined herein. After stringent wash, one set of reactions was incubated with PE at 50°C (see also Wallace et al, 2005), and the other set was incubated with PE at RT. Subsequently, the beads were washed in a filter plate at 50°C.

In another experiment, hybridization was performed at 50°C in two replicates, using the optimized hybridization and wash protocol. After stringent wash, all reactions were incubated with PE at 50°C (see also Wallace et al, 2005). After PE incubation at 50°C, one set of reactions was washed at 50°C (see also Wallace et al, 2005), and the duplicate set was washed at RT.

### Results:

PE incubation at different temperatures had a significant effect, as shown in table 9c. PE incubation at the hybrizidation temperature of 50°C results in higher absolute signals, as compared to PE incubation at RT. However, the specificity of the signal did not differ significantly.

Therefore, incubation at with Streptavidin-R-phycoerythrin at hybrizidation temperature is preferred. In contrast, washing at RT or hybridization temperature after incubation did not have a significant effect, although this may be more practical in some situations.

The influence of temperature on the washing step after PE incubation is not significant. Both the absolute signal as well as the specificity appear not to be affected by the temperature of the wash.

### Conclusion:

Maintenance of the hybridization temperature during incubation with Streptavidin-R-phycoerythrin, has a significant effect on the signal intensity but not on the signal specificity. The temperature of the wash after PE incubation has no significant effect.

### Example 7

### Objective:

To examine whether clogging of Luminex™ sampling probe can be prevented by a final wash with 1x SSC.

### Introduction:

In our optimized hybridization and wash protocol hybridization is performed in 3x SSC. At this concentration SSC does clog the Luminex™ sampling probe seriously obstructing processing of the samples. Therefore, the influence of a lower SSC concentration was investigated for a final wash.

### Results:

Initially we tried to maintain the SSC concentration of the hybridization. However, as a final wash with 3xSSC introduced a serious clogging of the Luminex™ sampling probe, no significant data could be produced. Simply performing this wash step with 1xSSC did result in significant data. Therefore, due to lacking data, a comparison by data can not be shown. Other SSC concentrations have not been investigated.

### Conclusion:

A final wash with 1x SSC prevents clogging of the Luminex™ sampling probe.

### Example 8

### Objective:

To examine if storage after the final wash at 4°C for at least 4 days of samples that are ready for measuring has any significant effect on the signal.

### Introduction:

To increase flexibility on the work floor we analyzed several steps with respect to the direct hybridization test protocol using the Luminex™ system. One procedure tested in particular is storage in between two steps of the direct hybridization procedure. Therefore, we investigated the influence of storage at 4°C.

### Materials and methods:

The effect of storage at 4°C after the final washing procedure was investigated using the SPF10 model system as follows.

To investigate this effect, Luminex™ beads were used, carrying a probe for HPV51 (name: 51SLPr2, see table 10a). This probe is specific for identification HPV51 sequences amplified with the SPF₁₀ primer set. To observe possible cross reactivity with 51SLPr2 amplimers of HPV31 were used. Target sequences of HPV 51 and, HPV31 differ in 4 positions (Table 10b).

Following the final wash procedure, sets of reactions were stored at 4°C, for 0, 4, 24, and 96 hrs, respectively. Next, these reaction sets were measured at RT.

### Results:

Results are shown in 10c. As demonstrated, storage after the final wash step does not affect signal intensity or specificity. Nevertheless, storage as such seems to introduce a very slight improve in raw signal intensity over time. Therefore, storage after the final wash step can be introduced if necessary for a maximum of 4 days, maintaining the original signal.

### Conclusion:

Storage after the final wash step has no significant effect on signal intensity and signal specificity, increasing flexibility on the work floor.

### Probe (spacer) design - Introduction

The key principle of the Luminex™ system is the immobilization of specific oligonucleotide probe on the surface of a microbead, which serves as a unique label, due to the color composition of the individual bead types.

At the molecular scale, the bead is much bigger that the specific oligonucleotide probe. Consequently, the specific probe sequence is positioned very closely to the surface of the Luminex™ bead. This probe location may not be the optimal for hybridization kinetics between the immobilized probe and the target molecules in solution, due to steric hindrance and various bead surface effects, such as surface hydrophobicity.

The following examples describe a number of approaches to change the positioning of the probe onto the bead surface, in order to optimize the hybridization kinetics between probe and target.

The following variants in probe design were tested:
1. Use of a carbon spacer of variable length
2. Use of an additional oligonucleotide spacer of variable length
3. Use of an oligonucleotide spacer of variable composition

The probe has three distinct regions, with different functions;
1. the coupling group, such as an NH2 group, which permits covalent coupling of the probe to the bead surface;
2. the spacer, which may serve (a) to create a distance between the bead surface and the specific probe sequence and/or (b) to position the specific probe more in a hydrophilic environment; and
3. the actual target-specific probe sequence. For this part of the probe, the normal parameters in the art, such as probe composition and length apply.

### Example 9

### Objective:

To determine the effect of the use of a carbon spacer of variable length.

### Materials and methods:

Luminex™ beads were used, carrying either a probe for HPV51 with a C₁₂ spacer (name: 51SLPr2, see table 11a) or a C₁₈ spacer (name: 51SLPr2C₁₈, see table 11a). These probes are specific for identification HPV51 sequences amplified with the SPF₁₀ primer set. To observe possible cross reactivity with these probes, amplimers of HPV33 were used. Target sequences of HPV 51 and HPV33 differ in 4 positions (Table 11b).

**Results:** Results are shown in table 11c. A C18 spacer resulted in a decrease in absolute signal, but the specificity was higher as compared to the C 12 probe. This phenomenon was not only seen for 51SLPr2C₁₈, but also for other probes with a C₁₈ carbon spacer (e.g. 33SLPr21 C_{18:} Table 11a, c, and d).

### Conclusion:

The use of different carbon spacer lengths has a significant effect on signal specificity. With respect to for example 51SLPr2, the best probe contains a C₁₈ carbon spacer.

### Example 10

### Objective :

To determine the effect of an oligonucleotide spacer of variable length.

### Materials and methods:

Luminex™ beads were used, carrying a probe for HPV51 with a spacer of either 0, 10, 20, 30, or 40 Thymines (name: 51SLPr2, 51SLPr2T10, 51 SLPr2T20, 51SLPr2T30, 51SLPr2T40, see table 12a). Each bead type carried a distinct probe variant. These probes are specific for identification HPV51 sequences amplified with the SPF₁₀ primer set. To observe possible cross reactivity with these probes, amplimers of HPV33 were used. Target sequences of HPV51 and HPV33 differ in 4 positions (Table 12c).

Apart from the SPF10 model system this effect was also studied using the MPF model system as follows. Luminex™ beads were used, carrying a probe for HPV52 with a spacer of either 0, 20, 30, or 40 Thymines (name: 52MLPr2, 52MLPr2T20, 5MLPr2T30, 52MLPr2T40, see table 12b). Each bead type carried a distinct probe variant. These probes are specific for identification HPV52 sequences amplified with the MPF primer set. To observe possible cross reactivity with these probes, amplimers of HPV16 were used. Target sequences of HPV52 and HPV16 differ in 2 positions (Table 12d).

### Results:

Results are shown in table 12e and 12f. Elongation of the spacer with a thymine stretch significantly increases the absolute signal level. Also, the specificity is significantly increased, as compared to a spacer without an additional thymine spacer. Comparing the spacers with different lengths, a minimum of 20 thymine residues is required to yield an optimal signal (e.g. 51SLPr2). Overall, probes perform best when they contain a spacer of 40 nucleotides (e.g 51SLPr2, and 52MLPr2). Therefore this spacer length is preferred.

### Conclusion:

The use of different spacers has a significant effect not only on signal intensity, but also on specificity. With respect to 51SLPr2Tₙ, a good probe contains a spacer of at least 20 thymine nucleotides increasing both signal intensity and specificity. In general, a spacer length of at least 40 nucleotides performs best.

### Example 11

### Object:

To determine whether use of a modified poly(T) spacer can prevent false-positive reactivity.

### Introduction:

It is well known that many Taq DNA polymerases add an additional A-nucleotide at the 3' end of a synthesized strand. It is not known whether also multiple A's can be added to the 3'end, thereby generating a subpopulation of molecules with an oligo-A tail at the 3'end. Although such molecules will only represent a very small proportion of the total amount of PCR product, these molecules can result in false-negative result, due to the high sensitivity of the detection method. This is due to the fact that hybridization between such oligo-A stretches at the PCR-product and the poly(T) spacer of the probe.

This PCR artifact occurs in some samples, and is hard to reproduce at the PCR level. It appears to be dependent on very small fluctuations in reaction conditions. The background is very reproducible at the detection level, i.e. a PCR product generating background will do so very reproducibly.

This PCR artifact can also cause false-positive results on a line probe assay (LiPA) system, since this system also comprises T-tailed probes. In a LiPA assay this results in a weak equal (background) signal with all probes, irrespective of their specific sequence. Also in the Luminex™ system such weak background signal readouts have been observed. Therefore, the effect of a modified spacer was investigated.

### Materials and methods:

Luminex™ beads were used, carrying either a probe for HPV 18 with a T40 spacer, or a modified (TTG)13 spacer (name: 18MLPr7T40 and 18MLPr7(TTG)₁₃, see table 13a). These probes are specific for identification of HPV 18 sequences amplified with the MPF primer set.

The (TTG) triplet was chosen as an alternative spacer because it shows one of the worst theoretical binding efficiencies with poly (A).

To observe possible cross reactivity with 18MLPr7T40 and 18MLPr7(TTG)₁₃ amplimers derived from samples showing this false-positive background were used (designated nc8).

### Results:

Results are shown in table 13b.

A spacer of 13 "TTG" nucleotide triplets was clearly able to almost completely eliminate the background signal, which was observed for the T40 spacer.

### Conclusion:

The use of an alternative T-based spacer, such as (TTG)₁₃ has a significant positive effect on the signal specificity, eliminating false-positive signals induced by A-rich PCR artifacts.

### Example 12

### Object:

To examine if positioning a Thymine based spacer at either the 5'- or 3'-end of a probe prohibits binding to an A-rich target region flanking the probe-target binding site.

### Introduction:

It is known that mismatches in the middle of a probe / target have the largest impact on its binding energy. Mismatches close to the sides of the binding region are more difficult to distinguish. In combination with the position of A-rich stretches flanking the probe / target binding region this may harm the selective strength of a probe. Therefore, we investigated the influence of the spacer position to minimize its binding to an A-rich target region flanking the probe-target binding site.

### Materials and methods:

The effect of a spacer position at either the 5'- or 3'-end of a probe, positioned between the Luminex™ bead and the specific probe sequence was investigated using the MPF model system as follows.

To investigate this effect, Luminex™ beads were used, carrying a probe for HPV 18 and HPV45 with a Thymine based spacer (name: 18MLPr7T40N5, 18MLPr7T40N3, 45MLPr8T40N5 and 45MLPr8T40N3, see table 14a). These probes are specific for identification of HPV 18 and HPV45 sequences amplified with the MPF primer set, respectively. To observe possible cross reactivity with 18MLPr7T40ₙ amplimers of HPV39 were used. Target sequences of HPV18 and, HPV39 differ in 2 positions (Table 14b). To observe possible cross reactivity with 45MLPr8T40ₙ amplimers of HPV13, 39, and 40 were used. Target sequences of HPV45 and, HPV13, 39 and 40 differ in 3, 2, and 1 position, respectively (Table 14c).

### Results:

Results are shown in table 14d. As demonstrated, a spacer at the 3'-end of a probe instead of the 5'-end decreases its binding to an A-rich target region flanking the probe-target binding site, affecting the binding energy (dG) and melting temperature (Tms). The exclusion of these aspecific signals can be explained by binding of the target to the spacer and probe. These results suggest that the binding of a target to the spacer can hamper probe specificity, which should be prevented. In principle a likewise mechanism may be involved using a "TTG" nucleotide triplet spacer. Therefore, when using a Thymine based spacer, the stability of the probe:target hybrid can be increased by weak cross-hybridization between spacer and sequences adjacent to the specific target region, resulting in false-positive signal which should be taken into account for the probe design.

### Conclusion:

The position of a Thymine based spacer at either the 5' or 3' end of a probe can have a significant effect with respect to binding an A-rich target region flanking the probe-target binding site.

### Literature references:

Cowan LS, Diem L, Brake MC, Crawford JT. Related Articles. Transfer of a Mycobacterium tuberculosis genotyping method, Spoligotyping, from a reverse line-blot hybridization, membrane-based assay to the Luminex multianalyte profiling system. J Clin Microbiol. 2004 Jan;42(1):474-7.
Dunbar SA. Applications of Luminex™(R) xMAPtrade mark technology for rapid, high-throughput multiplexed nucleic acid detection. Clin Chim Acta. 2005 Aug 12; [Epub ahead of print]
Taylor JD, Briley D, Nguyen Q, Long K, Iannone MA, Li MS, Ye F, Afshari A, Lai E, Wagner M, Chen J, Weiner MP. Flow cytometric platform for high-throughput single nucleotide polymorphism analysis. Biotechniques. 2001 Mar;30(3):661-6, 668-9.
de Villiers EM, Fauquet C, Broker TR, Bernard HU, zur Hausen H. Classification of papillomaviruses. Virology. 2004 Jun 20;324(1):17-27. Review.
Wallace J, Woda BA, Pihan G. Facile, comprehensive, high-throughput genotyping of human genital papillomaviruses using spectrally addressable liquid bead microarrays. J Mol Diagn. 2005 Feb;7(1):72-80.

### Tables example 2:

**Table 5a. 31SLPr31 = SPF₁₀ probe 31 version 31, C₁₂ = a stretch of 12 carbon atoms**

| **Name** | **Probe composition** |
|---|---|
| 31SLPr31 | NH₂-C₁₂-GGCAATCAGTTATTTG [SEQ ID NO:119] |

**Table 5b. Identical nucleotides are indicated by a "-".**

| **Target** | **Alignment with probe 31SLPr31** | **Number of mismatches** |
|---|---|---|
| HPV 31 | GGCAATCAGTTATTTG [SEQ ID NO: 119] | 0 |
| HPV 44 | --A------------- [SEQ ID NO: 120] | 1 |
| HPV 16 | --T-C-AC-------- [SEQ ID NO: 121] | 4 |

**Table 5c.**

| **Probe** | **Hybridized to target** | **Temperature after hybridization (°C)** | **Signal (MFI)** | **target / probe (%)** | **Signal / noise** | **Remark** | **Exp** |
|---|---|---|---|---|---|---|---|
| 31SLPr31 | SPF₁₀ HPV31 | 50 | 4457 | 100 | 48 | Specific | ID28 |
| 31SLPr31 | SPF₁₀ HPV44 | 50 | 1279 | 29 | 14 | Cross reaction | ID28 |
| 31SLPr31 | SPF₁₀ HPV16 | 50 | 19 | <1 | <1 | Negative | ID28 |
| 31SLPr31 | SPF₁₀ HPV31 | RT | 7544 | 100 | 13 | Specific | ID27 |
| 31SLPr31 | SPF₁₀ HPV44 | RT | 3783 | 50 | 6 | Cross reaction | ID27 |
| 31SLPr31 | SPF₁₀ HPV16 | RT | 24 | 1 | <1 | Negative | ID27 |

### Tables example 3:

**Table 6a. 31 SLPr31 = SPF₁₀ probe 31 version 31, C₁₂ = a stretch of 12 carbon atoms**

| **Name** | **Probe composition** |
|---|---|
| 31SLPr31 | NH₂-C₁₂-GGCAATCAGTTATTTG [SEQ ID NO: 119] |
| 51SLPr2 | NH₂-C₁₂-CTATTTGCTGGAACAATC [SEQ ID NO: 122] |

**Table 6b. Identical nucleotides are indicated by a "-".**

| **Target** | **Alignment with probe 31SLPr31** | **Number of mismatches** |
|---|---|---|
| HPV 31 | GGCAATCAGTTATTTG [SEQ ID NO: 119] | 0 |
| HPV 44 | --A [SEQ ID NO: 120] | 1 |
| HPV 16 | --T-C-AC-------- [SEQ ID NO: 121] | 4 |

**Table 6c. Identical nucleotides are indicated by a "-".**

| **Target** | **Alignment with probe 51SLPr2** | **Number of mismatches** |
|---|---|---|
| HPV 51 | CTATTTGCTGGAACAATC [SEQ ID NO: 122] | 0 |
| HPV 33 | T------T---GG----- [SEQ ID NO: 123] | 4 |
| HPV 16 | -------T---GGT--C- [SEQ ID NO: 124] | 5 |

**Table 6d.**

| **Probe** | **Hybridized to target** | **Add. wash procedure** | **Signal (MFI)** | **target / probe (%)** | **Signal / noise** | **Remark** | **Exp** |
|---|---|---|---|---|---|---|---|
| 31SLPr31 | SPF₁₀ HPV31 | None | 4457 | 100 | 48 | Specific | ID28 |
| 31SLPr31 | SPF₁₀ HPV44 | None | 1279 | 29 | 14 | Cross reaction | ID28 |
| 31SLPr31 | SPF₁₀ HPV16 | None | 19 | <1 | <1 | Negative | ID28 |
| 31SLPr31 | SPF₁₀ HPV31 | Direct | 2765 | 100 | 41 | Specific | ID31 |
| 31SLPr31 | SPF₁₀ HPV44 | Direct | 117 | 4 | 2 | Negative | ID31 |
| 31SLPr31 | SPF₁₀ HPV16 | Direct | 20 | 1 | <1 | Negative | ID31 |
| 31SLPr31 | SPF₁₀ HPV31 | Indirect | 3843 | 100 | 171 | Specific | ID32 |
| 31SLPr31 | SPF₁₀ HPV44 | Indirect | 25 | 1 | 1 | Negative | ID32 |
| 31SLPr31 | SPF₁₀ HPV16 | Indirect | 15 | <1 | 1 | Negative | ID32 |
| | | | | | | | |
| 51SLPr2 | SPF₁₀ HPV51 | None | 2316 | 100 | 201 | Specific | ID28 |
| 51SLPr2 | SPF₁₀ HPV33 | None | 631 | 27 | 55 | Cross reaction | ID28 |
| 51SLPr2 | SPF₁₀ HPV16 | None | 11 | <1 | 1 | Negative | ID28 |
| 51SLPr2 | SPF₁₀ HPV51 | Direct | 2057 | 100 | 110 | Specific | ID31 |
| 51SLPr2 | SPF₁₀ HPV33 | Direct | 432 | 21 | 23 | Cross reaction | ID31 |
| 51SLPr2 | SPF₁₀ HPV16 | Direct | 18 | 1 | 1 | Negative | ID31 |
| 51SLPr2 | SPF₁₀ HPV51 | Indirect | 1571 | 100 | 209 | Specific | ID32 |
| 51SLPr2 | SPF₁₀ HPV33 | Indirect | 354 | 23 | 47 | Cross reaction | ID32 |
| 51SLPr2 | SPF₁₀ HPV16 | Indirect | 7 | <1 | 1 | Negative | ID32 |

### Tables example 4:

**Table 7a. 31SLPr31 = SPF₁₀ probe 31 version 31, C₁₂ = a stretch of 12 carbon atoms**

| **Name** | **Probe composition** |
|---|---|
| 31SLPr31 | NH₂-C₁₂-GGCAATCAGTTATTTG [SEQ ID NO: 119] |

**Table 7b. Identical nucleotides are indicated by a "-".**

| **Target** | **Alignment with probe 31SLPr31** | **Number of mismatches** |
|---|---|---|
| HPV 31 | GGCAATCAGTTATTTG [SEQ ID NO: 119] | 0 |
| HPV 44 | --A------------- [SEQ ID NO: 120] | 1 |
| HPV 16 | --T-C-AC-------- [SEQ ID NO: 121] | 4 |

**Table 7c.**

| **Probe** | **Hybridized to target** | **Wash temp (°C)** | **Signal (MFI)** | **target / probe (%)** | **Signal / noise** | **Remark** | **Exp** |
|---|---|---|---|---|---|---|---|
| 31SLPr31 | SPF₁₀ HPV31 | 50 | 5747 | 100 | 162 | Specific | ID90 |
| 31SLPr31 | SPF₁₀ HPV44 | 50 | 56 | 1 | 2 | Negative | ID90 |
| 31SLPr31 | SPF₁₀ HPV16 | 50 | 20 | <1 | <1 | Negative | ID90 |
| 31SLPr31 | SPF₁₀ HPV31 | RT | 5701 | 100 | 33 | Specific | ID86 |
| 31SLPr31 | SPF₁₀ HPV44 | RT | 2422 | 42 | 14 | Cross react | ID86 |
| 31SLPr31 | SPF₁₀ HPV16 | RT | 13 | <1 | <1 | Negative | ID86 |
| 31SLPr31 | SPF₁₀ HPV31 | 4 | 4889 | 100 | 44 | Specific | ID34 |
| 31SLPr31 | SPF₁₀ HPV44 | 4 | 417 | 9 | 4 | Cross react | ID34 |
| 31SLPr31 | SPF₁₀ HPV16 | 4 | 33 | 1 | <1 | Negative | ID34 |

### Tables example 5:

**Table 8a. 18MLPr7 = MPF probe 18 version 7, C₁₂ = a stretch of 12 carbon atoms**

| **Name** | **Probe composition** |
|---|---|
| 18MLPr7T40 | NH₂-C₁₂-(T)₄₀-TTACATAAGGCACAGG [SEQ ID NO: 125] |
| 51MLPr2T40 | NH₂-C₁₂-(T)₄₀-TTATTGGCTCCACCGT [SEQ ID NO: 126] |

**Table 8b. Identical nucleotides are indicated by a "-".**

| **Target** | **Alignment with probe 18MLPr7** | **Number of mismatches** |
|---|---|---|
| HPV18 | TTACATAAGGCACAGG [SEQ ID NO: 127] | 0 |
| HPV51 | C-C--CCGT--G---- [SEQ ID NO: 128] | 7 |

**Table 8c. Identical nucleotides are indicated by a "-".**

| **Target** | **Alignment with probe 51MLPr2** | **Number of mismatches** |
|---|---|---|
| HPV51 | TTATTGGCTCCACCGT [SEQ ID NO: 70] | 0 |
| HPV18 | A------T-A--TAAG [SEQ ID NO: 129] | 7 |

**Table 8d.**

| **Probe** | **Hybridized to target** | **Hybr. proc.** | **Signal (MFI)** | **target / probe (%)** | **Signal / noise** | **Remark** | **Exp** |
|---|---|---|---|---|---|---|---|
| 18MLPr7T40 | MPF HPV 18 | Thermo Cycler | 1082 | 100 | 144 | Specific | ID 148 |
| 18MLPr7T40 | MPF HPV51 | Thermo Cycler | 6 | 1 | 1 | Negative | ID148 |
| 51MLPr2T40 | MPF HPV51 | Thermo Cycler | 1410 | 100 | 123 | Specific | ID 148 |
| 51MLPr2T40 | MPF HPV18 | Thermo Cycler | 20 | 1 | 1 | Negative | ID 148 |
| 18MLPr7T40 | MPF HPV 18 | Thermo Mixer | 2154 | 100 | 287 | Specific | ID 148 |
| 18MLPr7T40 | MPF HPV51 | Thermo Mixer | 6 | 0 | 1 | Negative | ID148 |
| 51MLPr2T40 | MPF HPV51 | Thermo Mixer | 2725 | 100 | 210 | Specific | ID 148 |
| 51MLPr2T40 | MPF HPV18 | Thermo Mixer | 25 | 1 | 2 | Negative | ID 148 |

### Tables example 6:

**Table 9a. 51SLPr2 = SPF₁₀ probe 51 version 2, C₁₂ = a stretch of 12 carbon atoms**

| **Name** | **Probe composition** |
|---|---|
| 51SLPr2 | NH₂-C₁₂-CTATTTGCTGGAACAATC [SEQ ID NO: 122] |

**Table 9b. Identical nucleotides are indicated by a "-".**

| **Target** | **Alignment with probe 51SLPr2** | **Number of mismatches** |
|---|---|---|
| HPV 51 | CTATTTGCTGGAACAATC [SEQ ID NO: 122] | 0 |
| HPV 33 | T------T---GG----- [SEQ ID NO: 123] | 4 |
| HPV 16 | -------T---GGT--C-[SEQ ID NO: 124] | 5 |

**Table 9c.**

| **Probe** | **Hybridized to target** | **PE inc. temp. (°C)** | **Signal (MFI)** | **target / probe (%)** | **Signal / noise** | **Remark** | **Exp** |
|---|---|---|---|---|---|---|---|
| 51SLPr2 | SPF₁₀ HPV51 | 50 | 3681 | 100 | 194 | Specific | ID44 |
| 51SLPr2 | SPF₁₀ HPV33 | 50 | 345 | 9 | 18 | Cross react | ID44 |
| 51SLPr2 | SPF₁₀ HPV16 | 50 | 30 | 1 | 2 | Negative | ID44 |
| 51SLPr2 | SPF₁₀ HPV51 | RT | 3074 | 100 | 615 | Specific | ID43 |
| 51SLPr2 | SPF₁₀ HPV33 | RT | 259 | 8 | 52 | Cross react | ID43 |
| 51SLPr2 | SPF₁₀ HPV16 | RT | 5 | <1 | 1 | Negative | ID43 |

**Table 9d.**

| **Probe** | **Hybridized to target** | **Wash temp. (°C)** | **Signal (MFI)** | **target/ probe (%)** | **Signal / noise** | **Remark** | **Exp** |
|---|---|---|---|---|---|---|---|
| 51SLPr2 | SPF₁₀ HPV51 | 50 | 2433 | 100 | 187 | Specific | ID90 |
| 51SLPr2 | SPF₁₀ HPV33 | 50 | 423 | 16 | 33 | Cross react | ID90 |
| 51SLPr2 | SPF₁₀ HPV16 | 50 | 8 | <1 | 1 | Negative | ID90 |
| 51SLPr2 | SPF₁₀ HPV51 | RT | 2777 | 100 | 179 | Specific | ID90 |
| 51SLPr2 | SPF₁₀ HPV33 | RT | 374 | 13 | 24 | Cross react | ID90 |
| 51SLPr2 | SPF₁₀ HPV16 | RT | 10 | <1 | 1 | Negative | ID90 |

### Tables example 8:

**Table 10a. 51SLPr2 = SPF₁₀ probe 51 version 2, C₁₂ = a stretch of 12 carbon atoms**

| **Name** | **Probe composition** |
|---|---|
| 51SLPr2 | NH₂-C₁₂-CTATTTGCTGGAACAATC [SEQ ID NO: 122] |

**Table 10b. Identical nucleotides are indicated by a "-".**

| **Target** | **Alignment with probe 51SLPr2** | **Number of mismatches** |
|---|---|---|
| HPV51 | CTATTTGCTGGAACAATC [SEQ ID NO: 122] | 0 |
| HPV 31 | T------T---GG----- [SEQ ID NO: 123] | 4 |

**Table 10c.**

| **Probe** | **Hybridized to target** | **Storage 4°C (hrs)** | **Signal (MFI)** | **target / probe (%)** | **Signal / noise** | **Remark** | **Exp** |
|---|---|---|---|---|---|---|---|
| 51SLPr2 | SPF₁₀ HPV51 | 0 | 1573 | 100 | 51 | Specific | ID110 |
| 51SLPr2 | SPF₁₀ HPV31 | 0 | 30 | 2 | 1 | Negative | ID110 |
| 51SLPr2 | SPF₁₀ HPV51 | 4 | 1611 | 100 | 59 | Specific | ID111 |
| 51SLPr2 | SPF₁₀ HPV31 | 4 | 28 | 2 | 1 | Negative | ID111 |
| 51SLPr2 | SPF₁₀ HPV51 | 24 | 1783 | 100 | 60 | Specific | ID113 |
| 51SLPr2 | SPF₁₀ HPV31 | 24 | 34 | 2 | 1 | Negative | ID 113 |
| 51SLPr2 | SPF₁₀ HPV51 | 96 | 1707 | 100 | 52 | Specific | ID114 |
| 51SLPr2 | SPF₁₀ HPV31 | 96 | 33 | 2 | 1 | Negative | ID114 |

### Tables example 9:

**Table 11a. 51SLPr2 = SPF₁₀ probe 51 version 2, C₁₂ = a stretch of 12 carbon atoms, C₁₈ = a stretch of 18 carbon atoms**

| **Name** | **Probe composition** |
|---|---|
| 51SLPr2 | NH₂-C₁₂-CTATTTGCTGGAACAATC [SEQ ID NO: 122] |
| 51SLPr2C₁₈ | NH₂-C₁₈-CTATTTGCTGGAACAATC [SEQ ID NO: 122] |
| 33SLPr21 | NH₂-C₁₂-GGGCAATCAGGTATT [SEQ ID NO: 130] |
| 33SLPr21C₁₈ | NH₂-C₁₈-GGGCAATCAGGTATT [SEQ ID NO: 130] |

**Table 11b. Identical nucleotides are indicated by a "-".**

| **Target** | **Alignment with probe 51SLPr2** | **Number of mismatches** |
|---|---|---|
| HPV 51 | CTATTTGCTGGAACAATC [SEQ ID NO: 122] | 0 |
| HPV 33 | T------T---GG----- [SEQ ID NO: 123] | 4 |

**Table 11c. Identical nucleotides are indicated by a "-".**

| **Target** | **Alignment with probe 33SLPr21** | **Number of mismatches** |
|---|---|---|
| HPV 33 | GGGCAATCAGGTATT [SEQ ID NO: 130] | 0 |
| HPV 51 | -AA---------C-T-- [SEQ ID NO: 131] | 4 |

**Table 11d.**

| **Probe** | **Hybridized to target** | **Signal (MFI)** | **target / probe (%)** | **Signal / noise** | **Remark** | **Exp** |
|---|---|---|---|---|---|---|
| 51SLPr2 | SPF₁₀ HPV51 | 4291 | 100 | 172 | Specific | ID64 |
| 51SLPr2 | SPF₁₀ HPV33 | 358 | 8 | 14 | Cross reaction | " |
| 51SLPr2C₁₈ | SPF₁₀ HPV51 | 3515 | 100 | 216 | Specific | ID67 |
| 51SLPr2C₁₈ | SPF₁₀ HPV33 | 16 | 0 | 1 | Negative | " |
| | | | | | | |
| 33SLPr21 | SPF₁₀ HPV33 | 429 | 100 | 48 | Specific | ID77 |
| 33SLPr21 | SPF₁₀ HPV51 | 52 | 12 | 6 | Cross reaction | " |
| 33SLPr21C₁₈ | SPF₁₀ HPV33 | 429 | 100 | 61 | Specific | " |
| 33SLPr21C₁₈ | SPF₁₀ HPV51 | 4 | 1 | 1 | Negative | " |

### Tables example 10:

**Table 12a. 51SLPr2 = SPF₁₀ probe 51 version 2, C₁₂ = a stretch of 12 carbon atoms, (T)₄₀ = a stretch of 40 Thymine nucleotides**

| **Name** | **Probe composition** |
|---|---|
| 51SLPr2 | NH₂-C₁₂-CTATTTGCTGGAACAATC [SEQ ID NO: 122] |
| 51SLPr2T10 | NH₂-C₁₂-(T)₁₀-CTATTTGCTGGAACAATC [SEQ ID NO: 132] |
| 51 SLPr2T20 | NH₂-C₁₂-(T)₂₀-CTATTTGCTGGAACAATC [SEQ ID NO: 133] |
| 51SLPr2T30 | NH₂-C₁₂-(T)₃₀-CTATTTGCTGGAACAATC [SEQ I D NO: 134] |
| 51 SLPr2T40 | NH₂-C₁₂-(T)₄₀-CTATTTGCTGGAACAATC [SEQ ID NO: 135] |

**Table 12b. 52MLPr2 = MPF probe 52 version 2, C₁₂= a stretch of 12 carbon atoms, (T)₄₀ = a stretch of 40 Thymine nucleotides**

| **Name** | **Probe composition** |
|---|---|
| 52MLPr2 | NH₂-C₁₂-CCGTACTGGTTACAACGA [SEQ ID NO: 73] |
| 52MLPr2T20 | NH₂-C₁₂-(T)₂₀- CCGTACTGGTTACAACGA [SEQ I D NO: 136] |
| 52MLPr2T30 | NH₂-C₁₂-(T)₃₀- CCGTACTGGTTACAACGA [SEQ ID NO: 137] |
| 52MLPr2T40 | NH₂-C₁₂-(T)₄₀- CCGTACTGGTTACAACGA [SEQ ID NO: 138] |

**Table 12c. Identical nucleotides are indicated by a "-".**

| **Target** | **Alignment with probe 51SLPr2** | **Number of mismatches** |
|---|---|---|
| HPV 51 | CTATTTGCTGGAACAATC [SEQ ID NO: 122] | 0 |
| HPV 33 | T------T---GG----- [SEQ ID NO: 123] | 4 |

**Table 12d. Identical nucleotides are indicated by a "-".**

| **Target** | **Alignment with probe 52MLPr2** | **Number of mismatches** |
|---|---|---|
| HPV 52 | CCGTACTGGTTACAACGA [SEQ ID NO: 73] | 0 |
| HPV 16 | --T--T------------ [SEQ ID NO: 139] | 2 |

**Table 12e.**

| **Probe** | **Hybridized to target** | **Signal (MFI)** | **target / probe (%)** | **Signal / noise** | **Remark** | **Exp** |
|---|---|---|---|---|---|---|
| 51SLPr2 | SPF₁₀ HPV51 | 4291 | 100 | 172 | Specific | ID64 |
| 51SLPr2 | SPF₁₀ HPV33 | 358 | 8 | 14 | Cross reaction | ID64 |
| 51SLPr2T10 | SPF₁₀ HPV51 | 4688 | 100 | 122 | Specific | ID64 |
| 51SLPr2T10 | SPF₁₀ HPV33 | 34 | 1 | 1 | Negative | ID64 |
| 51SLPr2T20 | SPF₁₀ HPV51 | 8712 | 100 | 387 | Specific | ID64 |
| 51 SLPr2T20 | SPF₁₀ HPV33 | 32 | 0 | 1 | Negative | ID64 |
| 51 SLPr2T30 | SPF₁₀ HPV51 | 8077 | 100 | 414 | Specific | ID64 |
| 51SLPr2T30 | SPF₁₀ HPV33 | 30 | 0 | 1 | Negative | ID64 |
| 51SLPr2T40 | SPF₁₀ HPV51 | 7356 | 100 | 320 | Specific | ID64 |
| 51SLPr2T40 | SPF₁₀ HPV33 | 32 | 0 | 1 | Negative | ID64 |

**Table 12f.**

| **Probe** | **Hybridized to target** | **Signal (MFI)** | **target / probe (%)** | **Signal / noise** | **Remark** | **Exp** |
|---|---|---|---|---|---|---|
| 51 MLPr2 | MPF HPV52 | 423 | 100 | 13 | Specific | ID69 |
| 51MLPr2 | MPF HPV16 | 32 | 8 | 1 | Cross reaction | ID69 |
| 51 MLPr2T20 | MPF HPV52 | 1233 | 100 | 95 | Specific | ID69 |
| 51MLPr2T20 | MPF HPV16 | 11 | 1 | 1 | Negative | ID69 |
| 51 MLPr2T30 | MPF HPV52 | 1250 | 100 | 139 | Specific | ID69 |
| 51MLPr2T30 | MPF HPV 16 | 8 | 1 | 1 | Negative | ID69 |
| 51MLPr2T40 | MPF HPV52 | 1510 | 100 | 126 | Specific | ID69 |
| 51MLPr2T40 | MPF HPV16 | 9 | 1 | 1 | Negative | ID69 |

### Tables example 11:

**Table 13a. 18MLPr7 = MPF probe 18 version 7, C₁₂= a stretch of 12 carbon atoms, (T)₄₀ = a stretch of 40 Thymine nucleotides, (TTG)₁₃ = a stretch of 13 Thymine-Thymine-Guanine nucleotide triplets (39 nucleotides total)**

| **Name** | **Probe composition** |
|---|---|
| 18MLPr7T40 | NH₂-C₁₂-(T)₄₀-TTACATAAGGCACAGG [SEQ ID NO: 125] |
| 18MLPr7(TTG)₁₃ | NH₂-C₁₂-(TTG)₁₃-TTACATAAGGCACAGG [SEQ ID NO: 140] |

**Table 13b. nc8 = negative control 8 showing cross reaction with all probes in a LiPA assay, DNA- = negative control**

| **Probe** | **Hybridized to target** | **Signal (MFI)** | **target / probe (%)** | **Signal / noise** | **Remark** | **Exp** |
|---|---|---|---|---|---|---|
| 18MLPr7T40 | MPF HPV18 | 2001 | 100 | 13 | Specific | ID 169 |
| 18MLPr7T40 | nc8 | 1104 | 54 | 7 | Cross reaction | ID 169 |
| 18MLPr7T40 | DNA- | 2 | 0 | 0 | Negative | ID 169 |
| 18MLPr7(TTG)₁₃ | MPF HPV18 | 2390 | 100 | 199 | Specific | ID 169 |
| 18MLPr7(TTG)₁₃ | nc8 | 23 | 1 | 2 | Negative | ID 169 |
| 18MLPr7(TTG)₁₃ | DNA- | 2 | 0 | 0 | Negative | ID 169 |

### Tables example 12:

**Table 14a. 18MLPr7 = MPF probe 18 version 7, C₁₂ = a stretch of 12 carbon atoms, (T)₄₀ = a stretch of 40 Thymine nucleotides, N5 = 5'-end amino linker, N3 = 3'-end amino linker**

| **Name** | **Probe composition** |
|---|---|
| 18MLPr7T40N5 | NH₂-C₁₂-(T)₄₀-TTACATAAGGCACAGG [SEQ ID NO: 125] |
| 18MLPr7T40N3 | TTACATAAGGCACAGG-(T)₄₀-C₁₂-NH₂ [SEQ ID NO: 141] |
| 45MLPr8T40N5 | NH₂-C₁₂-(T)₄₀-CCAGGGCCATAACAAG [SEQ ID NO: 142] |
| 45MLPr8T40N3 | CCAGGGCCATAACAAG-(T)₄₀-C₁₂-NH₂ [SEQ ID NO: 143] |

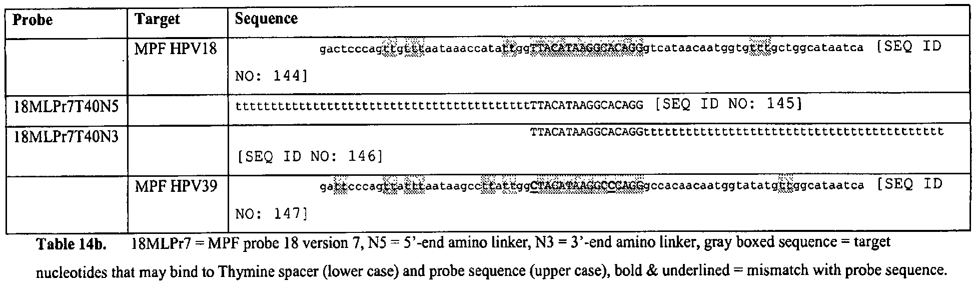

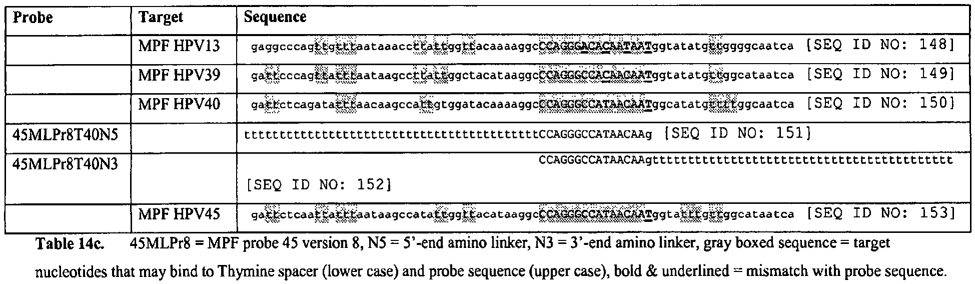

**Table 14d.**

| **Probe** | **Hybridized to target** | **Signal (MFI)** | **target / probe (%)** | **Signal / noise** | **Remark** | **Exp** |
|---|---|---|---|---|---|---|
| 18MLPr7T40N5 | MPF HPV18 | 1146 | 100 | 85 | Specific | ID141 |
| 18MLPr7T40N5 | MPF HPV39 | 518 | 45 | 38 | Cross reaction | ID141 |
| 18MLPr7T40N3 | MPF HPV18 | 694 | 100 | 139 | Specific | ID141 |
| 18MLPr7T40N3 | MPF HPV39 | 12 | 2 | 2 | Negative | ID141 |
| | | | | | | |
| 45MLPr8T40N5 | MPF HPV13 | 611 | 38 | 51 | Cross reaction | ID141 |
| 45MLPr8T40N5 | MPF HPV39 | 284 | 18 | 24 | Cross reaction | ID141 |
| 45MLPr8T40N5 | MPF HPV40 | 1021 | 64 | 85 | Cross reaction | ID141 |
| 45MLPr8T40N5 | MPF HPV45 | 1600 | 100 | 133 | Specific | ID141 |
| 45MLPr8T40N3 | MPF HPV 13 | 47 | 8 | 8 | Cross reaction | ID141 |
| 45MLPr8T40N3 | MPF HPV39 | 17 | 3 | 3 | Negative | ID141 |
| 45MLPr8T40N3 | MPF HPV40 | 116 | 19 | 19 | Cross reaction | ID141 |
| 45MLPr8T40N3 | MPF HPV45 | 615 | 100 | 103 | Specific | ID141 |

**Tables 15a and b:**

| **MFI** | | | | **% target/probe** | | |
|---|---|---|---|---|---|---|
| | **Bead/probe** | **Bead/probe** | | | **Bead/probe** | **Bead/probe** |
| **Target** | **A1** | **A2** | | **Target** | **A1** | **A2** |
| **a** | 988 | 4399 | | **a** | 100 | 100 |
| **b** | 13 | 14 | | **b** | 1 | 0 |
| **c** | 19 | 19,5 | | **c** | 2 | 0 |
| **d** | 5 | 13 | | **d** | 1 | 0 |
| **e** | 3 | 4 | | **e** | 0 | 0 |
| **f** | 11 | 6 | | **f** | 1 | 0 |
| **g** | 14 | 9 | | **g** | 1 | 0 |
| **h** | 3 | 3 | | **h** | 0 | 0 |

| | | | | | | |
|---|---|---|---|---|---|---|
| **% target / probe:** Al, a = 988/988 * 100 = 100%; Al, c = 19/988* 100 = 2% | | | | | | |

**Tables 16a and b:**

| **MFI** | | | | **Signal/noise** | | |
|---|---|---|---|---|---|---|
| | **Bead/probe** | **Bead/probe** | | | **Bead/probe** | **Bead/probe** |
| **Target** | **A1** | **A2** | | **Target** | **A1** | **A2** |
| A | 988 | 4399 | | a | 82 | 400 |
| B | 13 | 14 | | b | 1 | 1 |
| C | 19 | 19,5 | | c | 2 | 2 |
| D | 5 | 13 | | d | 0 | 1 |
| E | 3 | 4 | | e | 0 | 0 |
| F | 11 | 6 | | f | 1 | 1 |
| G | 14 | 9 | | g | 1 | 1 |
| H | 3 | 3 | | h | 0 | 0 |
| Median | 12 | 11 | | | | |

| | | | | | | |
|---|---|---|---|---|---|---|
| **Signal / noise:** Al, a = 988 / 12 (= median (988, 13, 19, 5, 3, 11, 14, 3)) = 82; Al, c = 19 / 12 (median (988, 13, 19, 5, 3, 11, 14, 3)) = 2. | | | | | | |

### Example 13

### HPV Probes suitable for use with bead based approaches, eg for Luminex based approaches:

**Table 17**

| **Name** | **Probe sequence** | **SEQ ID NO:** |
|---|---|---|
| 16MLP4T40N3 | GAGCACAGGGCCAC(T)₄₀ | 154 |
| 18MLPr7T40N3 | TTACATAAGGCACAGG(T)₄₀ | 146 |
| 26MLP7T40N3 | GTTACAACGTGCACAG(T)₄₀ | 155 |
| 31MLPr6T40N3 | GGATGCAACGTGCTC(T)₄₀ | 156 |
| 33MLPr4T40N5 | (T) ₄₀CATATTGGCTACAACGT | 157 |
| 35MLPr6T40N3 | GTGCACAAGGCCATA(T) ₄₀ | 158 |
| 39MLPr4T40N5 | (T) ₄₀GCCTTATTGGCTACATAA | 280 |
| 45MLPr6T40N5 | (T) ₄₀ggtGTTACATAAGGCCCAG | 160 |
| 45MLPr8T40N3 | CCAGGGCCATAACAAg (T) ₄₀ | 143 |
| 51MLPr2T40N5 | (T) ₄₀TTATTGGCTCCACCGT | 126 |
| 52MLPr2T40N5 | (T) ₄₀CCGTACTGGTTACAACGa | 138 |
| 53MLPr6T40N5 | (T) ₄₀ATATTGGCTGCAACGT | 161 |
| 56MLPr4T40N5 | (T) ₄₀GGCCCAAGGCCATAATAA | 162 |
| 58MLPr1T40N5 | (T) ₄₀CTTATTGGCTACAGCGT | 163 |
| 58MLPr5T40N3 | ACAGCGTGCACAAGG(T) ₄₀ | 164 |
| 59MLPr3T40N5 | (T) ₄₀CAAGGCTCAGGGTTTAA | 165 |
| 66MLPr6T40N3 | TGCACAGGGCCATA(T) ₄₀ | 166 |
| 66MLPr7T40N3 | TGCAACGTGCACAG (T) ₄₀ | 167 |
| 68MLPr8T40N5 | (T) ₄₀CTGCACAAGGCACAG | 168 |
| 68MLPr10T40N3 | GCACAAGGCACAGG(T) ₄₀ | 169 |
| 70MLPr4T40N5 | (T) ₄₀CCTATTGGTTGCATAAGG | 170 |
| 82MLPr3T40N3 | ATTGGTTGCATCGCG(T) ₄₀ | 171 |

In one aspect of the invention any 1, 2, 3, 4, 5, 6, 7, 8, 9, 10, 11, 12,13,14,15,16,17, 18, 19, 20, 21 or all 22 all the above probes may be used in a bead- based multiplex reaction under identical conditions for simultaneous detection of any HPV target DNA present in a sample. Such bead sets are suitable for use in the optimized reaction scheme outlined above. An additional polycarbon spacer may be incorporated.

### Example 14: Universal detection of HPV MPF amplimers in a 96 well microtiter plate assay, DNA Enzyme Immuno Assay (DEIA)

### Introduction

This example describes the use of a mixture of 8 probes for universal detection of HPV amplimers obtained after broad spectrum PCR with MPF primers.

(Within this work we have referred to the analysis of the regions of Figure 1 as MPF analysis, and the primers and probes used therein as MPF primers and probes. The amplified region is the MPF amplimer. In this way the primers and probes are differentiated from the "SPF10" primer and probe set also developed in this laboratory which are used in the analysis of a different region of the L1 gene.)

### Materials and Methods

For universal detection of HPV MPF amplimers, probes were selected from the alignment of HPV sequences in Figure 1. The sequences of the universal DEIA probes are listed in table 3.

MPF amplimers were obtained by amplification of HPV plasmids containing HPV genotypes 6, 11, 13, 16, 18, 26, 30, 31, 32, 33, 34, 35, 39, 43, 44, 45, 51, 52, 53, 54, 55, 56, 57, 58, 59, 66, 67, 68, 69, 70, 71 and 74 (kindly provided either by Dr. E-M. de Villiers, Dr. R. Ostrow, Dr. A. Lorincz, Dr. T. Matsukura, and Dr. G. Orth) or oligonucleotide sequences representing HPV genotypes 7, 40, 42, 61, 72, 73, 81-87, 90, 91 and 2 variant sequences of HPV genotype 16.

HPV DNA amplification was performed in a final volume of 50 µl, containing 10 µl of target DNA, 1x PCR buffer II (Perkin Elmer), 3.0 mM MgCl₂, 0.2 mM deoxynucleoside triphosphate, 10 pmol of each forward and reverse primer (table 1 and 2) and 1.5U of AmpliTaqGold (Perkin Elmer, Branchburg, New Jersey, USA). The PCR conditions were as follows: preheating for 9 min at 94°C, followed by 40 cycles of 30 seconds at 94°C, 45 seconds at 52°C and 45 seconds at 72°C, and a final extension at 72°C.

Amplimers, synthesized by biotinylated MPF PCR primers, were detected by hybridization to a mixture of 8 HPV-specific probes (see preferred probes of table 3). Ten microliters of PCR product was diluted in 100 µl of hybridization buffer (150 mmol/L NaCl, 15 mmol/L sodium citrate, pH 7.0, 0.1% Tween 20) and incubated at 42°C for 30 minutes in streptavidin-coated microtiter plates. Noncaptured materials were removed by three washes with hybridization buffer. The double-stranded captured PCR products were denatured by addition of 100 µl of denaturation solution (100 mmol/L NaOH) and incubated for 5 minutes at room temperature, followed by three washes with hybridization buffer. A mixture of digoxigenin (DIG)-labeled HPV-specific probes (see preferred probes of table 3) were diluted in hybridization buffer and added to the well and incubated at 42°C for 45 minutes. Wells were washed three times, and anti-DIG alkaline phosphatase conjugate was added and incubated at 42°C for 15 minutes. After five washes, substrate was added and incubated at room temperature for 15 minutes. The reaction was stopped by adding 100 µl of 0.5 mmol/L H₂SO₄. Optical densities (OD) were determined at 450 nm in a microtiter plate reader. Samples were considered positive if the OD₄₅₀ was 2.5 times higher than the negative PCR control (cut-off value). In each run, negative controls as well as positive and borderline positive controls were tested together with the samples.

### Results

All amplimers of HPV genotypes 6, 7, 11, 13, 16, 18, 26, 30-35, 39, 40, 42-45, 51-59, 66-74, 81-87, 90, 91 and 2 variant sequences of HPV genotype 16 were reactive with the mixture of 8 selected probes.

### Discussion

A mixture of 8 probes was developed for universal detection of HPV MPF amplimers. The 8 selected probes were successful in detection of the various HPV genotypes, although amplimers of HPV genotype 51, 57, 71, 84, 87, 13, 91, 11, 59, 30, 44, 55, 70, 52, 69, 84, 86, 74 and 2 variants of genotype 16 show 1 nucleotide mismatch to the best matching probe

### .Example 15: Development of a HPV MPF genotyping assay

### Introduction

This example describes an HPV MPF genotyping assay for simultaneous detection and identification of HPV genotypes. After HPV broad spectrum amplification by using MPF primers, synthesize amplimers can be detected and identified by hybridization to genotype specific probes that are applied on a reverse hybridisation strip.

### Materials and methods

### Selection of probes:

Based on the 31 bp sequences located between the forward and reverse primer target sequences of table 1 and 2, type-specific probes were selected. These probe sequences are listed in table 4 and table 18 below.

### HPV plasmids and HPV oligo's

Selected probes were analysed for analytical sensitivity and specificity. HPV MPF amplimers were obtained by PCR using 10 MPF forward primers and 8 MPF reverse primers containing a biotin moiety at the 5' end, see tables 1 and 2. HPV PCR was performed as described in example 1.

### Development of a HPV MPF reverse hybridisation genotyping assay:

For simultaneous detection and identification of different HPV genotypes a reverse hybridisation genotyping assay was developed. Analysis of multiple probes in a single hybridisation step requires selection of type-specific probes that have similar hybridisation characteristics.

In this experiment probes were chosen for HPV types 16, 18, 26, 31, 33, 35, 39, 45, 51, 52, 53, 56, 58, 59, 66, 68, 70, 82 and 2 confirmation probes for type 53 and 66. The probe name start with the HPV type number, except probes selected for confirmation. Those probes start with a 'c' followed by HPV type number. Probe c53L1nPr3 is selected for exclusion of type 61 and c66L1nPr5 is selected for exclusion of type 89.

Oligonucleotide probes were selected and ordered with a poly-T tail at the 5' or 3' end, respectively. These probes were immobilized in parallel lines on a nitrocellulose strip. To control the conjugate and substrate reaction, biotinylated DNA was also applied on the strip.

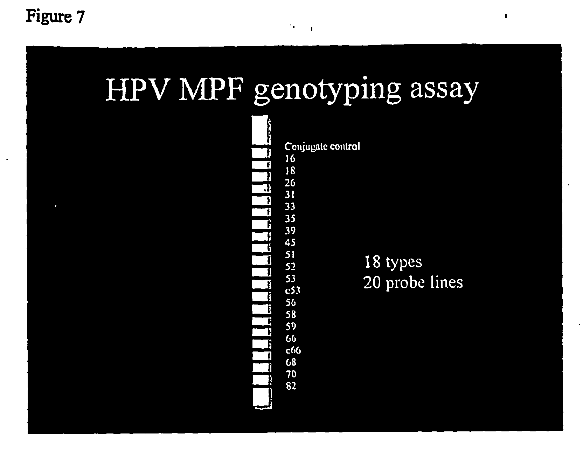

A possible outline of a strip that might be used is shown in figure 7.

Ten microliters of PCR product, containing biotin moieties at the 5' ends of the primers, was denatured by adding 10 µl of NaOH solution. After 10 min, a reverse hybridisation strip was put into the tray. Two milliliters of prewarmed (37°C) hybridization buffer (3× SSC [1× SSC is 15 mM Na-citrate and 150 mM NaCl], 0.1% sodium dodecyl sulfate) was added and incubated at 54 ± 0.5°C for 1 h. All incubations and washing steps were performed automatically in an Auto-LiPA. The strips were washed twice for 30 s and once for 30 min at 54°C with 2 ml of hybridization solution. Following this stringent wash, the strips were incubated with 2 ml of alkaline phosphatase-streptavidin conjugate for 30 min at room temperature. Strips were washed twice with 2 ml of rinse solution (phosphate buffer containing NaCL, Triton and 0.5%NaN₃) and once with 2 ml of substrate buffer. Two milliliters of substrate (5-bromo-4-chloro-3-indolylphosphate and nitroblue tetrazolium) was added and incubated for 30 min at room temperature. The reaction was stopped by aspiration of the substrate solution and addition of 2 ml of distilled water. After drying, the strip results were interpreted by eye.

### Results:

Amplimers obtained from HPV types 16, 18, 26, 31, 33 and 35 were used in a reverse hybridisation experiment to determine the specificity of the selected probes from table 18.

**Table 18**

| **name** | **Probe sequence** | **Start** | **length** | **T-tail 100xT** | **SEQ ID NO:** |
|---|---|---|---|---|---|
| **16L1nPr5.CH** | AGCACAGGGCCACA | 39 | 14 | 3' | 172 |
| **18L1nPr7.CH** | TTACATAAGGCACAGG | 31 | 16 | 3' | 293 |
| **26L1nPr7.CH** | GTTACAACGTGCACAG | 30 | 16 | 3' | 173 |
| **31L1nPr4.CH** | ACCATATTGGATGCAAC | 21 | 17 | 5' | 174 |
| **33L1nPr3.CH** | CCATATTGGCTACAACG | 22 | 17 | 5' | 304 |
| **35L1nPr6.CH** | GTGCACAAGGCCATA | 38 | 15 | 3' | 175 |
| **39L1nPr6.CH** | GCCTTATTGGCTACATAAG | 21 | 19 | 5' | 176 |
| **45L1nPr10.CH** | TTACATAAGGCCCAGG | 31 | 16 | 3' | 177 |
| **51L1nPr4.CH** | ggATTGGCTCCACCGTG | 24 | 15 | 5' | 178 |
| **52L1nPr4.CH** | ACCGTACTGGTTACAAC | 21 | 17 | 5' | 179 |
| **53L1CPr6.CH** | ATATTGGCTGCAACGT | 24 | 16 | 5' | 180 |
| **c53L1nPr3.CH** | ACGTGCCCAGGGAC | 36 | 14 | 5' | 181 |
| **56L1nPr6.CH** | TGCCCAAGGCCATAAT | 39 | 16 | 5' | 335 |
| **58L1nPr1.CH** | CTTATTGGCTACAGCGT | 23 | 17 | 5' | 336 |
| **59L1nPr3.CH** | CAAGGCTCAGGGTTTAA | 36 | 17 | 5' | 182 |
| **66L1nPr6.CH** | TGCACAGGGCCATA | 39 | 14 | 3' | 183 |
| **c66L1nPr5.CH** | GCAACGTGCACAGG | 33 | 14 | 3' | 184 |
| **68L1nPr10.CH** | GCACAAGGCACAGG | 33 | 14 | 3' | 185 |
| **70L1nPr4.CH** | CCTATTGGTTGCATAAGG | 23 | 18 | 5' | 358 |
| **82L1nPr3.CH** | ATTGGTTGCATCGCG | 26 | 15 | 3' | 186 |

| | | | | | |
|---|---|---|---|---|---|
| Lowercase is not type specific sequence | | | | | |

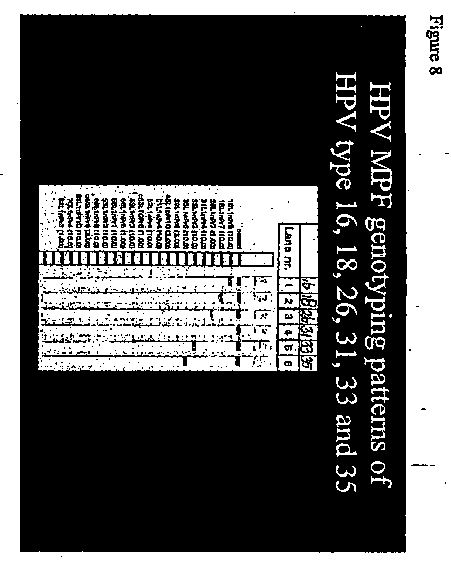

Results are shown in figure 8.

### Conclusion

The reverse hybridisation assay permits at least positive identification of HPV types 16, 18, 26, 31, 33 and 35. Thus the corresponding probes can also be used simultaneously in a multiplex reaction. The assay can be extended by adding probes for all other genital HPV types.

### Example 16: A High-risk MPF HPV DNA Enzyme ImmunoAssay (HR MPF HPV DEIA) for Detection of 13 High-risk HPV Genotypes

### Introduction

This example describes the use of a mixture of 13 digoxigenin-labeled HPV type-specific oligonucleotide probes in a DNA Enzyme ImmunoAssay (DEIA) for specific and simultaneous detection in microtiter plates of amplimers of 13 (selected) high-risk genotypes of HPV (types 16, 18, 31, 33, 35, 39, 45, 51, 52, 56, 58, 59, and 68) obtained after broad spectrum PCR, while amplimers of other HPV genotypes remain undetected.

### Materials and Methods

After universal HPV amplification, synthesized biotinylated amplimers can be detected in an DEIA by hybridization to a mixture of 13 high-risk HPV-specific digoxigen-labeled oligonucleotide probes (best choice table 19). The sequences of these probes were selected from the alignment of HPV sequences in figure 1, and are listed in table 19. Some oligonucleotide probes contain locked nucleic acids (LNAs).

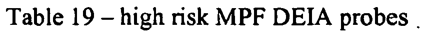

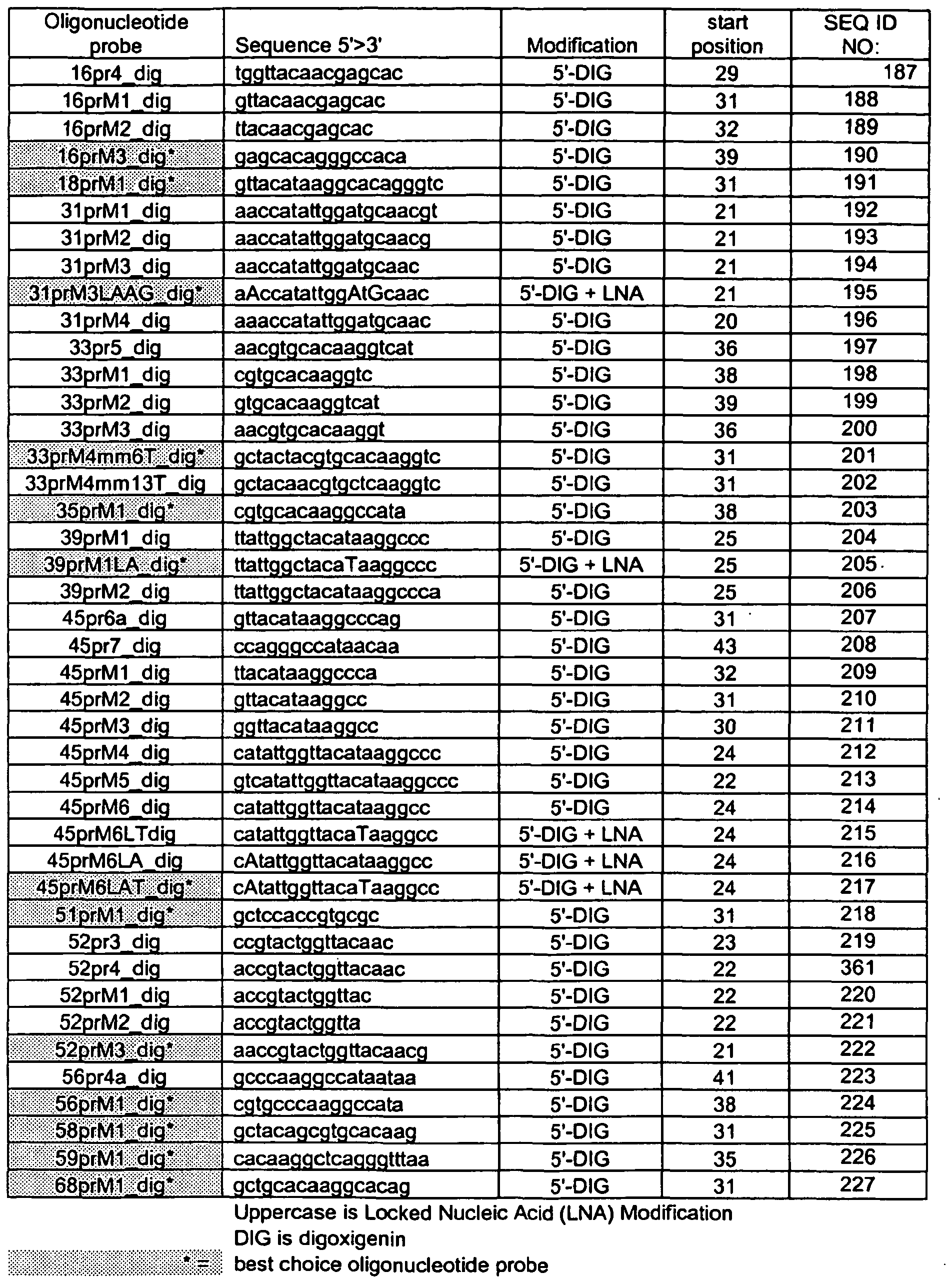

For evaluation of specificity of the DEIA, MPF amplimers were obtained by amplification of HPV plasmids containing HPV genotypes 6, 11, 16, 18, 26, 30, 31, 33, 34, 35, 39, 43, 44, 45, 51, 52, 53, 54, 55, 56, 58, 59, 66, 67, 68, 69, 70, 71 and 74 (kindly provided either by Dr. E-M. de Villiers, Dr. R. Ostrow, Dr. A. Lorincz, Dr. T. Matsukura, and Dr. G. Orth) or oligonucleotide sequences representing HPV genotypes 7, 40, 42, 61, 72, 81, 82, 83, 84, 85, 87, 91 and 2 variant sequences of HPV genotype 16.

HPV DNA amplification was performed in a final volume of 50 µl, containing 10 µl of target DNA, 1x PCR buffer II (Perkin Elmer), 3.0 mM MgCl₂, 0.2 mM deoxynucleoside triphosphate, 10 pmol of each forward and reverse primer (tables 1 and 2) and 1.5U of AmpliTaqGold (Perkin Elmer, Branchburg, New Jersey, USA). The PCR conditions were as follows: preheating for 9 min at 94°C, followed by 40 cycles of 30 seconds at 94°C, 45 seconds at 52°C and 45 seconds at 72°C, and a final extension of 5 minutes at 72°C.

Ten microliters of PCR product, synthesized by biotinylated MPF PCR primers, was diluted in 100 µl of hybridization buffer (150 mmol/L NaCl, 15 mmol/L sodium citrate, pH 7.0, 0.1 % Tween 20) and incubated at 45°C for 30 minutes in streptavidin-coated microtiter plates. Noncaptured materials were removed by three washes with hybridization buffer. The double-stranded captured PCR products were denatured by addition of 100 µl of denaturation solution (100 mmol/L NaOH) and incubated for 5-15 minutes at room temperature, followed by three washes with hybridization buffer. A mixture of digoxigenin (DIG)-labeled HPV-specific probes (see preferred probes of table 3). was diluted in hybridization buffer and added to the well and incubated at 45°C for 45 minutes. Wells were washed three times with stringent wash solution (37.5 mmol/L NaCl, 3.75 mmol/L sodium citrate, pH 7.0, 0.025% Tween 20), and 300 µl of stringent wash solution was added to the wells and incubated at 45°C for 45 minutes. Wells were washed twice with stringent wash solution and twice with hybridization buffer. Subsequently, anti-DIG alkaline phosphatase conjugate was added and incubated at 45°C for 15 minutes. After five washes, substrate was added and incubated at room temperature for 15 minutes. The reaction was stopped by adding 100 µl of 0.5 mmol/L H₂SO₄. Optical densities (OD) were determined at 450 nm in a microtiter plate reader. Samples were considered positive if the OD₄₅₀ was 2.5 times higher than the negative control. In each run, negative controls as well as positive and borderline controls were tested together with the clinical samples.

### Results

All amplimers of HPV genotypes 16, 18, 31, 33, 35, 39, 45, 51, 52, 56, 58, 59, and 68 and 2 variant sequences of HPV genotype 16 were reactive with the mixture of 13 selected probes, while amplimers of HPV genotypes 6, 7, 11, 26, 30, 34, 40, 42, 43, 44, 53, 54, 55, 61, 66, 67, 69, 70, 71, 72, 74, 81, 82, 83, 84, 85, 87, and 91 remain undetected.

### Discussion

The described HR MPF HPV DEIA detects simultaneously HPV high-risk genotypes 16, 18, 31, 33, 35, 39, 45, 51, 52, 56, 58, 59, and 68, while other HPV genotypes remain undetected. The 13 selected high-risk genotypes can de detected after universal PCR using the novel developed primer set as described in this patent. The detection assay can still be extended with probes for other potential high-risk HPV genotypes

### Example 17: Sensitivity of the universal MPF HPV DEIA and the HR MPF HPV DEIA

### Introduction

This example describes the determination of the analytical sensitivity of the universal MPF HPV DEIA and the HR MPF HPV DEIA and comparison to the SPF10 detection and typing system.

### Materials and Methods

For evaluation of analytical sensitivity of the universal MPF HPV DEIA and the HR MPF HPV DEIA, MPF amplimers were obtained by amplification of 10-fold dilutions of HPV plasmids containing HPV genotypes 18, 31, 33, 35, and 45 (kindly provided either by Dr. E-M. de Villiers, Dr. R. Ostrow, Dr. A. Lorincz, Dr. T. Matsukura, and Dr. G. Orth).

SPF10 PCR and amplimer analysis was performed according to Kleter et al 1998 and 1999 **[**Kleter, B., L. J. van Doorn, L. Schrauwen, A. Molijn, S. Sastrowijoto, J. ter Schegget, J. Lindeman, B. ter Harmsel, and W. G. V. Quint. 1999. Development and clinical evaluation of a highly sensitive PCR-reverse hybridization line probe assay for detection and identification of anogenital human papillomavirus. J.Clin.Microbiol. 37:2508-2517; Kleter, B., L. J. van Doorn, J. ter Schegget, L. Schrauwen, C. van Krimpen, M. P. Burger, B. ter Harmsel, and W. G. V. Quint. 1998. A novel short-fragment PCR assay for highly sensitive broad-spectrum detection of anogenital human papillomaviruses. Am.J.Pathol. 153:1731-1739]

### Results - See below

Using a borderline of 2.5 times the OD₄₅₀ of the negative control, the calculated analytical sensitivity of the universal MPF HPV DEIA and HR MPF HPV DEIA varied from 12 to 72 ag (corresponding to an equivalent of approximately 2 to 15 copies of the viral genome) and 48 to 722 ag (corresponding to an equivalent of approximately 10 to 150 copies of the viral genome), respectively. The formal limit of detection testing has not yet been performed.

**Results - table 20a - e**

| | | | ∼one copy | |
|---|---|---|---|---|
| | | | | |
| HPV18 | 4.8 fq/PCR | 480 ag/PCR | 48 ag/PCR | 4.8 ag/PCR |
| SPF10 | | | | |
| DEIA | + | + | + | + |
| SFP10 LiPA | + | + | + | + |
| MPF DEIA | + | + | + | - |
| HR MPF | | | | |
| DEIA | + | + | + | - |
| 20a | | | | |

| HPV31 | 5.6 fg/PCR | 560 ag/PCR | 56 aq/PCR | 5.6 ag/PCR |
|---|---|---|---|---|
| SPF10 | | | | |
| DEIA | + | + | + | - |
| SFP10 LiPA | + | + | + | - |
| MPF DEIA | + | + | + | - |
| HR MPF | | | | |
| DEIA | + | + | + | - |
| 20b | | | | |

| HPV33 | 4.9 fg/PCR | 490 ag/PCR | 49 ag/PCR | 4.9 ag/PCR |
|---|---|---|---|---|
| SPF10 | | | | |
| DEIA | + | + | - | - |
| SFP10 LiPA | + | + | +/- | - |
| MPF DEIA | + | + | + | - |
| HR MPF | | | | |
| DEIA | + | + | + | - |
| 20c | | | | |

| HPV35 | 7.22 fg/PCR | 722 ag/PCR | 72.2 ag/PCR | 7.22 ag/PCR |
|---|---|---|---|---|
| SPF10 | | | | |
| DEIA | + | + | + | - |
| SFP10 LiPA | + | + | + | - |
| MPF DEIA | + | + | + | - |
| HR MPF | | | | |
| DEIA | + | + | - | - |
| 20d | | | | |

| HPV45 | 12 fg/PCR | 1.2 fg/PCR | 120 ag/PCR | 12 ag/PCR |
|---|---|---|---|---|
| SPF10 DEIA | + | + | + | - |
| SFP10 LiPA | + | + | + | - |
| MPF DEIA | + | + | + | + |
| HR MPF DEIA | + | + | + | - |
| 20e | | | | |

### Discussion

In summary, the universal MPF HPV DEIA and HR MPF HPV DEIA have similar sensitivities as the SPF10 DEIA and LiPA.

## Claims

1. A method for detection and/or typing of Human papillomavirus (HPV) present in a biological sample, the method comprising the steps of:
(i) amplification of a polynucleic acid fragment comprising nucleotides 6566 - 6596 of the HPV 16 reference sequence K02718 (or K02718.1) having the sequence 5' TTATTGGTTACAACGAGCACAGGGCCACAAT3' or equivalent region from other HPV types (B Region) in the sample by use of:
- a 5' primer specifically hybridizing to the 'A' region of the genome of HPV 16 or equivalent region in another HPV genome, said 'A' region being indicated in Figure 1, and the A region of the reference sequence is 5' GATGCCCAAATATTCAATAAACC 3'.
- a 3' primer specifically hybridizing to the 'C' region of the genome of at least one HPV type, said 'C' region being indicated in Figure 1 and the C region of the reference sequence is 5' AATGGCATTTGTTGGGGTAACCA 3'.
(ii) hybridizing the amplified fragments from step (i) with at least one probe capable of specific hybridization to nucleotides 6566 - 6596 of the HPV 16 reference sequence K02718 (or K02718.1) or equivalent region from other HPV types.

2. A method according to claim 1 wherein the probe is capable of specific hybridization within the B region of the genome of only one HPV type.

3. A method according to Claim 1 or 2, hybridizing the polynucleic acid fragments amplified fragments from in step (i) with a plurality of probes comprising nucleotides 6566-6596 with reference to the sequence of HPV 16 sequence Genbank accession number K02718.1, or positions equivalent thereto in other HPV sequences, wherein each probe is capable of specific hybridization with a genome of only one HPV type.

4. A method according to claim 1, 2 or 3 wherein the probe is selected from the list consisting of the sequences listed in Tables 4, 5-14, 17, 18, 19.

5. A method according to any preceding claim wherein the amplification step uses a primer selected from the list comprising: HPV-MPF1F1 (SEQ ID No. 1), HPV-MPF1F2 (SEQ ID No. 2), HPV-MPF1F3 (SEQ ID No. 3), HPV-MPF1F4 (SEQ ID No. 4), HPV-MPF1F5 (SEQ ID No. 5), HPV-MPF1F6 (SEQ ID No. 6), HPV-MPF1F7 (SEQ ID No. 7), HPV-MPF1F8 (SEQ ID No. 8), HPV-MPF1F9 (SEQ ID No. 9), HPV-MPF1F10 (SEQ ID No. 10), HPV-MPF2R1 (SEQ ID No. 11), HPV-MPF2R2 (SEQ ID No. 12), HPV-MPF2R3 (SEQ ID No. 13), HPV-MPF2R4 (SEQ ID No. 14), HPV-MPF2R5 (SEQ ID No. 15), HPV-MPF2R6 (SEQ ID No. 16), HPV-MPF2R7 (SEQ ID No. 17), HPV-MPF2R8 (SEQ ID No. 18).

6. A method according to any preceding claim wherein the presence of HPV nucleic acid is confirmed in the sample prior to the typing step.

7. A method according to any preceding claim wherein the hybridization between the probe and target is carried out in the presence of a solid support.

8. A method according to claim 7 wherein the hybridization step uses a reverse hybridization format.

9. A method according to claim 7 wherein the probe is directly or indirectly attached onto a bead, optionally a florescent bead.

10. A method according to claim 9 wherein detection of hybridization is analysed using flow cytometry.

11. A kit comprising a forward and reverse primer set and a third oligonucleotide typing probe capable of hybridizing to region 6566-6595 of the HPV 16 reference sequence K02718 as defined in claim 1 or equivalent region from other HPV types (B region) wherein the primers are selected from the list consisting of HPV-MPF1F1 (SEQ ID No. 1), HPV-MPF1F2 (SEQ ID No. 2), HPV-MPF1F3 (SEQ ID No. 3), HPV-MPF1F4 (SEQ ID No. 4), HPV-MPF1F5 (SEQ ID No. 5), HPV-MPF1F6 (SEQ ID No. 6), HPV-MPF1F7 (SEQ ID No. 7), HPV-MPF1F8 (SEQ ID No. 8), HPV-MPF1F9 (SEQ ID No. 9), HPV-MPF1F10 (SEQ ID No. 10), HPV-MPF2R1 (SEQ ID No. 11), HPV-MPF2R2 (SEQ ID No. 12), HPV-MPF2R3 (SEQ ID No. 13), HPV-MPF2R4 (SEQ ID No. 14), HPV-MPF2R5 (SEQ ID No. 15), HPV-MPF2R6 (SEQ ID No. 16), HPV-MPF2R7 (SEQ ID No. 17), HPV-MPF2R8 (SEQ ID No. 18).

12. A kit comprising at least 2 probes capable of specific hybridization to the B region of HPV genome.

13. A kit according to claim 12 wherein the probes are any two probes selected form any of Table 4, 5-14, 17, 18, 19.

14. A kit according to claim 13 comprising a pair of forward and reverse primers of Table 1 or 2

15. A kit as claimed in any of claims 12 to 14 additionally comprising a probe from table 3.

## Patentansprüche

1. Verfahren zur Detektion und/oder Typisierung von in einer biologischen Probe vorhandenem humanem Papillomavirus (HPV), wobei das Verfahren die Schritte umfasst:
(i) Amplifikation eines Polynukleinsäure-Fragments, das die Nukleotide 6566-6596 der HPV 16-Referenzsequenz K02718 (oder K02718.1) mit der Sequenz 5' TTATTGGTTACAACGAGCACAGGGCCACAAT3' oder eine äquivalente Region aus anderen HPV-Typen (B-Region) umfasst, in der Probe durch Verwendung:
- eines 5'-Primers, der spezifisch an die 'A'-Region des Genoms von HPV 16 oder an eine äquivalente Region in einem anderen HPV-Genom hybridisiert, wobei die 'A'-Region in Figur 1 angegeben ist und die A-Region der Referenzsequenz 5' GATGCCCAAATATTCAATAAACC 3' ist;
- eines 3'-Primers, der spezifisch an die 'C'-Region des Genoms mindestens eines HPV-Typs hybridisiert, wobei die 'C'-Region in Figur 1 angegeben ist und die C-Region der Referenzsequenz 5' AATGGCATTTGTTGGGGTAACCA 3' ist;
(ii) Hybridisierung der amplifizierten Fragmente aus Schritt (i) mit mindestens einer Sonde, die fähig ist zur spezifischen Hybridisierung an die Nukleotide 6566-6596 der HPV 16-Referenzsequenz K02718 (oder K02718.1) oder an eine äquivalente Region aus anderen HPV-Typen.

2. Verfahren gemäß Anspruch 1, wobei die Sonde zur spezifischen Hybridisierung innerhalb der B-Region des Genoms nur eines HPV-Typs fähig ist.

3. Verfahren gemäß Anspruch 1 oder 2, das die von den Polynukleinsäure-Fragmenten amplifizierten Fragmente aus Schritt (i) mit einer Vielzahl von Sonden hybridisiert, umfassend die Nukleotide 6566-6596 mit Referenz zur Sequenz der Genbank-Eingangsnummer K02718.1 der HPV 16-Sequenz, oder Positionen in anderen HPV-Sequenzen, die dazu äquivalent sind, wobei jede Sonde zur spezifischen Hybridisierung mit einem Genom nur eines HPV-Typs fähig ist.

4. Verfahren gemäß Anspruch 1, 2 oder 3, wobei die Sonde ausgewählt ist aus der Liste, die aus den Sequenzen, die in den Tabellen 4, 5 bis 14, 17, 18 und 19 aufgelistet sind, besteht.

5. Verfahren gemäß einem der vorhergehenden Ansprüche, wobei der Amplifikationsschritt einen Primer verwender, der ausgewählt ist aus der Liste, die umfasst: HPV-MPF1F1 (SEQ ID Nr.: 1), HPV-MPF1F2 (SEQ ID Nr.: 2), HPV-MPF1F3 (SEQ ID Nr.: 3), HPV-MPF1F4 (SEQ ID Nr.: 4), HPV-MPF1F5 (SEQ ID Nr.: 5), HPV-MPF1F6 (SEQ ID Nr.: 6), HPV-MPF1F7 (SEQ ID Nr.: 7), HPV-MPF1F8 (SEQ ID Nr.: 8), HPV-MPF1F9 (SEQ ID Nr.: 9), HPV-MPF1F10 (SEQ ID Nr.: 10), HPV-MPF2R1 (SEQ ID Nr.: 11), HPV-MPF2R2 (SEQ ID Nr.: 12), HPV-MPF2R3 (SEQ ID Nr.: 13), HPV-MPF2R4 (SEQ ID Nr.: 14), HPV-MPF2R5 (SEQ ID Nr.: 15), HPV-MPF2R6 (SEQ ID Nr.: 16), HPV-MPF2R7 (SEQ ID Nr.: 17), HPV-MPF2R8 (SEQ ID Nr.: 18).

6. Verfahren gemäß einem der vorhergehenden Ansprüche, wobei das Vorhandensein der HPV-Nukleinsäure in der Probe vor dem Typisierungsschritt bestätigt wird.

7. Verfahren gemäß einem der vorhergehenden Ansprüche, wobei die Hybridisierung zwischen der Sonde und dem Zielobjekt in Gegenwart eines festen Supports durchgeführt wird.

8. Verfahren gemäß Anspruch 7, wobei der Hybridisierungsschritt ein umgekehrtes Hybridisierungsformat verwendet.

9. Verfahren gemäß Anspruch 7, wobei die Sonde direkt oder indirekt an ein Kügelchen angeheftet ist, ggf. ein fluoreszentes Kügelchen.

10. Verfahren gemäß Anspruch 9, wobei die Detektion der Hybridisierung mittels Durchflusszytometrie analysiert wird.

11. Kit, umfassend einen Vorwärts- und Rückwärts-Primersatz und eine dritte Oligonukleotid-Typisierungs-Sonde, die fähig ist zur Hybridisierung an die wie in Anspruch 1 definierte Region 6566-6595 der HPV 16-Referenzsequenz K02718 oder an eine äquivalente Region aus anderen HPV-Typen (B-Region), wobei die Primer ausgewählt sind aus der Liste bestehend aus HPV-MPF1F1 (SEQ ID Nr.: 1), HPV-MPF1F2 (SEQ ID Nr.: 2), HPV-MPF1F3 (SEQ ID Nr.: 3), HPV-MPF1F4 (SEQ ID Nr.: 4), HPV-MPF1F5 (SEQ ID Nr.: 5), HPV-MPF1F6 (SEQ ID Nr.: 6), HPV-MPF1F7 (SEQ ID Nr.: 7), HPV-MPF1F8 (SEQ ID Nr.: 8), HPV-MPF1F9 (SEQ ID Nr.: 9), HPV-MPF1F10 (SEQ ID Nr.: 10), HPV-MPF2R1 (SEQ ID Nr.: 11), HPV-MPF2R2 (SEQ ID Nr.: 12), HPV-MPF2R3 (SEQ ID Nr.: 13), HPV-MPF2R4 (SEQ ID Nr.: 14), HPV-MPF2R5 (SEQ ID Nr.: 15), HPV-MPF2R6 (SEQ ID Nr.: 16), HPV-MPF2R7 (SEQ ID Nr.: 17), HPV-MPF2R8 (SEQ ID Nr.: 18).

12. Kit, das mindestens 2 Sonden umfasst, die fähig sind zur spezifischen Hybridisierung an die B-Region des HPV-Genoms.

13. Kit gemäß Anspruch 12, wobei die Sonden beliebige zwei Sonden sind, die ausgewählt sind aus einer der Tabellen 4, 5 bis 14, 17, 18, 19.

14. Kit gemäß Anspruch 13, das ein Paar von Vorwärts- und Rückwärts-Primern von Tabelle 1 oder 2 umfasst.

15. Kit, wie in einem der Ansprüche 12 bis 14 beansprucht, das zusätzlich eine Sonde aus Tabelle 3 umfasst.

## Revendications

1. Procédé de détection et/ou de typage de papillomavirus humain (HPV) présent dans un échantillon biologique, le procédé comprenant les étapes suivantes :
(i) l'amplification d'un fragment d'acide polynucléique comprenant les nucléotides 6566 à 6596 de la séquence de référence du HPV 16 K02718 (ou K02718.1) ayant la séquence 5' TTATTGGTTACAACGAGCACAGGGCCACAAT 3' ou une région équivalente issue d'autres types de HPV (région B) dans l'échantillon par l'utilisation :
d'une amorce en 5' s'hybridant spécifiquement à la région A du génome du HPV 16 ou à une région équivalente dans le génome d'un autre HPV, ladite région A étant indiquée sur la figure 1 et la région A de la séquence de référence est 5' GATGCCCAAATATTCAATAAACC 3' ;
d'une amorce en 3' s'hybridant spécifiquement à la région C du génome d'au moins un type de HPV, ladite région C étant indiquée sur la figure 1 et la région C de la séquence de référence est 5' AATGGCATTTGTTGGGGTAACCA 3' ;
(ii) l'hybridation des fragments amplifiés issus de l'étape (i) avec au moins une sonde capable d'hybridation spécifique aux nucléotides 6566 à 6596 de la séquence de référence du HPV 16 K02718 (ou K02718.1) ou à une région équivalente provenant d'autres types de HPV.

2. Procédé selon la revendication 1, dans lequel la sonde est capable d'une hybridation spécifique au sein de la région B du génome de seulement un type de HPV.

3. Procédé selon la revendication 1 ou 2, hybridant les fragments amplifiés des fragments d'acide polynucléique issus de l'étape (i) avec une pluralité de sondes comprenant les nucléotides 6566 à 6596 avec référence à la séquence du HPV 16 de numéro d'accession GenBank K02718.1, ou des positions qui y sont équivalentes dans des séquences d'autres HPV, où chaque sonde est capable d'une hybridation spécifique avec un génome de seulement un type de HPV.

4. Procédé selon la revendication 1, 2 ou 3 dans lequel la sonde est choisie dans la liste constituée des séquences énumérées dans les tableaux 4, 5 à 14, 17, 18, 19.

5. Procédé selon l'une quelconque des revendications précédentes, dans lequel l'étape d'amplification utilise une amorce choisie dans la liste comprenant : HPV-MPF1F1 (SEQ ID NO : 1), HPV-MPF1F2 (SEQ ID NO : 2), HPV-MPF1F3 (SEQ ID NO : 3), HPV-MPF1F4 (SEQ ID NO : 4), HPV-MPF1F5 (SEQ ID NO : 5), HPV-MPF1F6 (SEQ ID NO : 6), HPV-MPF1F7 (SEQ ID NO : 7), HPV-MPF1F8 (SEQ ID NO : 8), HPV-MPF1F9 (SEQ ID NO : 9), HPV-MPF1F10 (SEQ ID NO : 10), HPV-MPF2R1 (SEQ ID NO : 11), HPV-MPF2R2 (SEQ ID NO : 12), HPV-MPF2R3 (SEQ ID NO : 13), HPV-MPF2R4 (SEQ ID NO : 14), HPV-MPF2R5 (SEQ ID NO : 15), HPV-MPF2R6 (SEQ ID NO : 16), HPV-MPF2R7 (SEQ ID NO : 17), HPV-MPF2R8 (SEQ ID NO : 18).

6. Procédé selon l'une quelconque des revendications précédentes, dans lequel la présence d'acide nucléique de HPV est confirmée dans l'échantillon avant l'étape de typage.

7. Procédé selon l'une quelconque des revendications précédentes, dans lequel l'hybridation entre la sonde et la cible est réalisée en présence d'un support solide.

8. Procédé selon la revendication 7, dans lequel l'étape d'hybridation utilise un format d'hybridation inverse.

9. Procédé selon la revendication 7, dans lequel la sonde est fixée directement ou indirectement sur une bille éventuellement une bille fluorescente.

10. Procédé selon la revendication 9, dans lequel la détection de l'hybridation est analysée en utilisant la cytométrie en flux.

11. Kit comprenant un ensemble d'amorces directe et inverse et une troisième sonde de typage oligonucléotidique capable de s'hybrider à la région 6566-6595 de la séquence de référence du HPV 16 K02718 telle que définie dans la revendication 1 ou à une région équivalente provenant d'autres types de HPV (région B), où les amorces sont choisies dans la liste constituée de HPV-MPF1F1 (SEQ ID NO : 1), HPV-MPF1F2 (SEQ ID NO : 2), HPV-MPF1F3 (SEQ ID NO : 3), HPV-MPF1F4 (SEQ ID NO : 4), HPV-MPF1F5 (SEQ ID NO : 5), HPV-MPF1F6 (SEQ ID NO : 6), HPV-MPF1F7 (SEQ ID NO : 7), HPV-MPF1F8 (SEQ ID NO : 8), HPV-MPF1F9 (SEQ ID NO : 9), HPV-MPF1F10 (SEQ ID NO : 10), HPV-MPF2R1 (SEQ ID NO : 11), HPV-MPF2R2 (SEQ ID NO : 12), HPV-MPF2R3 (SEQ ID NO : 13), HPV-MPF2R4 (SEQ ID NO : 14), HPV-MPF2R5 (SEQ ID NO : 15), HPV-MPF2R6 (SEQ ID NO : 16), HPV-MPF2R7 (SEQ ID NO : 17), HPV-MPF2R8 (SEQ ID NO : 18).

12. Kit comprenant au moins 2 sondes capables d'une hybridation spécifique à la région B du génome du HPV.

13. Kit selon la revendication 12, dans lequel les sondes sont deux sondes quelconques choisies dans l'un quelconque des tableaux 4, 5 à 14, 17, 18, 19.

14. Kit selon la revendication 13 comprenant une paire d'amorces directe et inverse du tableau 1 ou 2.

15. Kit selon l'une quelconque des revendications 12 à 14, comprenant en outre une sonde du tableau 3.
